(19) Europäisches Patentamt
European Patent Office
Office européen des brevets

(11) Veröffentlichungsnummer : **0 039 892**
**B1**

(12) # EUROPÄISCHE PATENTSCHRIFT

(45) Veröffentlichungstag der Patentschrift : **30.12.86**

(21) Anmeldenummer : **81103418.0**

(22) Anmeldetag : **06.05.81**

(51) Int. Cl.[4] : **C 07 D233/64**, C 07 D249/08, C 07 D249/12, C 07 D207/333, C 07 D209/12, C 07 D213/69, C 07 D213/80, C 07 D239/26, C 07 D239/36

(54) **Substituierte Phenyläther.**

(30) Priorität : **09.05.80 CH 3655/80**

(43) Veröffentlichungstag der Anmeldung : **18.11.81 Patentblatt 81/46**

(45) Bekanntmachung des Hinweises auf die Patenterteilung : **30.12.86 Patentblatt 86/52**

(84) Benannte Vertragsstaaten : **AT BE CH DE FR GB IT LI LU NL SE**

(56) Entgegenhaltungen :
**EP-A- 0 005 848**
**EP-A- 0 015 505**
**DE-A- 2 357 849**
**DE-A- 2 534 339**
**GB-A- 1 492 352**
**US-A- 4 012 444**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Fuhrer, Walter, Dr.**
**Munzackerweg 11**
**CH-4402 Frenkendorf (CH)**
Erfinder : **Ostermayer, Franz, Dr.**
**Am Hang 5**
**CH-4125 Riehen (CH)**
Erfinder : **Zimmermann, Markus, Dr.**
**Steinbrecheweg 8**
**CH-4125 Riehen (CH)**

(74) Vertreter : **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun.**
**Bräuhausstrasse 4**
**D-8000 München 2 (DE)**

EP 0 039 892 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind neue substituierte Phenyläther, Verfahren zu ihrer Herstellung, pharmazeutische Präparate enthaltend solche Verbindungen, und ihre Verwendung zur Herstellung von pharmazeutischen Präparaten, oder als pharmakologisch aktive Verbindungen.

Die Erfindung betrifft neue substituierte Phenyläther der Formel

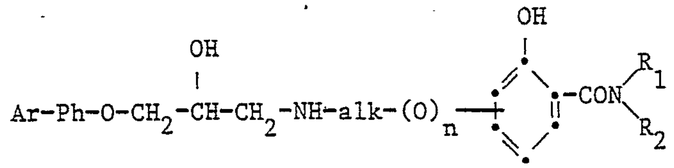

(I)

worin Ar einen gegebenenfalls substituierten Phenylrest, oder einen über ein Ringkohlenstoffatom an den Phenylenrest Ph gebundenen, ein Sauerstoff-, Schwefel- oder Stickstoffatom und gegebenenfalls 1 bis 3 zusätzliche Stickstoffatome als Ringglieder enthaltenden monocyclischen Heteroarylrest mit 5 bis 6 Ringgliedern bedeutet, der mindestens partiell hydriert und in diesem Fall durch Oxo substituiert sein kann, wobei Substituenten gegebenenfalls substituiertes Niederalkyl, oder Niederalkenyl, gegebenenfalls veräthertes oder verestertes Hydroxy, Niederalkylthio, Niederalkylsulfonyl, oder Halogen, Acyl, gegebenenfalls verestertes Carboxy, Carbamoyl, Cyan, Nitro, gegebenenfalls substituiertes, wie acyliertes Amino, gegebenenfalls durch Niederalkyl, Niederalkoxy, Halogen, Halogenniederalkyl, Nitro oder Cyan substituiertes Phenyl oder Benzoyl, und/oder gegebenenfalls, wie angegeben, substituiertes monocyclisches, ein Sauerstoff- oder Stickstoffatom und gegebenenfalls 1 bis 3 weitere Stickstoffatome enthaltendes Heteroaryl bedeuten, Ph gegebenenfalls durch Niederalkyl, Niederalkoxy, Halogen oder Cyan substituiertes 1,4-Phenylen darstellt, n die Zahl 0 oder 1, und alk einen Alkylenrest mit 2 bis 4 Kohlenstoffatomen bedeutet, wobei das Stickstoffatom und das Sauerstoffatom, oder, falls n für 0 steht, der aromatische Rest durch 2 bis 3 Kohlenstoffatome in der unverzweigten Kette voneinander getrennt sind, $R_1$ und $R_2$ für Wasserstoff oder Niederalkyl stehen oder zusammen mit dem Stickstoff der Amidgruppe Pyrrolidino oder Morpholino bilden.

Zum Gegenstand der Erfindung gehören auch N-Oxide von Heterocyclen sowie Salze von Verbindungen der Formel I.

Der Rest Ar ist ein Phenylrest oder ein über ein Ringkohlenstoffatom mit dem Phenylenrest Ph verbundener, ein Sauerstoff-, Schwefel- oder Stickstoffatom und gegebenenfalls 1 bis 3, zusätzliche Stickstoffatome als Ringglieder enthaltender Rest, vorzugsweise mit 5 oder 6 Ringgliedern dar, der mindestens partiell hydriert und in diesem Fall durch Oxo substituiert sein kann, und bedeutet insbesondere Pyridyl, z. B. 2-, 3- oder 4-Pyridyl, oder Dihydro-oxo-pyridinyl z. B. 1,6-Dihydro-6-oxo-2-pyridinyl, Pyridazinyl, z. B. 3-Pyridazinyl, Pyrazinyl, z. B. 2-Pyrazinyl, Pyrimidinyl, z. B. 2-, 4- oder 5-Pyrimidinyl, oder Dihydro-oxo-pyrimidinyl z. B. 3,4-Dihydro-4-oxo-2-pyrimidinyl, Furyl z. B. 2- oder 3-Furyl, Pyrryl z. B. 2- oder 3-Pyrryl, Thienyl z. B. 2- oder 3-Thienyl, Oxazolyl z. B. 2- oder 4-Oxazolyl, Thiazolyl, z. B. 2-, 4- oder 5-Thiazolyl, Thiadiazolyl z. B. 1,2,4-Thiadiazol-3- oder -5-yl oder 1,2,5-Thiadiazol-3-yl, Imidazolyl z. B. Imidazol-2- oder -4-yl, Pyrazolyl z. B. 3- oder 4-Pyrazolyl, Triazolyl z. B. 1,2,3-Triazol-4-yl oder 1,2,4-Triazol-3-yl, Tetrazolyl wie Tetrazol-5-yl.

Substituenten eines Phenyl- oder heterocyclischen Arylrestes, wobei in letzterem Falle in erster Linie ein Ringkohlenstoffatom, aber auch ein sekundäres Ringstickstoffatom substituiert sein kann, und solche Substituenten ein- oder mehrfach, vorzugsweise höchstens vierfach vorhanden sein können, sind z. B. gegebenenfalls substituiertes Niederalkyl, z. B. Niederalkyl, gegebenenfalls veräthertes oder verestertes Hydroxy-niederalkyl, wie Hydroxyniederalkyl, Niederalkoxyniederalkyl oder Halogenniederalkyl, oder gegebenenfalls substituiertes, wie acyliertes Aminoniederalkyl, wie Niederalkanoylaminoniederalkyl oder Niederalkoxycarbonylaminoniederalkyl, oder Niederalkenyl, gegebenenfalls veräthertes oder verestertes Hydroxy oder Mercapto, wie Hydroxy, gegebenenfalls, z. B. durch Aryl, durch gegebenenfalls veräthertes oder verestertes Hydroxy oder Mercapto oder durch Acyl substituiertes Niederalkoxy, z. B. Niederalkoxy, Phenylniederalkoxy, Hydroxyniederalkoxy, Niederalkoxyniederalkoxy, Niederalkylthioniederalkoxy, Halogenniederalkoxy oder Niederalkanoylniederalkoxy, oder Niederalkenyloxy, Niederalkylthio oder Halogen, Acyl, z. B. Niederalkanoyl, gegebenenfalls verestertes Carboxy, wie Carboxy oder Niederalkoxycarbonyl, Carbamoyl, Cyan, Nitro oder gegebenenfalls substituiertes, wie acyliertes Amino, z. B. Niederalkanoylamino, Niederalkoxycarbonylamino, Niederalkylsulfonyl, ferner Amino, N-Niederalkylamino oder N,N-Diniederalkylamino. Substituenten eines heterocyclischen Arylrestes Ar sind ausserdem gegebenenfalls durch Niederalkyl, Niederalkoxy, Halogenniederalkyl, Carbamoyl oder Cyan substituiertes Phenyl oder Benzoyl, oder gegebenenfalls, z. B. wie angegeben, substituierte heterocyclische Arylreste, z. B. die genannten, die an ein Ringkohlenstoffatom oder ein Ringstickstoffatom des zu substituierenden heterocyclischen Arylrestes gebunden sind.

Die im Zusammenhang mit der vorliegenden Beschreibung mit « nieder » bezeichneten Reste und Verbindungen enthalten vorzugsweise bis 7 und in erster Linie bis 4 Kohlenstoffatome.

Niederalkyl ist z. B. Methyl, Aethyl, n-Propyl, Isopropyl, n-Butyl, Isobutyl oder tert.-Butyl ; substituiertes Niederalkyl ist insbesondere entsprechendes Methyl oder 1- oder 2-Aethyl.

Niederalkenyl ist z. B. Vinyl, Allyl, 2- oder 3-Methallyl oder 3,3-Dimethylallyl.

Niederalkoxy ist z. B. Methoxy, Aethoxy, n-Propyloxy, Isopropyloxy, n-Butyloxy oder Isobutyloxy, während Phenylniederalkoxy z. B. Benzyloxy oder 1- oder 2-Phenyläthoxy, Niederalkenyloxy z. B. Allyloxy, 2- oder 3-Methallyloxy oder 3,3-Dimethylallyloxy darstellt.

Niederalkylthio ist z. B. Methylthio, Aethylthio, n-Propylthio oder Isopropylthio.

Niederalkylsulfonyl ist z. B. Methylsulfonyl oder Aethylsulfonyl.

Halogen ist vorzugsweise Halogen mit Atomnummer bis zu 35, d. h. Fluor, Chlor oder Brom.

Niederalkanoyl ist z. B. Acetyl, Propionyl oder Butyryl.

Niederalkoxycarbonyl ist z. B. Methoxycarbonyl oder Aethoxycarbonyl.

Niederalkanoylamino ist z. B. Acetylamino oder Propionylamino.

Niederalkoxycarbonylamino ist z. B. Methoxycarbonylamino oder Aethoxycarbonylamino.

N-Niederalkylamino und N,N-Diniederalkylamino sind z. B. Methylamino, Aethylamino, Dimethylamino oder Diäthylamino.

Hydroxyniederalkyl ist vorzugsweise Hydroxymethyl oder 1- und in erster Linie 2-Hydroxyäthyl.

Niederalkoxyniederalkyl ist vorzugsweise Niederalkoxymethyl oder 1- und in erster Linie 2-Niederalkoxyäthyl, z. B. Methoxymethyl, Aethoxymethyl, 2-Methoxy-äthyl oder 2-Aethoxy-äthyl.

Halogenniederalkyl ist vorzugsweise Halogenmethyl, z. B. Trifluormethyl.

Niederalkanoylaminoniederalkyl ist insbesondere Niederalkanoylaminomethyl oder 1- und in erster Linie 2-Niederalkanoylaminoäthyl, z. B. Acetylaminomethyl, 2-Acetylamino-äthyl oder 2-Propionylaminoäthyl.

Niederalkoxycarbonylaminoniederalkyl ist insbesondere Niederalkoxycarbonylaminomethyl, oder 1- und in erster Linie 2-Niederalkoxycarbonylamino-äthyl, z. B. Methoxycarbonylaminomethyl, 2-Methoxycarbonylamino-äthyl oder 2-Aethoxycarbonylamino-äthyl.

Niederalkoxyniederalkoxy ist u. a. Niederalkoxymethoxy oder 1- und insbesondere 2-Niederalkoxyäthoxy, z. B. Methoxymethoxy, 2-Methoxy-äthoxy oder 2-Aethoxy-äthoxy.

Niederalkylthioniederalkoxy ist insbesondere Niederalkylthiomethoxy oder 1- und in erster Linie 2-Niederalkylthioäthoxy, z. B. 2-Methylthio-äthoxy oder 2-Aethylthio-äthoxy.

Halogenniederalkoxy ist insbesondere 2-Halogenäthoxy, z. B. 2-Chloräthoxy.

Niederalkanoylniederalkoxy ist z. B. Niederalkanoylmethoxy oder 1- oder 2-Niederalkanoyläthoxy, z. B. Acetylmethoxy.

Substituenten in einem monocyclischen heterocyclischen Arylrest Ar, z. B. den genannten, wie Pyridyl, Pyrimidinyl, Furyl, Pyrryl, Thienyl, Thiadiazolyl, Indolyl, Chinolyl, Imidazolyl oder Benzimidazolyl sind weiterhin gegebenenfalls, z. B. wie angegeben, substituierte monocyclische Heteroarylreste, z. B. solche wie angegeben.

In diesem Sinne substituierte Reste Ar sind beispielsweise Pyridyl-phenyl, wie z. B. Niederalkyl- wie (Methyl-2-pyridyl)-phenyl, (Cyan-2-pyridyl)-phenyl, Pyrimidinyl-phenyl wie (2-Pyrimidinyl)-phenyl oder (Hydroxy-2-pyrimidinyl)-phenyl, Furyl-phenyl z. B. Niederalkoxy-, wie Methoxy-furyl-(2)-phenyl, Pyrrylphenyl wie Pyrrol-2-yl- oder -3-yl-phenyl, z. B. Halogenniederalkyl-, wie Trifluormethyl-pyrrol-2-yl oder -3-yl-phenyl, Imidazolyl-phenyl z. B. Halogenniederalkyl-, wie Trifluormethylimidazol-2-yl-phenyl, Imidazolylfuryl z. B. Halogenniederalkyl- wie (Trifluormethyl)-imidazol-2-yl-furyl, Pyrimidinyl-furyl wie z. B. Hydroxy-2-pyrimidinyl-furyl, Pyrryl-furyl z. B. Halogen- wie Chlor-pyrrol-3-furyl, Thienyl-furyl z. B. Niederalkyl- wie Methyl-2-thienyl-furyl, Thiadiazolyl-furyl z. B. 1,2,4-Thiadiazol-3-yl-furyl, Indolyl-furyl wie Niederalkoxy- z. B. 4-Methoxyindol-2-yl-furyl, Benzimidazolyl-furyl z. B. Niederalkyl- wie 4-Methyl-benzimidazol-2-yl-furyl, Triazolylfuryl wie 1,2,4-Triazol-3-yl-furyl, Tetrazolyl-furyl wie Tetrazol-5-yl-furyl, Pyrazolyl-furyl wie Pyrazol-3-yl- oder 4-yl-furyl, Chinolinyl-furyl z. B. Niederalkoxy- wie Methoxy-2-chinolinyl-furyl, Thienylimidazolyl z. B. Niederalkoxy- wie (Methoxy-2-thienyl- oder 3-thienyl)-imidazolyl, Thiazolylimidazolyl z. B. Halogen- wie Chlor-thiazol-2-yl-imidazolyl oder 1,2,5-Thiadiazol-3-yl-imidazolyl.

Ein Phenylenrest Ph ist ein 1,4-Phenylenrest, der 1- bis 3-fach substituiert sein kann. Substituenten sind vor allem Niederalkyl, wie Methyl, Niederalkoxy wie Methoxy, Halogen z. B. Chlor, oder Cyan.

Alkylen alk kann geradkettig oder verzweigt sein und ist z. B. 1,2-Aethylen, 1,2-, 2,3- oder 1,3-Propylen, 1,4- oder 2,4-Butylen, oder 1,1-Dimethyläthylen.

Zusammen mit dem Stickstoffatom der Amidgruppe sind $R_1$ und $R_2$ Pyrrolidino oder Morpholino.

Die aliphatische Seitenkette kann den Salicylamidring in beliebiger Stellung, vorzugsweise in der para-Stellung zur Carbamoylgruppe, insbesondere in der para-Stellung zur Hydroxygruppe substituieren.

N-Oxide leiten sich von solchen Verbindungen ab, worin Ar mindestens ein tertiäres Stickstoffatom enthaltendes mono- oder bicyclisches Heteroaryl, z. B. Pyridyl, Pyrazinyl, Pyrimidinyl, Imidazolyl, Pyrazolyl, Triazolyl, Tetrazolyl, Thiazolyl oder Thiadiazolyl darstellt.

Die neuen Verbindungen können in Form ihrer Salze, wie ihrer Säureadditionssalze und in erster Linie ihrer pharmazeutisch verwendbaren nicht-toxischen Säureadditionssalze vorliegen. Geeignete Salze sind z. B. solche mit anorganischen Säuren, wie Halogenwasserstoffsäuren, z. B. Chlorwasserstoffsäure oder Bromwasserstoffsäure, Schwefelsäure, oder Phosphorsäure, oder mit organischen Säuren, wie aliphatischen, cycloaliphatischen, aromatischen oder heterocyclischen Carbon- oder Sulfonsäuren, z. B. Ameisen-, Essig-, Propion-, Bernstein-, Glykol-, Milch-, Aepfel-, Wein-, Zitronen-, Malein-, Hydroxymalein-, Brenztrauben-, Fumar-, Benzoe-, 4-Aminobenzoe-, Anthranil-, 4-Hydroxybenzoe-, Salicyl-, Embon-, Methansulfon-, Aethansulfon-, 2-Hydroxyäthansulfon-, Aethylensulfon-, Toluolsulfon-, Naphthalinsulfon- oder Sulfanilsäure, oder mit anderen sauren organischen Stoffen, wie Ascorbinsäure.

Die neuen Verbindungen der Formel I besitzen wertvolle pharmakologische Eigenschaften. Insbesondere wirken sie in spezifischer Weise auf β-adrenerge Rezeptoren. Dieser Wirkung liegt als gemeinsame Eigenschaft ihre Affinität zu diesen Rezeptoren zugrunde, die sich bei fehlender oder sehr geringer stimulierender Eigenwirkung als reine Blockade, bei geringer bis mässiger stimulierender Eigenwirkung als Blockade mit gleichzeitiger ISA, d. h. Intrinsic Sympothomimetic Activity, und bei stärkerer stimulierender Eigenwirkung als überwiegende Stimulierung der β-adrenergen Rezeptoren äussert. Die Grenzen zwischen β-Rezeptoren-Blockern ohne oder mit mehr oder weniger ausgeprägter ISA sind fliessend, ebenso die therapeutischen Anwendungsbereiche dieser Verbindungstypen.

Sowohl bei den Verbindungen mit überwiegend β-blockierenden Eigenschaften als auch bei jenen mit überwiegend β-stimulierenden Eigenschaften können deutliche Unterschiede in ihrer Affinität zu kardialen ($\beta_1$-) und zu vaskulären oder tracheobronchialen ($\beta_2$-) Rezeptoren vorliegen. Schliesslich können auch noch blutdrucksenkende und bradykarde Wirkungen sowie blockierende Wirkungen auf α-adrenerge Rezeptoren und ferner noch stimulierende Wirkungen auf dopaminerge Rezeptoren hinzukommen.

Von den Verbindungen der Formel I zeigt z. B. das 1-[2-(3-Carbamoyl-4-hydroxy-phenoxy)-äthylamino]-3-[4-[1-methyl-4-(trifluormethyl)-1H-imidazol-2-yl]-phenoxy]-2-propanol als Hauptwirkung eine blockierende Wirkung auf β-Rezeptoren mit deutlich bevorzugter Hemmung der cardialen Rezeptoren, zu der als zusätzliche Wirksamkeiten noch eine bradykarde und blutdrucksenkende sowie eine schwache blockierende Wirkung auf α-Rezeptoren und ferner noch stimulierende Wirkungen auf dopaminerge Rezeptoren hinzukommen.

Bei anderen Verbindungen der Formel I lassen sich sowohl β-Rezeptoren-blockinde als vor allem auch β-Rezeptoren-stimulierende Wirkungen feststellen. Diese Verbindungen erweisen sich einerseits bei *in vitro*-Versuchen am Meerschweinchenherzen als potente Blocker von cardialen β-Rezeptoren, andererseits bei *in vivo*-Versuchen an der narkotisierten Katze als stark wirkende, überwiegend β-Rezeptorenstimulierende Verbindungen mit zusätzlichen β-Rezeptoren-blockierenden Eigenschaften. Zu dieser Gruppe gehören z. B. solche Verbindungen, in denen Ar einen Heteroarylrest mit der Gruppe —NH— als Ringglied darstellt.

So lässt sich z. B. mit dem 1-[2-(3-Carbamoyl-4-hydroxy-phenoxy)-äthylamino]-3-[4-[(4-trifluormethyl)-1H-imidazol-2-yl]-phenoxy]-2-propanol bei *in vivo* Versuchen an der Katze eine Stimulation von β-Rezeptoren zeigen. Als zusätzliche Wirkungen lassen sich bei Verbindungen dieser Art eine Blutdrucksenkung als auch eine schwache Blockade von adrenergen α-Rezeptoren feststellen.

Die vorstehenden Angaben betreffend pharmakologische Eigenschaften beruhen auf den Resultaten von entsprechenden pharmakologischen Versuchen in üblichen Testverfahren. So zeigen die neuen β-blockierenden Verbindungen eine Hemmung der durch Isoproterenol induzierten Tachykardie am isolierten Meerschweinchenherzen in einem Konzentrationsbereich von etwa 0,001 μg/ml bis etwa 1 μg/ml und an der narkotisierten Katze in einem Dosenbereich von etwa 0,001 mg/kg bis etwa 10 mg/kg bei intravenöser Verabreichung. Die Hemmung der durch Isoproterenol induzierten Vasodilation an der narkotisierten Katze mit Perfusion der Arteria femoralis ist in Abhängigkeit vom Ausmass der Cardioselektivität bei intravenöser Verabreichung erst in höheren Dosen nachweisbar. Die ISA von Verbindungen der Formel I mit β-blockierenden Eigenschaften ergibt sich aus der Zunahme der basalen Herzfrequenz an der narkotisierten, mit Reserpin vorbehandelten Katze bei intravenöser Verabreichung in einem Dosenbereich von etwa 0,001 mg/kg bis etwa 3 mg/kg. Die neuen Verbindungen bewirken in einem Dosenbereich von etwa 0,01 mg/kg bis etwa 10 mg/kg bei intravenöser Verabreichung eine Senkung des arteriellen Blutdrucks an der narkotisierten Katze. Die bradykarde Wirkung tritt an der narkotisierten Katze in Dosen von etwa 0,01 mg/kg bis etwa 10 mg/kg auf und lässt sich auch am spontan schlagenden, isolierten rechten Meerschweinchenvorhof in einem Konzentrationsbereich von etwa 1 μg/ml bis etwa 30 μg/ml nachweisen. Die zusätzliche α-blockierende Wirksamkeit geht z. B. aus der Antagonisierung der durch Noradrenalin induzierten Kontraktion des isolierten Vas deferens der Ratte durch solche Verbindungen in einem Konzentrationsbereich von 1 μg/ml bis etwa 30 μg/ml hervor. Die neuen β-blockierenden Verbindungen der Formel I können als β-Rezeptoren-Blocker mit oder ohne Cardioselektivität und mit oder ohne ISA, z. B. zur Behandlung von Angina pectoris, hypertrophen Kardiomyopathien und Herzrhythmusstörungen sowie als blutdrucksenkende Mittel verwendet werden. Weitere therapeutische Anwendungsmöglichkeiten sind z. B. bei erhöhtem intraokularem Druck oder bei der Thyreotoxikose gegeben.

Die Verbindungen der Formel I mit β-Rezeptoren-stimulierenden Eigenschaften bewirken eine Zunahme von Herzfrequenz und myocardialer Kontraktionskraft am isolierten Meerschweinchenvorhof in einem Konzentrationsbereich von etwa 0,001 μg/ml bis 1 μg/ml und eine Zunahme der Herzfrequenz an der mit Reserpin vorbehandelten narkotisierten Katze in einem Dosenbereich von etwa 0,001 mg/kg bis etwa 1 mg/kg i. v. Die zur Senkung des artiellen Blutdrucks an der narkotisierten Katze erforderlichen Dosen liegen demgegenüber, entsprechend einem Dosenbereich von etwa 0,01 mg/kg bis etwa 10 mg/kg i. v., höher, d. h. die neuen Verbindungen stimulieren bevorzugt die cardialen β-Rezeptoren ($\beta_1$-Rezeptoren) im Vergleich zu den β-Rezeptoren in den Blutgefässen ($\beta_2$-Rezeptoren) und unterscheiden sich dadurch von Isoproterenol, welches die β-Rezeptoren des Herzens und der Blutgefässe etwa gleich stark stimuliert.

Die neuen Verbindungen mit β-Rezeptoren-stimulierenden Eigenschaften können somit als positiv inotrop wirkende Mittel, insbesondere als Cardiotonica zur Behandlung der Herzmuskelinsuffizienz, allein oder in Kombination mit anderen Präparaten, wie Herzglykosiden, verwendet werden.

Die Verbindungen der Formel I können auch als wertvolle Zwischenprodukte für die Herstellung anderer wertvoller, insbesondere pharmazeutisch wirksamer Verbindungen verwendet werden.

In der GB-Patentschrift No 1.492.352 werden 1-(Imidazolylaryloxy)-2-hydroxy-3-aminopropane mit antihypertensiven und β-adrenergische Rezeptoren blockierenden und teilweise cardioselektiven Eigenschaften beschrieben, aufgrund derer solche Verbindungen zur Behandlung von Angina pectoris und bestimmten Formen von Herzarrhythmien geeignet sind.

Aus der EP-Anmeldung 0 005 848 sind (Carbamoyl-hydroxy-phenyl- oder -phenoxyalkyl)-aminomethyl-benzylalkohole mit überwiegend cardiale β-Rezeptoren stimulierenden Eigenschaften bekannt, welche solche Substanzen als zur Behandlung der Herzinsuffizienz geeignet erscheinen lassen. Ueberraschenderweise wurde nun gefunden, dass die zu Verbindungen der vorliegenden Anmeldung führende Verknüpfung der in β-stimulierend wirksamen Verbindungen der EP-Anmeldung 0 005 848 vorhandenen Carbamoyl-hydroxy-phenyl- oder -phenoxyalkylgruppe mit dem in der Seitenkette von Imidazolverbindungen der GB Patentschrift No 1.492.352 stehenden N-Atom, wobei das 1H-Stickstoffatom des Imidazolringes z. B. eine $C_{1-10}$-Alkylgruppe, etwa Methyl, trägt, Verbindungen mit zur Hauptsache β-blockierenden Eigenschaften ergibt, während in ebenfalls überraschender Weise die beschriebene Verknüpfung der Carbamoyl-hydroxy-phenyl- oder phenoxyalkylgruppe an solche Imidazolverbindungen der GB-Patentschrift No 1.492.352, worin das 1H-Stickstoffatom des Imidazolringes unsubstituiert ist, und solche Imidazolverbindungen dort als bevorzugte, β-blockierend Wirksame Substanzen beschrieben sind, zu Verbindungen gemäss der vorliegenden Anmeldung mit überwiegend β-stimulierenden Eigenschaften führt.

Die Erfindung betrifft insbesondere Verbindungen der Formel I, worin Ar einen gegebenenfalls substituierten Phenylrest, oder einen über ein Ringkohlenstoffatom an den Phenylenrest Ph gebundenen, ein Sauerstoff-, Schwefel- oder Stickstoffatom und gegebenenfalls 1 bis 2 zusätzliche Stickstoffatome als Ringglieder enthaltenden monocyclischen Heteroarylrest mit 5 bis 6 Ringgliedern bedeutet, oder einen mindestens partiell hydrierten Oxo-Heteroarylrest mit 1-2 Stickstoffatomen als Ringglieder darstellt, wobei Substituenten gegebenenfalls substituiertes Niederalkyl, z. B. Niederalkyl, Hydroxyniederalkyl, Halogenniederalkyl, gegebenenfalls veräthertes oder verestertes Hydroxy, z. B. Hydroxy, Niederalkoxy, Phenylniederalkoxy, Niederalkylthio, Niederalkylsulfonyl oder Halogen, Acyl, z. B. Niederalkanoyl, gegebenenfalls verestertes Carboxy wie Carboxy oder Niederalkoxycarbonyl, Carbamoyl, Cyan, gegebenenfalls substituiertes, wie acyliertes Amino, z. B. Amino, N-Niederalkylamino, N,N-Diniederalkylamino, Niederalkanoylamino, Niederalkoxycarbonylamino, gegebenenfalls durch Niederalkyl, Niederalkoxy, Halogen, Halogenniederalkyl oder Cyan substituiertes Phenyl oder Benzoyl, und/oder gegebenenfalls, wie angegeben, substituiertes monocyclisches, ein Sauerstoff- oder Stickstoffatom und gegebenenfalls 1 bis 2 weitere Stickstoffatome enthaltendes Heteroaryl bedeuten, Ph gegebenenfalls durch Niederalkyl, Niederalkoxy, Halogen oder Cyan substituiertes 1,4-Phenylen darstellt, n die Zahl 0 oder 1, und alk einen Alkylenrest mit 2 bis 3 Kohlenstoffatomen bedeutet, wobei das Stickstoffatom und das Sauerstoffatom, oder, falls n für 0 steht, der aromatische Rest durch 2 bis 3 Kohlenstoffatome in der unverzweigten Kette voneinander getrennt sind, $R_1$ und $R_2$ für Wasserstoff oder Niederalkyl stehen oder zusammen mit dem Stickstoffatom der Amidgruppe Morpholino bilden, oder deren N-Oxide und Salze, insbesondere Säureadditionssalze, vor allem pharmazeutisch verwendbare, nicht-toxische Säureadditionssalze davon.

Die Erfindung betrifft vor allem Verbindungen der Formel I, worin Ar einen gegebenenfalls substituierten, über ein Ringkohlenstoffatom an den Phenylenrest Ph gebundenen ein Sauerstoff-, Schwefel- oder ein Stickstoffatom und gegebenenfalls 1 bis 2 zusätzliche Stickstoffatome als Ringglieder enthaltenden Heteroarylrest mit 5 bis 6 Ringgliedern, z. B. Furyl, Oxazolyl, Imidazol, Triazolyl, Thiazolyl, Indolyl oder Pyridyl, bedeutet, oder einen Dihydro-oxo-Heteroarylrest mit 1-2 Stickstoffatomen als Ringglieder, z. B. Dihydro-oxo-2-pyridinyl oder Dihydro-4-oxo-2-pyrimidinyl darstellt, wobei Substituenten Niederalkyl, z. B. Methyl, Hydroxyniederalkyl, z. B. 2-Hydroxyäthyl, Halogenniederalkyl, z. B. Trifluormethyl, Hydroxy, Niederalkoxy, z. B. Methoxy, Niederalkylthio, z. B. Methylthio, Niederalkylsulfonyl z. B. Methylsulfonyl, Halogen, Niederalkanoyl z. B. Acetyl, Carboxy, Niederalkoxycarbonyl z. B. Methoxycarbonyl, Cyan, Carbamoyl, Amino, Niederalkanoylamino z. B. Acetylamino, Niederalkoxycarbonylamino z. B. Methoxycarbonylamino, und/oder gegebenenfalls, wie angegeben, substituiertes z. B. Imidazolyl oder monocyclisches, ein Sauerstoff- oder Stickstoffatom und gegebenenfalls ein weiteres Stickstoffatom enthaltendes Heteroaryl, z. B. Furyl oder Oxazolyl bedeuten, Ph gegebenenfalls durch Niederalkyl, wie Methyl, oder Halogen, wie Chlor, substituiertes 1,4-Phenylen darstellt, n die Zahl 0 oder 1, alk einen Alkylenrest mit 2 bis 3 Kohlenstoffatomen bedeutet, wobei das Stickstoffatom und das Sauerstoffatom, oder, falls n für 0 steht, der aromatische Rest, durch 2 bis 3 Kohlenstoffatome in der unverzweigten Kette voneinander getrennt sind, $R_1$ und $R_2$ jeweils für Wasserstoff stehen, oder deren N-Oxide und Salze, insbesondere Säureadditionssalze, vor allem pharmazeutisch verwendbare, nicht-toxische Säureadditionssalze davon.

Die Erfindung betrifft speziell die in den Beispielen genannten Verbindungen der Formel I, oder deren N-Oxide und Salze, insbesondere Säureadditionssalze, in erster Linie pharmazeutisch verwendbare, nicht-toxische Säureadditionssalze davon.

Die neuen Verbindungen der Formel I werden in an sich bekannter Weise hergestellt. Man kann sie z. B. erhalten, indem man in einer Verbindung der Formel

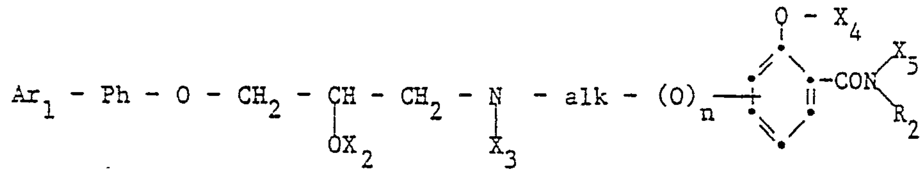

(II)

worin $Ar_1$ den Rest Ar oder einen diesem entsprechenden, mindestens eine funktionelle Gruppierung in geschützter Form enthaltenden Rest darstellt, $X_2$, $X_3$ und $X_4$ jeweils Wasserstoff oder einen durch Wasserstoff ersetzbaren Substituenten bedeuten, $X_5$ für $R_1$ oder einen durch Wasserstoff ersetzbaren Substituenten steht, oder $X_2$ und $X_3$ und/oder $X_4$ und $X_5$ zusammen einen zweiwertigen, durch zwei Wasserstoffatome ersetzbaren Rest bedeuten, und/oder im Rest $Ar_1$ gegebenenfalls vorhandene funktionelle Gruppen gegebenenfalls in geschützter Form vorliegen mit der Massgabe, dass mindestens einer der Reste $X_2$, $X_3$, $X_4$ oder $X_5$ von Wasserstoff verschieden ist, oder mindestens $Ar_1$ einen Rest Ar bedeutet, der mindestens eine funktionelle Gruppierung in geschützter Form enthält, oder mindestens $X_2$ und $X_3$ zusammen oder $X_4$ und $X_5$ zusammen einen zweiwertigen durch zwei Wasserstoffatome ersetzbaren Rest darstellen, oder in einem Salz davon, die von Wasserstoff verschiedenen Gruppen $X_2$, $X_3$, $X_4$ oder $X_5$ bzw. $X_2$ und $X_3$ zusammen oder $X_4$ und $X_5$ zusammen durch Wasserstoffatome ersetzt, und/oder in einem Rest $Ar_1$ die an eine oder mehrere funktionelle Gruppen gebundenen Schutzgruppen abspaltet und durch Wasserstoff ersetzt, und, wenn erwünscht, eine so erhältliche Verbindung in eine andere Verbindung der Formel I umwandelt und/oder, wenn erwünscht, eine erhaltene freie Verbindung in ein Salz oder ein erhaltenes Salz in eine freie Verbindung überführt, und/oder, wenn erwünscht, ein erhaltenes Isomerengemisch in die Isomeren oder ein erhaltenes Racemat in die Antipoden auftrennt.

Geschützte funktionelle Gruppen in einem Rest $Ar_1$ sind z. B. geschützte Hydroxy-, Amino- oder Mercaptogruppen, ferner eine als Ringglied in einem Heteroarylrest Ar stehende geschützte Gruppe —NH—, wobei Schutzgruppen abspaltbare und durch Wasserstoff ersetzbare Gruppen darstellen.

Die Abspaltung der Gruppen $X_2$, $X_3$ oder $X_4$ oder jeweils $X_2$ und $X_3$ oder $X_4$ und $X_5$ zusammen, sowie der in einem Rest $Ar_1$ an funktionellen Gruppen, z. B. Hydroxy- und/oder Aminogruppen stehenden Schutzgruppen wird mittels Solvolyse, wie Hydrolyse, Alkoholyse oder Acidolyse, oder mittels Reduktion, einschliesslich Hydrogenolyse vorgenommen.

Eine besonders geeignete, abspaltbare Gruppe $X_3$ oder $X_4$, oder eine Hydroxy- oder Amino-Schutzgruppe in einem Rest $Ar_1$ ist in erster Linie eine hydrogenolytisch abspaltbare α-Arylniederalkylgruppe, wie eine gegebenenfalls substituierte 1-Polyphenylniederalkyl- oder 1-Phenylniederalkylgruppe, z. B. Benzydryl oder Trityl, worin Substituenten, insbesondere des Phenylteils, z. B. Niederalkyl, wie Methyl, oder Niederalkoxy wie Methoxy, sein können, und in erster Linie Benzyl. Eine Gruppe $X_3$ und insbesondere $X_2$ und $X_4$ sowie Hydroxy- und/oder Amino-Schutzgruppen in einem Rest Ar können auch einen solvolytisch, wie hydrolytisch oder acidolytisch, ferner einen reduktiv, einschliesslich hydrogenolytisch, abspaltbaren Rest, insbesondere einen entsprechenden Acylrest, wie den Acylrest einer organischen Carbonsäure, z. B. Niederalkanoyl, wie Acetyl, oder Aroyl, wie Benzoyl, ferner den Acylrest eines Halbesters der Kohlensäure, wie Niederalkoxycarbonyl, z. B. Methoxycarbonyl, Aethoxycarbonyl oder tert.-Butyloxycarbonyl, 2-Halogenniederalkoxycarbonyl, z. B. 2,2,2-Trichloräthoxycarbonyl oder 2-Jodäthoxycarbonyl, gegebenenfalls substituiertes 1-Phenylniederalkoxycarbonyl, z. B. Benzyloxycarbonyl oder Diphenylmethoxycarbonyl, oder Aroylmethoxycarbonyl, z. B. Phenacyloxycarbonyl, ferner eine gegebenenfalls substituierte 1-Polyphenyl-niederalkylgruppe, z. B. wie oben angegeben und in erster Linie Trityl darstellen.

Ein durch $X_2$ und $X_3$ oder $X_4$ und $X_5$ zusammen gebildeter, abspaltbarer Rest ist in erster Linie eine hydrogenolytisch abspaltbare Gruppe, wie gegebenenfalls substituiertes 1-Phenyl-niederalkyliden, worin Substituenten, insbesondere des Phenylteils, z. B. Niederalkyl oder Niederalkoxy sein können, und insbesondere Benzyliden, oder Cycloalkyliden, z. B. Cyclopentyliden oder Cyclohexyliden, ferner für $X_2$ und $X_3$ zusammen die Carbonylgruppe darstellt.

In der Form von Salzen verwendbare Ausgangsstoffe werden in erster Linie in der Form von Säureadditionssalzen, z. B. mit Mineralsäuren, sowie mit organischen Säuren verwendet.

Hydrogenolytisch abspaltbare Reste $X_2$, $X_3$, $X_4$ und/oder $X_5$, insbesondere gegebenenfalls substituierte 1-Phenylniederalkylgruppen, ferner auch geeignete Acylgruppen, wie gegebenenfalls substituiertes 1-Phenylniederalkoxycarbonyl, sowie durch die Gruppen $X_2$ und $X_3$ sowie $X_4$ und $X_5$ zusammen gebildete, gegebenenfalls substituierte 1-Phenylniederalkylidengruppen, sowie in einem Rest Ar vorhandene Hydroxy- und/oder Amino-Schutzgruppen dieser Art können durch Behandeln mit katalytisch aktiviertem Wasserstoff, z. B. mit Wasserstoff in Gegenwart eines Katalysators, wie eines geeigneten Edelmetallkatalysators, z. B. Palladium oder Platin abgespalten werden.

Hydrolytisch abspaltbare Gruppen $X_2$, $X_3$, $X_4$ und/oder $X_5$, wie Acylreste von organischen Carbonsäuren, z. B. Niederalkanoyl, und von Halbestern der Kohlensäure, z. B. Niederalkoxycarbonyl, ferner z. B. Tritylreste, sowie durch die Reste $X_2$ und $X_3$ und/oder $X_4$ und $X_5$ zusammen gebildete Niederalkyliden-, 1-Phenyl-niederalkyliden- oder Cycloalkylidengruppen, sowie in einem Rest $Ar_1$ an funktionellen Gruppen, wie an Hydroxy-, Mercapto-, Amino- und/oder an einer Gruppe —NH— als Ringglied eines Heteroarylrestes Ar stehende Schutzgruppen dieser Art können je nach Art solcher Reste durch Behandeln mit Wasser unter sauren oder basischen Bedingungen, z. B. in Gegenwart einer Mineralsäure, wie Chlorwasserstoff- oder Schwefelsäure, oder eines Alkalimetall- oder Erdalkalimetallhydroxids oder -carbonats oder eines Amins, wie Isopropylamin, abgespalten werden.

Acidolytisch abspaltbare Reste $X_2$, $X_3$, $X_4$ und/oder $X_5$ und/oder an funktionellen Gruppen, etwa den genannten, z. B. an Hydroxy, Mercapto oder Amino stehende Schutzgruppen in einem Rest $Ar_1$, sind insbesondere gewisse Acylreste von Halbestern der Kohlensäure, wie z. B. tert.-Niederalkoxycarbonyl oder gegebenenfalls substituierte Diphenylmethoxycarbonylreste, ferner auch ein tert.-Niederalkylrest ; solche Reste können z. B. durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $X_2$, $X_3$, $X_4$ und/oder $X_5$ und/oder an funktionellen Gruppen, etwa den genannten, z. B. an Hydroxy und/oder Amino stehende Schutzgruppen in einem Rest $Ar_1$, werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z. B. beim Behandeln mit einem reduzierenden Schwermetall, wie Zink, oder mit einem reduzierenden Schwermetallsalz, wie einem Chrom(II)salz, z. B. -chlorid oder -acetat, üblicherweise in Gegenwart einer organischen Carbonsäure, wie Ameisensäure oder Essigsäure, und von Wasser abgespalten werden können.

Schutzgruppen, welche an in einem Rest $Ar_1$ vorhandenen funktionellen, z. B. Hydroxy- und/oder Aminogruppen stehen, entsprechenden vorhin genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Die obigen Reaktionen werden üblicherweise in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z. B. in einem offenen oder geschlossenen Gefäss und/oder in der Atmosphäre eines Inertgases, z. B. Stickstoff.

Die Ausgangsstoffe der Formel II lassen sich z. B. durch Umsetzung einer Verbindung der Formel

$$Ar_1—Ph—OH \qquad (IIa),$$

oder einem Salz davon, mit einer Verbindung der Formel

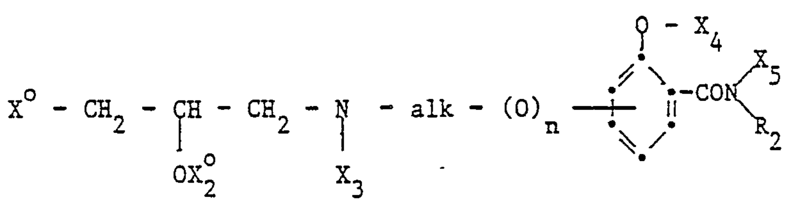

(IIb)

worin $X^0$ für eine reaktionsfähige veresterte Hydroxygruppe und $X_2^0$ für die Gruppe $X_2$ steht, oder $X^0$ und $X_2^0$ die direkte Bindung darstellen, und $Ar_1$ sowie die anderen Gruppen die oben gegebenen Bedeutungen haben, oder durch Behandeln einer Verbindung der Formel

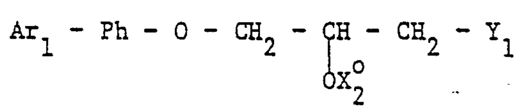

(IIc)

mit einer Verbindung der Formel

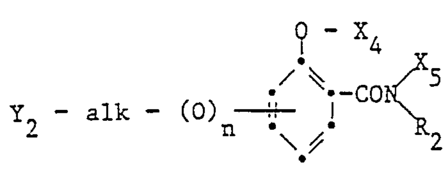

(IId)

worin $X_2^0$ die oben für $X_2$ gegebene Bedeutung hat, und eine der Gruppen $Y_1$ und $Y_2$ für eine reaktionsfähige veresterte Hydroxygruppe, z. B. Halogen, etwa Chlor oder Brom, und die andere für die Gruppe der Formel —NH($X_3$) steht, oder worin $X_2^0$ und $Y_1$ die direkte Bindung darstellen und $Y_2$ für die

Gruppe der Formel —$NH(X_3)$ steht, worin $X_3$ jeweils obige Bedeutung hat, erhalten. Die obigen Reaktionen werden in an sich bekannter Weise durchgeführt.

Falls z. B. $Y_1$ oder $Y_2$ eine reaktionsfähige, veresterte Hydroxygruppe, z. B. Chlor darstellt, arbeitet man in Gegenwart eines basischen Mittels, etwa eines Alkali- oder Erdalkalicarbonats, wie Kalium- oder Calciumcarbonat, oder eines Alkali- oder Erdalkalihydroxids, wie Natrium- oder Calciumhydroxid, zweckmässigerweise in Gegenwart eines geeigneten Lösungsmittels, etwa eines Niederalkanols, wie Aethanol, oder eines Alkanons, wie z. B. Aceton.

Man kann ferner z. B. die durch Umsetzung einer Verbindung der Formel

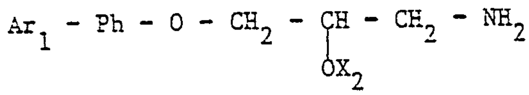

(IIe)

mit einer Carbonylverbindung der Formel

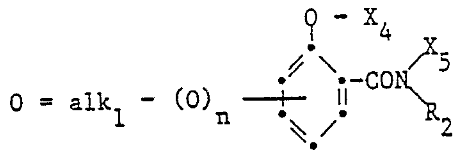

(IIf)

worin $alk_1$ einen dem Rest alk entsprechenden Alkylidenrest, und mindestens einer der Reste $X_2$, $X_4$ oder $X_4$ und $X_5$ zusammen eine der angegebenen Schutzgruppen darstellt, oder mindestens $Ar_1$ einen Rest Ar darstellt, in welchem mindestens eine funktionelle Gruppierung in geschützter Form vorliegt, gebildete Schiff'sche Base mit einem Reduktionsmittel, z. B. einem Borhydrid, etwa Natriumborhydrid, zur Verbindung der Formel II reduzieren. Die Reduktion kann auch mittels aktiviertem Wasserstoffs, z. B. Wasserstoff in Gegenwart eines Hydrierkatalysators, z. B. eines Platin-auf-Kohle-Katalysators erfolgen.

Carbonylverbindungen der Formel (IIf), in der n 1 ist, wiederum können durch Umsetzung einer Verbindung der Formel

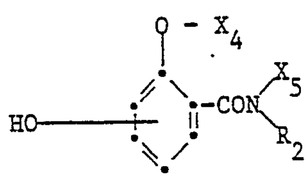

(IIg)

oder eines Salzes davon mit einer Verbindung der Formel ($O=alk_1$)—Hal (IIh), worin $alk_1$ obige Bedeutung hat, Hal für Halogen steht und eine solche Verbindung z. B. ein Halogenketon, etwa Chloraceton darstellt, in üblicher Weise erhalten werden.

Phenole der Formel IIa wiederum sind erhältlich, indem man in einer Verbindung der Formel

$$R_3—Ph—OH \qquad (IIi),$$

worin $R_3$ einen zur Bildung eines mono- oder bicyclischen Heteroarylrestes $Ar_1$ befähigten, gegebenenfalls Schutzgruppen enthaltenden Substituenten darstellt, und die phenolische Hydroxygruppe gegebenenfalls in geschützter, wie verätherter, z. B. als Methoxy, veresterter, z. B. als Acetyloxy, oder silylierter Form, z. B. als Trimethylsilyloxygruppe vorliegt, die mono- oder bicyclische Heteroarylgruppe $Ar_1$ bildet und gegebenenfalls vorhandene Schutzgruppen gleichzeitig oder nachfolgend abspaltet und durch Wasserstoff ersetzt.

So können Verbindungen der Formel IIa, worin Ar die angegebene Bedeutung hat, und z. B. einen Imidazolyl-, Benzimidazolyl-, Pyrrolyl-, Indolyl-, Pyrazolyl-, Thiazolyl-, Thiadiazolyl-, Pyrimidinyl- oder Triazolylrest bedeutet, aus einer Verbindung der Formel IIi analog den für die Bildung eines entsprechenden Heteroarylrestes Ar in einer Verbindung der Formel VIII beschriebenen Methoden in üblicher Weise erhalten werden.

Die neuen Verbindungen der Formel I können ebenfalls erhalten werden, indem man eine Verbindung der Formel

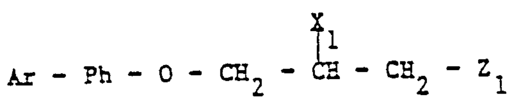

(III)

mit einer Verbindung der Formel

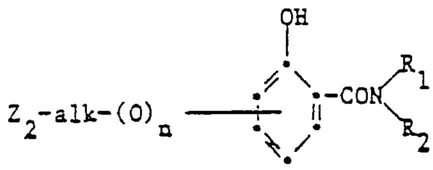

(IV)

oder einem Salz davon, worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, und Ar, Ph, n, alk, $R_1$ und $R_2$ obige Bedeutung haben, umsetzt, und, wenn erwünscht, die zusätzlichen Verfahrensschritte durchführt.

Eine reaktionsfähige veresterte Hydroxygruppe $Z_1$ bzw. $Z_2$ ist eine durch eine starke Säure, insbesondere eine starke anorganische Säure, wie eine Halogenwasserstoffsäure, insbesondere Chlor-, Brom- oder Jodwasserstoffsäure, oder Schwefelsäure, oder eine starke organische Sulfonsäure, wie eine aliphatische oder aromatische Sulfonsäure, z. B. Methansulfonsäure, 4-Methylphenylsulfonsäure oder 4-Bromphenylsulfonsäure, veresterte Hydroxygruppe, und stellt in erster Linie Halogen, z. B. Chlor, Brom oder Jod, oder aliphatisch oder aromatisch substituiertes Sulfonyloxy, z. B. Methylsulfonyloxy oder 4-Methylphenylsulfonyloxy dar.

Die obige Reaktion wird in an sich bekannter Weise durchgeführt, wobei man, besonders bei Verwendung eines Ausgangsmaterials mit einer reaktionsfähigen veresterten Hydroxygruppe, vorteilhafterweise in Gegenwart eines basischen Mittels, wie einer anorganischen Base, z. B. eines Alkalimetall- oder Erdalkalimetallcarbonats oder -hydroxids, oder eines organischen basischen Mittels, wie eines Alkalimetallniederalkanolats, und/oder eines Ueberschusses des basischen Reaktionsteilnehmers und üblicherweise in Gegenwart eines Lösungsmittels oder Lösungsmittelgemisches und, wenn notwendig, unter Kühlen oder Erwärmen, z. B. in einem Temperaturbereich von etwa — 20 °C bis etwa + 150 °C, in einem offenen oder geschlossenen Gefäss und/oder in einer Inertgasatmosphäre, z. B. in einer Stickstoffatmosphäre, arbeitet.

Ausgangsstoffe der Formel III sind bekannt oder können in an sich bekannter Weise hergestellt werden. So können diese erhalten werden, indem man z. B. ein Phenol der

Ar—Ph—OH (IIIa)

oder ein Salz davon, mit einer Verbindung der Formel

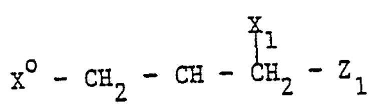

(IIIb)

worin $X^0$ eine geeignete Abgangsgruppe z. B. eine reaktionsfähige veresterte Hydroxygruppe, wie Halogen, z. B. Chlor, und vorzugsweise $X_1$ und $Z_1$ zusammen Epoxy bedeuten, umsetzt.

Phenole der Formel IIIa wiederum sind in üblicher Weise, z. B. auf für die Herstellung von Phenolen der oben erläuterten Formel IIa analoge Weise erhältlich.

Ausgangsstoffe der Formel IV, worin $Z_2$ Halogen und n die Zahl 1 bedeutet, können z. B. durch Umsetzung eines Hydroxysalicylamids mit einem der Bedeutung von alk entsprechenden Dihalogenalkan, etwa einem Chlorbrom- oder Dibromalkan in Gegenwart eines alkalischen Kondensationsmittels, wie einem Alkalicarbonat, hergestellt werden. Hieraus kann z. B. durch Umsetzung mit Hexamethylentetramin und Zersetzung des gebildeten Addukts mit einer wässrigen Mineralsäure, z. B. verdünnter Salzsäure, ein Ausgangsmaterial der Formel III erhalten werden, worin $Z_2$ primäres Amino ist. Diese Umsetzungen werden in üblicher Weise vorgenommen.

Ausgangsstoffe der Formel IV, worin $Z_2$ Halogen bedeutet, und n für die Zahl 0 steht, können z. B. durch Umsetzung eines Phenols der Formel

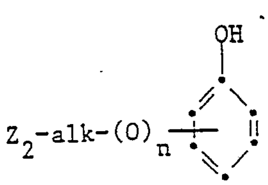

(IVa)

oder eines Alkalisalzes, z. B. des Natriumsalzes, mit Kohlendioxid unter den Bedingungen der Kolbesynthese zur entsprechend substituierten Salicylsäure, und deren Umwandlung, etwa über das mittels Thionylchlorid in üblicher Weise erhältliche Säurechlorid, in das den Resten $R_1$ und $R_2$ entsprechende substituierte Salicylamid in an sich bekannter Weise erhalten werden.

Die neuen Verbindungen können ebenfalls erhalten werden, indem man in einer Verbindung der

Formel

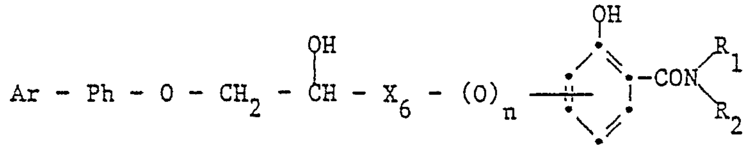

(V)

worin $X_6$ eine reduzierbare Gruppe der Formel

$$—CH=N—alk— \quad (Va) \quad oder \quad —CH_2—N=alk_1— \quad (Vb)$$

darstellt, wobei $alk_1$ für einen dem Rest alk entsprechenden Alkylylidenrest steht, und n für die Zahl 0 oder 1 steht, $X_6$ zur Gruppe der Formel

$$—CH_2—NH—alk— \quad (Vc)$$

reduziert, gegebenenfalls vorhandene, an der Umsetzung nicht teilnehmende, an funktionellen Gruppen stehende Schutzgruppen gleichzeitig oder nachfolgend abspaltet und durch Wasserstoff ersetzt, und, wenn erwünscht, die zusätzlichen Verfahrensschritte durchführt. Schutzgruppen von geschützten funktionellen Gruppen, z. B. solche der vorhin genannten Art, wie geschützte Hydroxy- und/oder Aminogruppen, sind in erster Linie hydrogenolytisch abspaltbare Gruppen, z. B. eine α-Arylniederalkylgruppe, wie eine gegebenenfalls substituierte 1-Phenylniederalkylgruppe, worin Substituenten, etwa des Phenylrestes, z. B. Niederalkoxy, wie Methoxy sein können, und ganz besonders Benzyl, wobei solche Schutzgruppen im Zuge des beschriebenen Verfahrens gleichzeitig oder nachfolgend in einer getrennten Verfahrensmassnahme abgespalten werden.

Ausgangsstoffe der Formel V mit einer Gruppe $X_6$ der Formel Vb können auch in der isomeren Form von Ring-Tautomeren der Formel

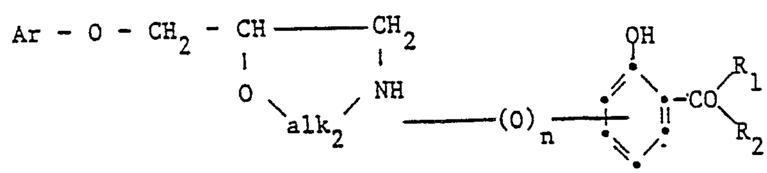

(Vd)

worin $alk_2$ der Bedeutung von $alk_1$ entspricht oder eine Alkylidengruppe darstellt, und das Sauerstoff- und Stickstoffatom des Ringes an das gleiche Kohlenstoffatom gebunden sind, vorliegen.

Eine Alkyl-ylidengruppe alk ist z. B. Methin oder Aethyl-yliden, während eine Alkylidengruppe $alk_2$ z. B. Methylen, Aethyliden oder 1-Methyl-äthyliden darstellt.

Die Reduktion der Stickstoff-Kohlenstoff-Doppelbindung in Ausgangsstoffen der Formel V, die als $X_6$ eine Gruppe Va oder Vb enthalten, zur Stickstoff-Kohlenstoff-Einfachbindung der Gruppe Vc, kann in an sich bekannter Weise, z. B. durch Behandeln mit katalytisch aktiviertem Wasserstoff, wie Wasserstoff in Gegenwart eines geeigneten Hydrierkatalysators, z. B. eines Nickel-, Platin- oder Palladiumkatalysators, erfolgen, wobei hydrogenolytisch abspaltbare Schutzgruppen zugleich abgespalten und durch Wasserstoff ersetzt werden ; oder man arbeitet mit einem geeigneten Hydridreduktionsmittel, wie einem Alkalimetallborhydrid, z. B. Natriumborhydrid. Bei Anwendung eines Hydridreduktionsmittels können an Sauerstoff als Schutzgruppen gebundene Acylreste von Carbonsäuren, wie z. B. der Essigsäure, im gleichen Arbeitsgang abgespalten werden.

Ein Ausgangsmaterial der Formel V kann in an sich bekannter Weise gegebenenfalls *in situ*, d. h. unter den Bedingungen des beschriebenen Verfahrens, hergestellt werden. So kann man eine Verbindung der Formel

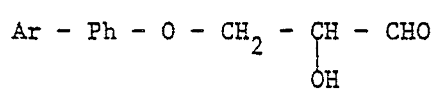

(Ve)

mit einem Amin der Formel

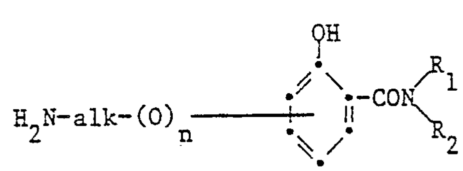

(Vf)

wobei funktionelle Gruppen z. B. Hydroxygruppen gegebenenfalls, z. B. wie beschrieben, in geschützter Form vorliegen, zu einem Ausgangsprodukt der Formel V mit der Gruppe $X_6$ der Formel Va umgesetzt werden.

Durch Umsetzung einer Verbindung der Formel

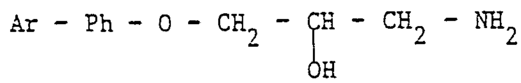 (Vh)

mit einer Carbonylverbindung der Formel

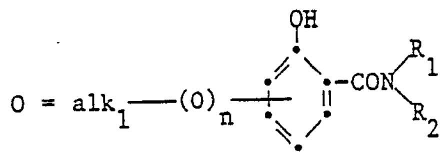 (Vi)

worin $alk_1$ die unter der Formel IIf angegebene Bedeutung hat, und wobei an der Umsetzung nicht beteiligte funktionelle Gruppen, z. B. Hydroxygruppen, gegebenenfalls in geschützter Form, z. B. wie beschrieben, vorliegen, kann man zu Ausgangsstoffen der Formel V gelangen.

Oxoverbindungen der Formel Vi wiederum, worin n für 1 steht, sind z. B. Umsetzung einer Verbindung der Formel

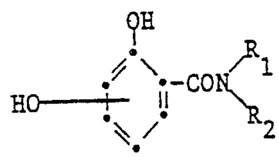 (Vk)

oder eines Salzes davon, mit einer Halogenalkanon-Verbindung der oben erläuterten Formel IIh, z. B. Chloraceton, in Gegenwart eines alkalischen Kondensationsmittels, etwa Kaliumcarbonat, oder einer organischen Base, wie Triäthylamin, erhältlich.

Amine der Formel Vh wiederum werden z. B. durch Umsetzung einer Verbindung der Formel

Ar—Ph—OH (Vl),

oder eines Salzes davon mit Epichlorhydrin zu einer Verbindung der Formel

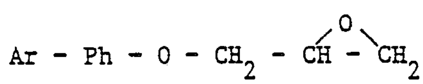 (Vm)

und deren Umsetzung mit Ammoniak in üblicher Weise erhalten. Phenole der Formel VI wiederum sind in für die Herstellung von Ausgangsmaterialien der Formel IIa, entsprechender, etwa wie beschrieben, an sich bekannter Weise zugänglich.

Die neuen Verbindungen der Formel I können ebenfalls erhalten werden, indem man eine Verbindung der Formel

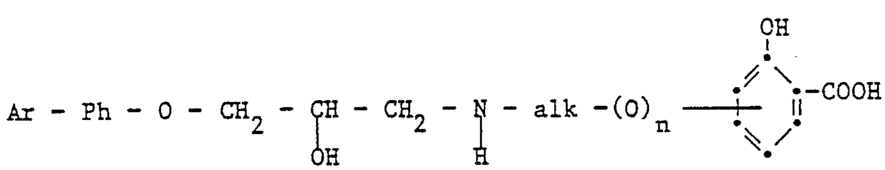 (VI)

oder reaktionsfähiges Derivat einer solchen Carbonsäure, worin an der Umsetzung nicht teilnehmende funktionelle Gruppen gegebenenfalls durch, mittels Aminolyse oder Ammonolyse abspaltbare und durch Wasserstoff ersetzbare Schutzgruppen geschützt sind, mit einer Verbindung der Formel

$HNR_1R_2$ (VIa)

umsetzt, und gleichzeitig oder nachfolgend gegebenenfalls vorhandende Schutzgruppen abspaltet und durch Wasserstoff ersetzt, und, wenn erwünscht, die zusätzlichen Verfahrensschritte durchführt.

Schutzgruppen von geschützten funktionellen Gruppen, z. B. solche der genannten Art, wie an Hydroxy- und/oder Aminogruppen stehende Schutzgruppen, sind aminolytisch und insbesondere ammonolytisch abspaltbare Reste, z. B. Acylreste von organischen Carbonsäuren, z. B. Aroyl, wie

Benzoyl, oder Niederalkanoyl, wie Acetyl.

Reaktionsfähige Derivate der in Formel VI definierten Carbonsäuren sind z. B. Säureanhydride, insbesondere gemischte Säureanhydride, z. B. die Halogenide, wie die Chloride oder Bromide, ferner die Azide oder mit z. B. Niederalkancarbonsäuren, wie Essigsäure oder Propionsäure, Niederalkoxyalkancarbonsäuren, wie 2-Methoxyessigsäure. Reaktionsfähige Derivate von Carbonsäuren der Formel VI sind insbesondere Ester, z. B. Niederalkyl-, wie Methyl- oder tert.-Butylester, ferner mit Arylniederalkanolen, etwa gegebenenfalls durch Niederalkyl, z. B. Methyl oder Niederalkoxy z. B. Methoxy, substituiertem Benzylalkohol, oder Phenolen, die gegebenenfalls durch geeignete Substituenten aktiviert sind, z. B. durch Halogen, etwa 4-Halogen, wie 4-Chlor, Niederalkoxy, etwa 4-Niederalkoxy wie 4-Methoxy, 4-Nitro- oder 2,4-Dinitro, wie etwa 4-Chlorphenol, 2,3,4,5,6-Pentachlorphenol, 4-Methoxyphenol, 4-Nitro- oder 2,4-Dinitrophenol, ferner mit Cycloalkanolen, wie etwa Cyclopentanol der Cyclohexanol, die gegebenenfalls durch Niederalkyl, z. B. Methyl, substituiert sein können. Die Umsetzung wird in an sich bekannter Weise, üblicherweise in Gegenwart eines inerten Lösungsmittels, und in einem Temperaturbereich von etwa —10° bis + 150° in einem geschlossenen Gefäss durchgeführt.

Die Ausgangsstoffe der Formel VI lassen sich in an sich bekannter Weise erhalten, indem man eine Verbindung der Formel (III), in der $X_1$ und $Z_1$ zusammen die Epoxygruppe bedeuten, mit einer Aminoverbindung der Formel

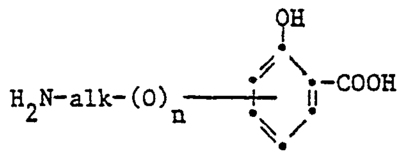

(VIb)

oder einem reaktionsfähigen Derivat einer solchen Carbonsäure, z. B. wie unter Formel VI beschrieben, umsetzt.

Man kann ferner die durch Umsetzung einer Verbindung der vorhin erläuterten Formel Vh mit einer Carbonylverbindung der Formel

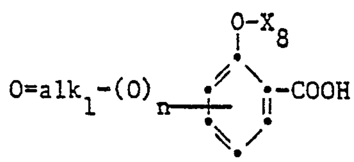

(VIc)

oder einem reaktionsfähigen Carbonsäurederivat davon, z. B. wie unter Formel VI beschrieben, worin $alk_1$ die unter Formel IIf angegebene Bedeutung hat, gebildete Schiff'sche Base mit einem Borhydrid, etwa Natriumborhydrid, reduzieren. Die Reduktion kann auch mittels aktiviertem Wasserstoff in Gegenwart eines Hydrierkatalysators, z. B. eines Platin-auf-Kohle-Katalysators, erfolgen.

Carbonylverbindungen der Formel (VIc) wiederum, worin n 1 bedeutet, können durch Umsetzung einer Verbindung der Formel

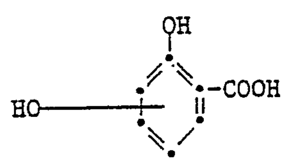

(VId)

oder einem reaktionsfähigen Carbonsäurederivat davon, z. B. wie unter Formel VI beschrieben, mit einer Verbindung der oben erläuterten Formel IIh, z. B. Chloraceton, in Gegenwart eines alkalischen Mittels, etwa Kaliumcarbonat, oder einer organischen Base, wie Triäthylamin, erhalten werden. Bei diesen Umsetzungen liegen hieran nicht beteiligte funktionelle Gruppen, z. B. Hydroxygruppen gegebenenfalls in geschützter, Form, z. B. wie beschrieben, vor. Diese Reaktionen werden in an sich bekannter Weise vorgenommen.

Die neuen Verbindungen der Formel I können ebenfalls erhalten werden, indem man in einer Verbindung der Formel

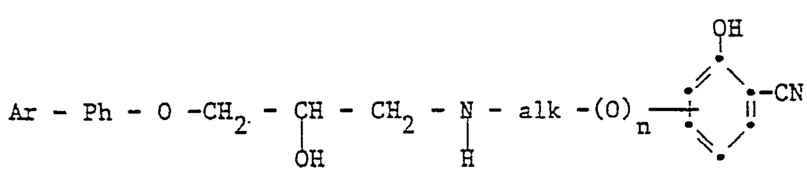

(VII)

worin an der Umsetzung nicht teilnehmende, funktionelle Gruppen gegebenenfalls durch mittels Hydrolyse abspaltbare und durch Wasserstoff ersetzbare Schutzgruppen geschützt sind, die unter den

Bedingungen der Verfahrens abgespalten und durch Wasserstoff ersetzt werden, die Gruppe —CN mittels Hydrolyse in die Gruppe —$CONH_2$ umwandelt, und gleichzeitig gegebenenfalls vorhandene Schutzgruppen abspaltet und durch Wasserstoff ersetzt, und, wenn erwünscht, die zusätzlichen Verfahrensschritte durchführt.

Die obigen Reaktionen werden in an sich bekannter Weise durchgeführt. Die Hydrolyse wird in einem basischen oder vorteilhafterweise in einem sauren Medium, insbesondere in Gegenwart konzentrierter wässriger Mineralsäure, wie z. B. konz. Salzsäure, und wenn notwendig, unter Kühlen oder Erwärmen, z. B. in einem Temperaturbereich von etwa 0 bis 60°, vorzugsweise von etwa 40-50°, in einem offenen oder geschlossenen Gefäss und/oder in einer Inertgasatmosphäre, z. B. in einer Stickstoffatmosphäre durchgeführt.

Die Ausgangsstoffe der Formel VII lassen sich z. B. durch Umsetzen einer Verbindung der vorhin erläuterten Formel III mit einer Verbindung der Formel

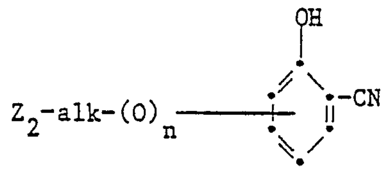

(VIIa)

worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, und Ar, Ph, n, alk, $R_1$ und $R_2$ obige Bedeutung haben, herstellen. Reaktionsfähige veresterte Hydroxygruppen $Z_1$ bzw. $Z_2$ sind vorzugsweise Halogen, insbesondere Chlor, Brom oder Jod. Die Umsetzung wird, falls $Z_1$ bzw. $Z_2$ eine reaktionsfähige veresterte Hydroxygruppe darstellen, vorteilhafterweise in Gegenwart eines basischen Mittels in an sich bekannter Weise durchgeführt.

Die Verbindung VIIa wiederum kann durch Einwirkung von Essigsäureanhydrid auf das dem Cyanid entsprechende Oxim erhalten werden. Dies geschieht zweckmässigerweise durch Kochen unter Rückfluss. Das Oxim kann seinerseits aus dem entsprechenden Aldehyd duch Kochen mit Hydroxylaminhydrochlorid in Gegenwart von alkoholischer Sodalösung unter Rückfluss hergestellt werden. Der entsprechende Aldehyd wiederum kann durch Umsetzung von 2,4-Dihydroxybenzaldehyd mit einem $\alpha,\omega$-Dihalogenniederalkan, vorzugsweise in Gegenwart eines basischen Mittels hergestellt werden. Man kann aber auch in analoger Weise ein Hydroxysalicylonitril, z. B. das 2,4-Dihydroxybenzonitril [Chem. Ber. 24, 3657 (1891)] oder das 2,5-Dihydroxybenzonitril [Helv. Chim. Acta *30*, 149, 153 (1947)] mit einem nichtgeminalen Dihalogenniederalkan zu einer Verbindung der Formel VIIa umsetzen.

Die neuen Verbindungen der Formel I, worin Ar einen mono- oder bicyclischen Heteroarylrest darstellt, können ebenfalls erhalten werden, indem man in einer Verbindung der Formel

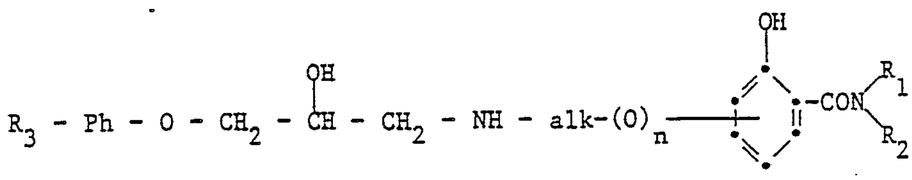

(VIII)

worin Hydroxygruppen und/oder die Aminogruppe durch Schutzgruppen geschützt sind, und $R_3$ einen zur Bildung eines mono- oder bicyclischen Heteroarylrestes Ar befähigten Substituenten darstellt, die mono- oder bicyclische Heteroarylgruppe Ar bildet, gegebenenfalls vorhandene Schutzgruppen gleichzeitig oder nachfolgend abspaltet und durch Wasserstoff ersetzt, und, wenn erwünscht, die oben genannte zusätzlichen Verfahrensschritte durchführt. Schutzgruppen sind z. B. Acyl, wie Niederalkanoyl, wie Acetyl, die unter den zur Bildung des Heteroarylrestes Ar angewendeten Verfahrensbedingungen abgespalten und durch Wasserstoff ersetzt werden.

So kann man zur Herstellung einer Verbindung der Formel I, worin Ar einen Imidazolylrest bedeutet, der z. B. in 2-Stellung durch die substituierte Gruppe Ph substituiert ist, eine Verbindung der Formel VIII, worin $R_3$ die Formylgruppe darstellt, in üblicher Weise mit einer 1,2-Dicarbonylverbindung der Formel

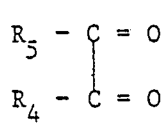

(VIIIa)

worin $R_4$ und/oder $R_5$ jeweils Wasserstoff oder einen die Heteroarylgruppe substituierenden Rest, z. B. wie oben beschrieben, darstellt in Gegenwart von Ammoniak oder mit einer der Formel VIIIa entsprechenden Monooximino-Verbindung umsetzten. Je nach Art des Restes $R_4$ und/oder $R_5$ kann man die

Verbindung der Formel VIIIa als solche einsetzen oder sie kann im Reaktionsgemisch aus einer geeigneten Vorstufe *in situ* gebildet werden. Hierzu geeignete Vorstufen stellen z. B. Acyloine dar, die sich vom Hydroxyaceton ableiten, und stellen z. B. 1-Niederalkanoyloxy-, wie 1-Acetyloxyaceton oder 1,1-Dihalogen-, wie z. B. 1,1-Dibromaceton dar, welches gegebenenfalls in 1 und/oder 3-Stellung durch die Gruppe $R_4$ und/oder $R_5$ substituiert ist. Weitere Vorstufen sind solche, in denen die Carbonylverbindung der Formel VIIIa in abgewandelter Form, z. B. als Acetal, etwa als Dimethylacetal, als Hydrat oder als Bisulfitadditionsverbindung, vorliegt. Aus solchen Vorstufen kann im Falle der Acyloine, wie dem genannten 1-Acetyloxyaceton, in Gegenwart eines Kupfer-(II)-salzes, z. B. Kupfer-(II)-acetat, oder im Falle der 1,1-Dihalogenacetonderivate durch Behandlung mit hydrolysierenden Mitteln, vorzugsweise schwachen Alkalien, z. B. Natriumacetat, oder im Falle der Acetale oder Bisulfitadditionsverbindungen mit wässrigen Säuren, die Verbindung der Formel VIIIa *in situ* erhalten werden. Auf diese Weise erhält man Verbindungen der Formel I, worin die substituierte Gruppe Ph eine 2-Stellung, eine Gruppe $R_4$ und/oder $R_5$ jeweils die 4- bzw. 5-Stellung des gebildeten Imidazolrestes substituieren.

Verbindungen der Formel I, worin ein Imidazolrest Ar z. B. in 4-Stellung durch die substituierte Gruppe Ph substituiert ist, kann man z. B. durch Umsetzung einer Verbindung der Formel VIII, worin $R_3$ die Gruppe

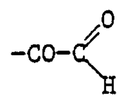
(VIIIb)

darstellt, mit einer Verbindung der Formel

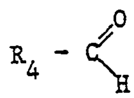
(VIIIc)

und Ammoniak erhalten. Hierbei können die Gruppen der Formel VIIIb oder VIIIc auch in funktionell abgewandelter Form, z. B. als Acetale, wie Dimethylacetale, als Hydrate oder als Bisulfitadditionsverbindungen vorliegen, aus denen durch Behandeln mit wässrigen Säuren die freien Verbindungen *in situ* gebildet und anschliessend im gleichen Reaktionsansatz weiter umgesetzt werden. Diese Umsetzungen werden in üblicher Weise, z. B. in Gegenwart eines Lösungsmittels oder Lösungsmittelgemisches, und, wenn notwendig, unter Kühlen oder Erwärmen, z. B. in einem Temperaturbereich von etwa —30° bis etwa + 150 °C, falls notwendig in einem geschlossenen Gefäss und/oder in einer Inertgasatmosphäre, z. B. unter Stickstoff, durchgeführt.

Die Ausgangsstoffe können in an sich bekannter Weise hergestellt werden.

So lassen sich Verbindungen der Formel VIII, worin $R_3$ die Formylgruppe oder die Gruppe der Formel VIIIb darstellt, durch Umsetzung einer Verbindung der Formel $R_3$—Ph—OH (VIIId), worin die dem Rest $R_3$ entsprechende Formylgruppe oder die Gruppe der Formel VIIIb vorzugsweise in geschützter Form, z. B. als Acetal, wie Dimethylacetal, als Hydrat oder als Bisulfitadditionsverbindung, z. B. der mit Natriumsulfit gebildeten, vorliegen, mit Epichlorhydrin zur entsprechenden 2,3-Epoxypropoxy-Verbindung und deren weitere Umsetzung mit einer Verbindung der Formel

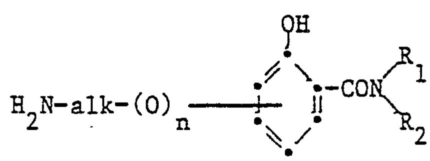
(VIIIe)

z. B. mit 5-(2-Aminoäthoxy)-salicylamid, erhalten. Ein hierbei aus der Formylgruppe bzw. der Gruppe der Formel VIIIb und einer Verbindung der Formel VIIIe gebildetes Zwischenprodukt, z. B. eine Schiff'sche Base, wird anschliessend hydrolysiert, z. B. mit sauren Mitteln, wie einer Mineralsäure, z. B. wässriger Salzsäure, wonach die Verbindung der Formel VIII erhalten wird.

Anstelle der beschriebenen Umsetzung kann man ferner eine Verbindung der Formel VIIId, worin die Gruppe $R_3$ die Formylgruppe oder die Gruppe der Formel VIIIb darstellt, und die vorzugsweise in geschützer Form, z. B. in Acetalform vorliegt, oder ein Salz davon, z. B. ein Alkalimetall- oder Erdalkalimetall-, z. B. ein Natrium- oder Calciumsalz, mit einer gegebenenfalls reaktionsfähigen veresterten Oxazolidinverbindung der Formel

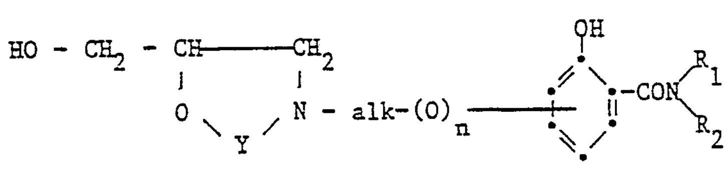
(VIIIf)

umsetzen, worin Y die Gruppe der Formel

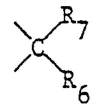 (VIIIg)

bedeutet, wobei $R_6$ und $R_7$ jeweils Wasserstoff ist, oder die Gruppe der Formel VIIIg gegebenenfalls substituiertes 1-Phenyl-niederalkyliden bedeutet, wobei Substituenten, insbesondere des Phenylteils z. B. Niederalkyl oder Niederalkoxy sein können, und insbesondere Benzyliden oder Cycloalkyliden, z. B. Cyclopentyliden oder Cyclohexyliden, oder die Gruppe Y die Carbonylgruppe

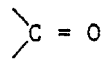 (VIIIh)

darstellt, und die erhaltene Verbindung der Formel

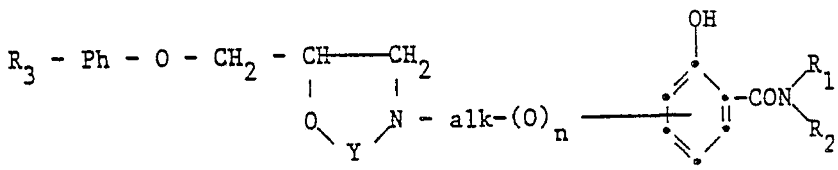 (VIIIi)

hydrolysieren. Eine reaktionsfähige veresterte Verbindung der Formel VIIIf ist eine solche, worin die primäre Alkoholgruppe mit einer geeigneten Säure, z. B. wie beschrieben, etwa einer Halogenwasserstoffsäure wie Salzsäure, oder einer starken organischen Sulfonsäure, wie z. B. p-Toluolsulfonsäure, verestert ist. Die Hydrolyse einer Verbindung der Formel VIIIi wird mittels Wasser gegebenenfalls in Gegenwar sauer wirkender Mittel, wie z. B. einer Mineralsäure, wie Salzsäure oder Schwefelsäure, vorgenommen, wobei die saure Hydrolyse insbesondere bei Verbindungen der Formel VIIIi mit einer Gruppe der Formel VIIIg angewendet wird, wobei gleichzeitig gegebenenfalls vorhandene Schutzgruppen abgespalten und durch Wasserstoff ersetzt werden können. Verbindungen der Formel VIIIi mit einer Gruppe Y der Formel VIIIh werden vorteilhafterweise in Gegenwart von alkalisch reagierenden Stoffen, z. B. starken Basen, wie Alkalihydroxiden, z. B. Natriumhydroxid, hydrolysiert, wobei es zweckmässig sein kann, ein höher siedendes Lösungsmittel, z. B. Butanol, zu verwenden. Gegebenenfalls vorhandene Schutzgruppen können gleichzeitig oder anschliessend im sauren Medium, etwa wie beschrieben, abgespalten werden.

Diese Umsetzungen werden in an sich bekannter Weise vorgenommen.

Verbindungen der Formel VIIIf wiederum, worin Y den Rest der Formel VIIIg bedeutet, können aus einer Verbindung der Formel

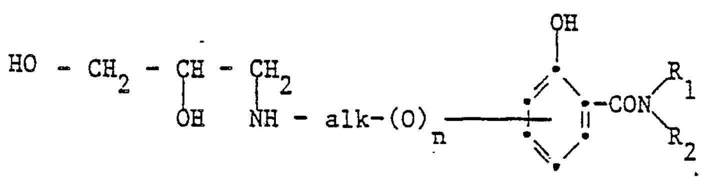 (VIIIk)

durch Umsetzung mit einer Oxoverbindung der Formel

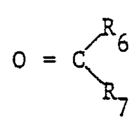 (VIIIl)

wie z. B. Formaldehyd oder Benzaldehyd, oder deren reaktionsfähigen Derivaten, z. B. den Acetalen, wie den Dimethylacetalen, in üblicher Weise erhalten werden, während Verbindungen der Formel VIIIf, worin Y den Rest der Formel VIIIh darstellt, durch Einwirkung von Phosgen auf eine Verbindung der Formel VIIIk, worin die primäre Hydroxygruppe in reaktionsfähiger, veresterter Form vorliegt, in bekannter Weise zugänglich sind.

Ausgangsstoffe der Formel VIIIf schliesslich sind durch Umsetzung einer Verbindung der Formel

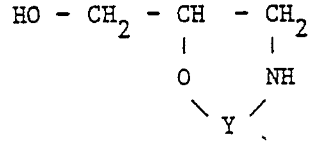 (VIIIm)

mit einer Verbindung der Formel

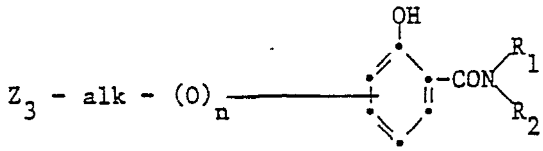

(VIIIn)

worin $Z_3$ eine reaktionsfähige veresterte Hydroxygruppe, etwa wie oben definiert, z. B. Halogen, wie Chlor oder Brom, darstellt, in üblicher Weise, vorzugsweise in Gegenwart eines alkalischen Kondensationsmittels, z. B. Kaliumcarbonat, und in Abwesenheit von Wasser, zugänglich, wobei gegebenenfalls vorhandene Schutzgruppen gleichzeitig oder nachfolgend abgespalten und durch durch Wasserstoff ersetzt werden.

Eine Verbindung der Formel I, worin Ar den Thiazolylrest bedeutet, der z. B. in 2-Stellung durch die substituierte Gruppe Ph substituiert ist, kann erhalten werden, indem man z. B. eine Verbindung der Formel VIII, worin $R_3$ die Gruppe der Formel

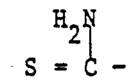

(IXa)

bedeutet, mit einer Verbindung der Formel

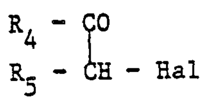

(IXb)

worin Hal für Halogen, z. B. für Chlor und insbesondere für Brom steht, und $R_4$ und/oder $R_5$ jeweils Wasserstoff oder einen die Heteroarylgruppe substitutierten Rest, z. B. wie oben beschrieben, darstellt, umsetzt. Je nach Art des Restes $R_4$ und/oder $R_5$ kann man eine Verbindung der Formel IXb in Form ihrer Derivate, z. B. der Acetale, etwa der Diäthylacetale, oder der α,β-Dihalogen-, wie α,β-Dibromäthyläther, z. B. α,β-Dibromäthyl-methyläther, oder der α,β-Dihalogen-, wie α,β-Dichloräthylacetate einsetzen. Anstelle einer Carbonylverbindung der Formel IXb kann man ferner ein Epoxid der Formel

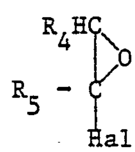

(IXc)

worin Hal für Halogen, z. B. Chlor, steht für die beschriebene Umsetzung verwenden.

Zur Herstellung von Verbindungen der Formel I, worin Ar einen in 4- oder 5-Stellung durch die substituierte Gruppe Ph substituierten Thiazolylrest darstellt, kann man z. B. eine Verbindung der Formel VIII, worin $R_3$ die Gruppe der Formel

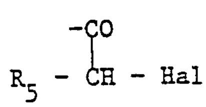

(IXd)

oder der Formel

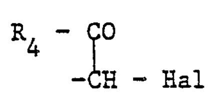

(IXe)

ist, und die Carbonylgruppe gegebenenfalls in geschützer, z. B. Acetalform vorliegt, worin Hal jeweils für Halogen, z. B. für Chlor oder Brom steht, und $R_4$ und $R_5$ obige Bedeutungen haben, mit einer Verbindung der Formel

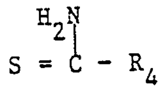

(IXf)

umsetzen. Diese Reaktionen werden in an sich bekannter Weise, z. B. in Gegenwart eines geeigneten

Lösungsmittels, z. B. eines Niederalkanols, wie Aethanol, oder einer ätherartigen Flüssigkeit, wie Dioxan, gegebenenfalls bei erhöhter Temperatur und gegebenenfalls in Anwesenheit eines basischen Mittels, z. B. eines Metall-, wie Alkali- oder Erdalkalisalzes einer schwachen Säure, wie Kohlensäure oder Essigsäure, z. B. Magnesiumcarbonat oder Natriumacetat, und, falls notwendig, in einem geschlossenen Gefäss und/oder in einer Inertgasatmosphäre, z. B. unter Stickstoff durchgeführt.

Die Ausgangsstoffe lassen sich nach bekannten Methoden herstellen. So kann man eine Verbindung der Formel VIII mit der Gruppe IXa als $R_3$ erhalten, indem man in einer Verbindung der Formel

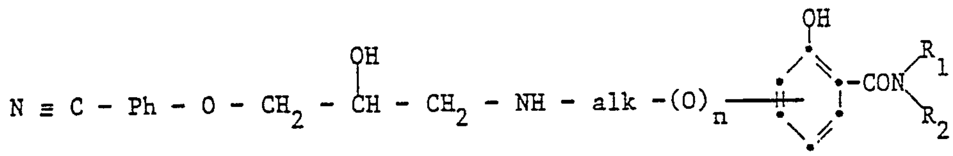

(IXg)

die Gruppe N≡C— durch Umsetzung mit Schwefelwasserstoff in einem geeigneten, z. B. einem basischen organischen Lösungsmittel, wie z. B. Pyridin oder Triäthylamin oder Gemischen davon, in die Gruppe IXa umwandeln.

Ausgangsstoffe der Formel IXg wiederum können erhalten werden, indem man eine Verbindung der Formel

N≡C—Ph—OH (IXh)

mit Epichlorhydrin zur entsprechenden 2,3-Epoxypropoxy-Verbindung umsetzt und aus dieser Verbindung durch Umsetzung mit einer Verbindung der oben erläuterten Formel VIIIe, z. B. mit 5-(2-Aminoäthoxy)-salicylamid, eine Verbindung der Formel IXg herstellt.

Ausgangsstoffe der Formel VIII, worin $R_3$ für eine Gruppe der Formel IXd oder IXe steht, können z. B. durch Umsetzung eines Phenols der Formel

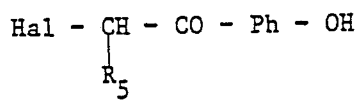

(IXi)

oder der Formel

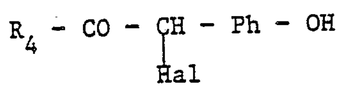

(IXk)

worin Hal für Halogen und insbesondere für Chlor oder Brom steht, mit einer Verbindung der Formel

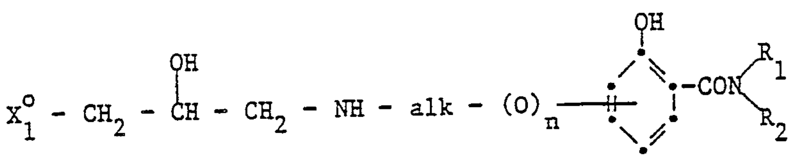

(IXl)

oder einer entsprechenden Oxazolidin- oder Oxazolidinonverbindung der Formel

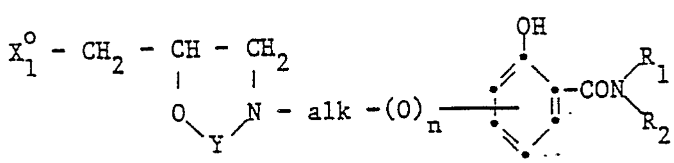

(IXm)

worin Y die unter der Formel VIIIf angegebene Bedeutung hat, und $X_1^0$ eine geeignete Abgangsgruppe, wie Halogen, z. B. Chlor oder insbesondere Brom, oder einen Sulfonyloxyrest, z. B. den p-Tosyloxyrest darstellt, und eine Verbindung der Formel IXl, beispielsweise 5-(2-Bromäthoxy)-salicylamid ist, umsetzt. Vorteilhafterweise arbeitet man in Gegenwart eines basischen Mittels, wie einer anorganischen Base, z. B. eines Alkalimetall- oder Erdalkalimetallcarbonats oder -hydroxids, oder eines organischen basischen Mittels, wie eines Alkalimetallniederalkanolats, wie Natriummethoxid und üblicherweise in Gegenwart eines Lösungsmittels oder Lösungsmittelgemisches. Hierbei verwendet man vorteilhafterweise polare Lösungsmittel, z. B. ein Niederalkanol wie Aethanol, oder ein gegebenenfalls N-niederalkyliertes Fettsäureamid, wie Dimethylformamid oder N-Methylacetamid, Dimethylsulfoxid oder Sulfolan oder Gemische solcher Lösungsmittel.

Sofern für die Umsetzung eine Verbindung der Formel IXm verwendet wurde, schliesst sich hieran

die Umwandlung der erhaltenen Verbindung in ein Ausgangsmaterial der Formel VIII mit der Gruppe IXd oder IXe als $R_3$ an, indem man das Reaktionsprodukt der Hydrolyse unterwirft. Diese wird in üblicher Weise mittels Wasser, gegebenenfalls unter Zusatz von sauer reagierenden Stoffen, wie einer Mineralsäure, z. B. Salzsäure, oder einer Base, wie Z. B. eines Alkali- oder Erdalkalihydroxids, durchgeführt. Ausgangsstoffe der Formel IXI wiederum sind z. B. durch Umsetzung einer Verbindung der Formel VIIIe mit Epichlorhydrin in üblicher Weise, z. B. in einem Lösungsmittel, etwa einem Niederalkanol, erhältlich, während eine Verbindung der Formel IXm durch Umsetzung einer Verbindung der oben erläuterten Formel VIIIn mit einer Verbindung der Formel

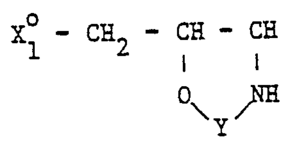

(IXn)

zugänglich ist. Diese Umsetzungen werden in üblicher Weise, z. B. in einem geeigneten Lösungsmittel, etwa einem Niederalkanol, oder einem stark polaren nicht-wässrigen Lösungsmittel, wie einem gegebenenfalls niederalkylierten Fettsäureamid, wie Dimethylformamid, N-Methyl- oder N,N-Dimethylacetamid, ferner Dimethylsulfoxid, Sulfolan oder N,N,N',N'-Tetramethylharnstoff, zweckmässigerweise in Gegenwart eines basischen Mittels, etwa eines Alkalihydroxids, wie Natriumhydroxid, oder Natriumhydrid vorgenommen.

Eine Verbindung der Formel I, worin Ar einen Pyrimidinylrest bedeutet, der z. B. in 4(6)-Stellung durch die substituierte Gruppe Ph substituiert ist, kann erhalten werden, indem man z. B. eine Verbindung der Formel VIII, worin $R_3$ die Gruppe der Formel

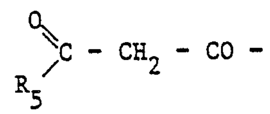

(Xa)

darstellt, worin die Carbonylgruppe(n) gegebenenfalls in geschützter, z. B. acetalisierter Form, z. B. als Dimethylacetal, oder als Bisulfitadditionsverbindung, z. B. mit Natriumbisulfit, vorliegen, mit einer Verbindung der Formel

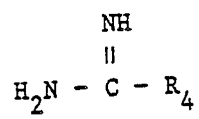

(Xb)

worin $R_4$ und $R_5$ obige Bedeutungen haben, umsetzt.

Diese Reaktion wird in üblicher Weise und vorzugsweise unter sauren Bedingungen, z. B. in Gegenwart eines sauer reagierenden Mittels, wie z. B. Ammoniumchlorid, vorgenommen.

Die Ausgangsstoffe können nach an sich bekannten Methoden erhalten werden.

So kann man z. B. ein Ausgangsmaterial der Formel VIII mit der Gruppe Xa als $R_3$ herstellen, indem man eine Verbindung der Formel

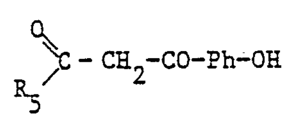

(Xc)

mit vorzugsweise geschützter, z. B. wie beschrieben, acetalisierter Carboxaldehydgruppe mit Epichlorhydrin in Gegenwart einer Base, z. B. eines organischen Amins, z. B. Pyridin oder Collidin zu einer entsprechenden Verbindung der Formel

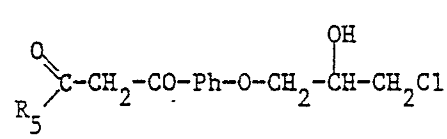

(Xd)

umsetzt, hieraus das durch Umsetzung mit Hexamethylentetramin in üblicher Weise erhaltene Addukt durch Zersetzung mittels wässriger Mineralsäure, z. B. verdünnter Schwefelsäure, in eine entsprechende Verbindung der Formel

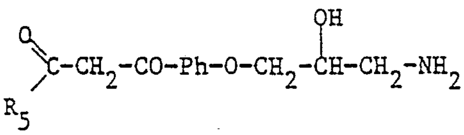

(Xe)

umwandelt, welche anschliessend mit einer Verbindung der oben erläuterten Formel VIIIn in analoger Weise, z. B. wie dort beschrieben, umgesetzt wird, und nach Umwandlung der gegebenenfalls geschützten Carboxaldehydgruppe in die freie Carboxaldehydgruppe, z. B. mittels saurer Mittel, wie wässriger Mineralsäure, z. B. verdünnter Schwefelsäure, zu einem Ausgangsmaterial der Formel VIII mit der Gruppe Xa als $R_3$ führt. Diese Umsetzungen werden in an sich bekannter Weise vorgenommen.

Zur Herstellung von Verbindungen der Formel I, worin Ar einen Pyrimidinylrest darstellt, der z. B. in 2-Stellung durch die substituierte Gruppe Ph substituiert ist, kann man z. B. eine Verbindung der Formel VIII, worin $R_3$ die Gruppe der Formel

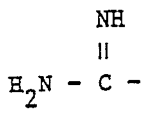

'Xf)

bedeutet, mit einer Verbindung der Formel

$$R_4—CO—CH_2—CO—R_5 \quad (Xg)$$

worin die Carbonylgruppe (n) gegebenenfalls in geschützter, z. B. acetalisierter, Form, etwa als Dimethylacetale, vorliegen und $R_4$ und $R_5$ die gegebenen Bedeutungen haben, umsetzen. Diese Umsetzung wird auf an sich bekannte Weise, z. B. unter sauren Bedingungen, etwa wie beschrieben, z. B. in Gegenwart von Ammoniumchlorid, vorgenommen.

Die Ausgangsstoffe können nach an sich bekannten Methoden hergestellt werden. So kann man z. B. eine Verbindung der Formel VIII mit der Gruppe Xf als $R_3$ erhalten, indem man z. B. eine Verbindung der oben erläuterten Formel (IXg) in den entsprechenden Imidoester z. B. durch Sättigen einer absolutäthanolischen Lösung einer solchen Verbindung mit Chlorwasserstoff, umwandelt und hieraus durch Umsetzung mit wasserfrieem Ammoniak eine die Gruppe Xf als $R_3$ enthaltende Verbindung der Formel VIII, vorzugsweise in Form eines Salzes, etwa mit einer starken Säure, z. B. als Hydrochlorid, erhält, und gegebenenfalls vorhandene Schutzgruppen gleichzeitig oder nachfolgend entfernt und durch Wasserstoff ersetzt.

Bei der Auswahl des geeigneten obigen Verfahrens zur Herstellung von Verbindungen der Formel I muss darauf geachtet werden, dass vorhandene Substituenten, in erster Linie der Reste Ar, nicht umgewandelt oder abgespalten werden, falls solche Umwandlungen bzw. Abspaltungen nicht erwünscht sind. So können insbesondere funktionell abgewandelte Carboxylgruppen, wie veresterte oder amidierte Carboxylgruppen, sowie Cyangruppen, als Substituenten der Reste Ar während Solvolysen, insbesondere Hydrolysen, ferner auch bei Reduktionen an der Reaktion beteiligt sein und umgewandelt werden. Andererseits können gleichzeitige Umwandlungen von Substituenten erwünscht sein ; z. B. können ungesättigte Substituenten, wie Niederalkenyl, unter den Bedingungen eines erfindungsgemäss eingesetzten Reduktionsverfahrens, z. B. zu Niederalkyl, reduziert werden.

In erhaltenen Verbindungen kann man im Rahmen der Definition der Verbindungen der Formel I in üblicher Weise verfahrensgemäss erhaltene Verbindungen in andere Endstoffe überführen, z. B. indem man geeignete Substituenten abwandelt, einführt oder abspaltet.

Freie Carboxylgruppen in den Resten Ar lassen sich in üblicher Weise verestern, beispielsweise durch Umsetzen mit einem entsprechenden Alkohol, vorteilhaft in Gegenwart einer Säure, wie einer Mineralsäure, z. B. Schwefelsäure oder Chlorwasserstoffsäure, oder in Gegenwart eines wasserbindenden Mittels, wie Dicyclohexylcarbodiimid, oder durch Umsetzen mit einer entsprechenden Diazoverbindung, z. B. Diazomethan. Die Veresterung kann auch durch Umsetzen eines Salzes, vorzugsweise eines Alkalimetallsalzes der Säure mit einem reaktionsfähigen veresterten Alkohol, z. B. einem entsprechenden Halogenid, wie Chlorid, durchgeführt werden.

Freie Carboxylgruppen lassen sich in üblicher Weise amidideren beispielsweise durch Umsetzen mit Ammoniak, oder mit einem primären oder sekundären Amin, vorteilhaft in Gegenwart eines wasserbindenden Mittels, wie Dicyclohexylcarbodiimid, oder durch Ueberführen der Carboxylgruppe in eine Halogencarbonyl-, z. B. Chlorcarbonylgruppe, und anschliessendem Umsetzen mit Ammoniak oder mit einem primären oder sekundären Amin.

In Verbindungen, die eine veresterte Carboxylgruppe enthalten, kann diese in üblicher Weise, z. B. durch Hydrolyse, vorzugsweise in Gegenwart von starken Basen, wie einem Alkalimetallhydroxyd, z. B. Natrium- oder Kaliumhydroxyd, oder starken Säuren, z. B. einer starken Mineralsäure, wie einer Halogenwasserstoffsäure, z. B. Chlorwasserstoffsäure oder Schwefelsäure, in eine freie Carboxylgruppe übergeführt werden.

In Verbindungen mit einer veresterten Carboxylgruppe als Substituent kann diese in üblicher Weise, z. B. durch Ammonolyse oder Aminolyse mit Ammoniak oder einem primären oder sekundären Amin in die entsprechende Carbamoylgruppe übergeführt werden.

Verbindungen mit einer Carbamoylgruppe und von Wasserstoff verschiedenen Reste $R_1$ und $R_2$ können in üblicher Weise, z. B. durch Einwirkung wasserentziehender Mittel, wie Phosphorpentoxid, Phosphoroxychlorid oder Trifluoressigsäureanhydrid, vorzugsweise bei höheren Temperaturen, zu den entsprechenden Cyanverbindungen dehydratisiert werden.

In Verbindungen mit einer veresterten Carboxylgruppe als Substituent und vorzugsweise von Wasserstoff verschiedenen Resten $R_1$ und $R_2$, kann die veresterte Carboxylgruppe in üblicher Weise, z. B. durch Einwirkung einer organischen, etwa einer Diniederalkylaluminiumamid-Verbindung, z. B. Diäthylaluminiumamid, in eine Cyanogruppe umgewandelt werden.

Verbindungen mit von Wasserstoff verschiedenen Resten $R_1$ und $R_2$, die einen Cyansubstituenten enthalten, können in üblicher Weise, z. B. in Gegenwart konzentrierter wässriger Mineralsäuren oder Alkalimetallhydroxiden, zu den entsprechenden Carbamoyl- oder direkt zu den Carboxylverbindungen hydrolysiert werden.

Verbindungen mit einer Cyangruppe als Substitutenten können in üblicher Weise, z. B. durch Addition von Alkoholen in Gegenwart einer wasserfreien Säure, wie Chlorwasserstoff, und nachträglicher Hydrolyse des entstandenen Imidoesters zu den entsprechenden Verbindungen mit veresterten Carboxylgruppen alkoholysiert werden.

In Verbindungen der Formel I, in denen Ar die Gruppe —NH— als Ringglied enthält, kann man diese durch Einführen eines Substituenten z. B. einer gegebenenfalls substituierten Niederalkylgruppe, wie Methyl oder Benzyl, auf übliche Weise, z. B. unter Verwendung eines entsprechenden reaktionsfähigen veresterten Alkohols, wie eines entsprechenden Halogenids, z. B. Chlorids oder Bromids, oder eines Diazoalkans, z. B. Diazomethan, in solche Verbindungen der Formel I umwandeln, in denen Ar eine tertiäre Aminogruppe als Ringglied enthält.

Verbindungen, worin Ar wenigstens ein tertiäres Stickstoffatom als Ringglied enthält, können auf übliche Weise durch Behandeln mit einer Peroxyverbindung, wie Wasserstoffperoxid, oder einem Säurederivat davon, wie Peressigsäure oder m-Chlorperbenzoesäure, in N-Oxide umgewandelt werden.

Verbindungen, die als N-Oxide vorliegen, können in an sich bekannter Weise in die nicht N-oxidierten Verbindungen umgewandelt werden, z. B. mittels schwefliger Säure, oder Phosphoroxychlorid, oder vor allem mittels Wasserstoff in Gegenwart eines Hydrierkatalysators, z. B. eines Edelmetallkatalysators, wie Platin oder Palladium, ferner auch Raney-Nickel.

Verbindungen, die im aromatischen Ring Ar eine Niederalkylthio-, z. B. Methylthiogruppe, tragen, können durch Behandeln mit geeigneten desulfurierend wirkenden Mitteln, z. B. Raney-Nickel, in einem geeigneten Lösungsmittel, z. B. Dioxan, in die schwefelfreien Verbindungen umgewandelt werden.

Wie bei den Herstellungsverfahren muss auch bei der Durchführung der Zusatzschritte darauf geachtet werden, dass unerwünschte Nebenreaktionen, welche die Umwandlung zusätzlicher Gruppierungen zur Folge haben können, nicht eintreten.

Die oben beschriebenen Reaktionen können gegebenenfalls gleichzeitig oder nacheinander, ferner in beliebiger Reihenfolge durchgeführt werden. Falls notwendig, erfolgen sie in Anwesenheit von Verdünnungsmitteln, Kondensationsmitteln und/oder katalytisch wirkenden Mitteln, bei erniedrigter oder erhöhter Temperatur, im geschlossenen Gefäss unter Druck und/oder in einer Inertgasatmosphäre.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z. B. durch Behandeln mit basischen Mitteln, wie Alkalimetallhydroxiden, -carbonaten oder -hydrogencarbonaten oder Ionenaustauschern, in die freie Verbindungen übergeführt werden. Andererseits können erhaltene freie Basen mit organischen oder anorganischen Säuren, z. B. mit den genannten Säuren, Säureadditionssalze bilden, wobei zu deren Herstellung insbesondere solche Säuren verwendet werden, die sich zur Bildung von pharmazeutisch annehmbaren, nicht-toxischen Salzen eignen.

Diese oder andere Salze, insbesondere Säureadditionssalze der neuen Verbindungen, wie z. B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach der Wahl der Ausgangsstoffe und Arbeitsweisen, als optische Antipoden oder Racemate, oder sofern sie mindestens zwei asymmetrische Kohlenstoffatome enthalten, auch als Racematgemische vorliegen. Die Ausgangsstoffe können auch als bestimmte optische Antipoden eingesetzt werden.

Erhaltene Racematgemische können auf Grund der physikalisch-chemischen Unterschiede der Diastereoisomeren in bekannter Weise, z. B. durch Chromatographie und/oder fraktionierte Kristallisation, in die beiden stereoisomeren (diastereoisomeren) Racemate aufgetrennt werden.

Erhaltene Racemate lassen sich nach an sich bekannten Methoden in die Antipoden zerlegen, z. B. durch Umkristallisieren aus einem optisch aktiven Lösungsmittel, durch Behandeln mit geeigneten Mikroorganismen oder durch Umsetzen mit einer, mit der racemischen Verbindung Salze bildenden

optisch aktiven Substanz, insbesondere Säuren, und Trennen des auf diese Weise erhaltenen Salzgemisches, z. B. auf Grund von verschiedenen Löslichkeiten, in die diastereoisomeren Salze, aus denen die freien Antipoden durch Einwirkung geeigneter Mittel freigesetzt werden können. Besonders gebräuchliche, optische aktive Säuren sind z. B. die D- und L-Formen von Weinsäure, Di-O,O'-(p-Toluoyl)-weinsäure, Aepfelsäure, Mandelsäure, Camphersulfonsäure, Glutaminsäure, Asparaginsäure, oder Chinasäure. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonder erwähnten Gruppen von Endstoffen und besonders zu den speziell beschriebenen oder hervorgehobenen Endstoffen führen.

Die Ausgangsstoffe sind bekannt oder können, falls sie neu sind, nach an sich bekannten Methoden, wie oben, z. B. analog wie in den Beispielen, beschrieben, erhalten werden. Neue Ausgangsstoffe bilden ebenfalls einen Gegenstand der Erfindung. Die Erfindung betrifft auch verfahrensgemäss erhältliche neue Zwischenprodukte.

Die neuen Verbindungen können z. B. in Form pharmazeutischer Präparate Verwendung finden, welche eine pharmakologisch wirksame Menge der Aktivsubstanz, gegebenenfalls zusammen mit pharmazeutisch verwendbaren Trägerstoffen enthalten, die sich zur enteralen, z. B. oralen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z. B. Laktose, Dextrose, Sukrose, Mannitol, Sorbitol, Cellulose und/oder Glycerin und/oder Schmiermitteln, z. B. Kieselerde, Talk, Stearinsäure oder Salze davon, wie Magnesium- oder Calciumstearat, und/oder Polyäthylenglykol, aufweisen. Tabletten können ebenfalls Bindemittel, z. B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die neuen pharmakologisch wirksamen Verbindungen in Form von parenteral verabreichbaren Präparaten oder von Infusionslösungen verwenden. Solche Lösungen sind vorzugsweise isotonische wässerige Lösungen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z. B. mittels konventioneller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffes.

Die Dosierung kann von verschiedenen Faktoren, wie Applikationsweise, Spezies, Alter und/oder individuellen Zustand abhängen. So liegen die täglich in einer oder mehreren, vorzugsweise höchstens 4 Einzeldosen zu verabreichenden Dosen bei oraler Applikation an Warmblüter für β-Rezeptoren-Blocker der Formel I zwischen 0,03 und 3 mg/kg und für Warmblüter von etwa 70 kg vorzugsweise zwischen etwa 0,02 g und etwa 0,2 g.

Die folgenden Beispiele dienen zur Illustration der Erfindung ; Temperaturen werden in Celsiusgraden angegeben.

## Beispiel 1

Eine Lösung von 9,7 g rohem 1-[N-Benzyl-2-(3-carbamoyl-4-hydroxy-phenoxy)-äthylamino]-3-[4-[1-methyl-4-(trifluormethyl)-1H-imidazol-2-yl]-phenoxy]-2-propanol in 100 ml Methanol wird nach Zusatz von 1 g Palladium-auf-Kohle-Katalysator (5 %) unter Normalbedingungen bis zur beendeten Wasserstoffaufnahme hydriert, wobei das Reaktionsprodukt teilweise aus der Lösung ausfällt. Die Suspension wird mit 200 ml Dioxan/Methanol 1 : 1 verdünnt, erwärmt, und durch ein Filterhilfsmittel filtriert. Das Filtrat wird am Rotationsverdampfer vom Lösungsmittel befreit und der Rückstand 2 mal aus Methanol umkristallisiert, wobei man das 1-[2-(3-Carbamoyl-4-hydroxy-phenoxy)-äthylamino]-3-[4-[1-methyl-4-(trifluormethyl)-1H-imidazol-2-yl]-phenoxy]-2-propanol vom Smp. 195-197° erhält.

Das auf übliche Weise erhaltene Monomethansulfonat zeigt nach dem Umkristallisieren aus Acetonitril den Smp. 139-142°.

Das als Ausgangsmaterial benötigte 1-[N-Benzyl-2-(3-carbamoyl-4-hydroxy-phenoxy)-äthylamino]-3-[4-[1-methyl-4-(trifluormethyl)-1H-imidazol-2-yl)-phenoxy]-2-propanol kann wie folgt hergestellt werden :

a) zu einer Lösung von 12 g 2-[4-(2,3-Epoxypropoxy)-phenyl]-4-(trifluormethyl)-imidazol (J. med.

Chem. *20*, 1024 (1977)) in 90 ml Dimethylformamid, werden unter Kühlung im Eisbad 2 g Natriumhydrid-Dispersion (55 % in Oel) zugegeben und die gelbe Suspension 1 1/2 Stunden bei Raumtemperatur gerührt. Nach Zugabe von 8 ml Methyljodid wird während weiteren 12 Stunden bei Raumtemperatur gerührt, das Reaktionsgemisch anschliessend in Wasser eingegossen und mehrmals mit Essigsäureäthylester extrahiert. Nach Abtrennen der organischen Phase und Enfernen des Lösungsmittels wird der Rückstand in Chloroform gelöst und über Kieselgel filtriert. Das Filtrat wird erneut eingedampft und der Rückstand aus Aether/Hexan umkristallisiert, wobei das 2-[4-(2,3-Epoxypropoxy)-phenyl]-1-methyl-4-(trifluormethyl)-imidazol vom Smp. 65-68° erhalten wird.

b) Eine Lösung von 5 g 2-[4-(2,3-Epoxypropoxy)-phenyl]-1-methyl-4-(trifluormethyl)-imidazol und 4,7 g 5-[2-Benzylamino)-äthoxy]-salicylamid in 100 ml Isopropanol wird während 15 Stunden unter Rückfluss erhitzt und das Lösungsmittel anschliessend am Rotationsverdampfer entfernt. Das dabei als Oel anfallende, rohe 1-(N-Benzyl-2-(3-carbamoyl-4-hydroxy-phenoxy)-äthylamino]-3-[4-[1-methyl-4-(trifluormethyl)-1H-imidazol-2-yl]-phenoxy]-2-propanol wird als solches weiterverarbeitet.

## Beispiel 2

Eine Lösung von 5,0 g 1-[2-(3-Carbamoyl-4-hydroxy-phenoxy)-N-benzyl-äthylamino]-3-[4-[5-[(4-trifluormethyl)-1-benzyl-1H-imidazol-2-yl]-2-furyl]-phenoxy]-2-propanol in 50 ml Methanol wird in Gegenwart von 1 g Palladium-auf-Kohle-Katalysator (5 %) unter Normaldruck bei 50° bis zur beendeten Wasserstoffaufnahme hydriert. Die entstehende Suspension wird mit 1 Liter Methanol aufgekocht und heiss filtriert. Das Filtrat wird am Rotationsverdampfer auf ein kleines Volumen eingeengt, 1,3 g Methansulfonsäure zugegeben, die Lösung kurz erwärmt und durch Abkühlung zur Kristallisation gebracht. Nach nochmaliger Umkristallisation des Rohprodukts aus Methanol erhält man das 1-[2-(3-Carbamoyl-4-hydroxy-phenoxy)-äthylamino]-3-[4-[5-[4-(trifluormethyl)-1H-imidazol-2-yl]-2-furyl]-phenoxy]-2-propanol-dimethansulfonat vom Smp. 232-234°.

Das als Ausgangsmaterial verwendete 1-[2-(3-Carbamoyl-4-hydroxy-phenoxy)-N-benzyl-äthylamino]-3-[4-[5-[4-(trifluormethyl)-1-benzyl-1H-imidazol-2-yl]-2-furyl]-phenoxy]-2-propanol kann wie folgt hergestellt werden :

a) 64,0 g 1,4-Anisidin werden in 370 ml Wasser aufgeschlämmt und mit 172 ml konzentrierter Salzsäure versetzt. Nach Kühlung im Eisbad wird im Verlaufe einer Stunde unter Rühren eine Lösung von 36,4 g Natriumnitrit in 120 ml Wasser zugetropft. Nach 1 1/2 Stunden werden zuerst 84 g Natriumacetat, anschliessend eine Lösung von 28,8 g Kupfer(II)-chlorid-dihydrat in 160 ml Wasser und schliesslich eine Emulsion von 100,8 g Furan-2-carboxaldehyd in 200 ml Wasser zugegeben. Das dunkle Reaktionsgemisch wird über Nacht im Eisbad und anschliessend 7 Tage bei Raumtemperatur gerührt.

Zur Aufarbeitung wird das Reaktionsgemisch mit Toluol mehrmals extrahiert, die vereinigten organischen Phasen mit Wasser gewaschen, getrocknet und das nach Entfernung des Lösungsmittels zurückbleibende Oel mit Essigsäureäthylester/Hexan 1 : 4 durch Kieselgel filtriert. Man erhält so den 5-(4-Methoxyphenyl)-furan-2-carboxaldehyd in Form eines kristallisierenden Oels, welches als solches in der nächsten Stufe eingesetzt wird.

b) Eine Lösung von 39,9 g Natriumacetat und 40,2 g 1,1 Dibrom-3,3,3-trifluoraceton in 90 ml Wasser wird während 30 Minuten unter Rückfluss erhitzt, anschliessend auf 0° abgekühlt und zu einer Lösung von 30 g 5-(4-Methoxyphenyl)-furan-2-carboxaldehyd in 1 Liter Methanol bei 0° in einem Guss zugegeben. Bei 5-10° werden 165 ml konzentrierte Ammoniaklösung im Verlaufe von 30 Minuten zugetropft. Nach 50 Stunden Rühren bei Raumtemperatur wird filtriert und der Rückstand mit Chloroform gewaschen, wobei das 2-[(4-Trifluormethyl)-1H-imidazol-2-yl]-5-(4-methoxy-phenyl)-furan als gelbliches Kristallisat ausfällt.

c) Eine Lösung von 18,7 g 2-[4-(Trifluormethyl)-1H-imidazol-2-yl]-5-(4-methoxyphenyl)-furan in 200 ml Dimethylformamid wird mit 2,75 g Natriumhydrid-Dispersion (55 %ig) versetzt und eine Stunde bei Raumtemperatur gerührt. Nach Zugabe von 10,8 g Benzylbromid rührt man weitere 20 Stunden bei Raumtemperatur, entfernt anschliessend das Lösungsmittel am Hochvakuum, suspendiert den Rückstand in Essigsäureäthylester/Hexan 1 : 4 und filtriert über Kieselgel. Das Eluat wird zur Trockene eingedampft und das erhaltene rohe 2-[1-Benzyl-4-(trifluormethyl)-1H-imidazol-2-yl]-5-(4-methoxyphenyl)-furan als solches weiterverarbeitet.

d) Eine Lösung von 19,6 g 2-(1-Benzyl-4-(trifluormethyl)-imidazol-2-yl]-5-(4-methoxyphenyl)-furan und 8,1 g D,L-Methionin in 110 ml Methansulfonsäure wird unter Stickstoff während 20 Stunden bei 100° gerührt. Nach dem Abkühlen wird das Reaktionsgemisch auf Eis gegossen, mit konzentriertem Ammoniak auf pH 8 eingestellt und mehrmals mit Methylenchlorid extrahiert. Waschen mit Wasser, Trocknen und Eindampfen der organischen Phase liefert das kristalline 2-[1-Benzyl-4-(trifluormethyl)-1H-imidazol-2-yl]-5-(4-hydroxyphenyl)-furan, welches als solches in der nächsten Stufe eingesetzt wird.

e) Eine Lösung von 18,5 g 2-[1-Benzyl-4-(trifluormethyl)-1H-imidazol-2-yl]-5-(4-hydroxyphenyl)-furan in 160 ml Dioxan wird mit einer Lösung von 48,1 ml 1-n.Natronlauge und 18,5 ml Wasser versetzt und das Gemisch 10 Minuten bei Raumtemperatur gerührt. Nach Zugabe von 13,3 g Epichlorhydrin wird weitere 48 Stunden bei Raumtemperatur gerührt, anschliessend filtriert und der Filterrückstand mit Dioxan/Wasser 1 : 1 und anschliessend mit Aether gewaschen. Nach dem Trocknen erhält man das 2-[4-(2,3-Epoxypropoxy)-phenyl]-5-[1-Benzyl-4-(trifluormethyl)-1H-imidazol-2-yl]-furan als weisses Kristallisat.

f) Eine Lösung 7,0 g 2-(4-Epoxypropoxy)-phenyl]-5-[1-Benzyl-4-(trifluormethyl)-1H-imidazol-2-yl]-furan und 4-2-(Benzylamino)-äthoxy]-salicylamid in 120 ml Isopropanol wird während 12 Stunden unter Rückfluss gerührt. Nach Zugabe von Aktivkohle wird heiss filtriert und das Lösungsmittel am Rotationsverdampfer entfernt. Der Rückstand wird anschliessend über Kieselgel mit einem Gemisch von Chloroform/Methanol/Ammoniak (350 : 50 : 1) chromatographiert. Nach dem Aufarbeiten erhält man das 1-[2-(3-Carbamoyl-4-hydroxy-phenoxy)-N-benzyl-äthylamino]-3-[4-[5-[(4-trifluormethyl)-1-benzyl-1H-imidazol-2-yl]-2-furyl]-phenoxy]-2-propanol als gelblichen Schaum, welcher als solcher weiterverarbeitet wird.

Beispiel 3

Eine Lösung von 3 g rohem 1-[N-Benzyl-2-(3-carbamoyl-4-hydroxy-phenoxy)-äthylamino]-3-[4-[(4-trifluormethyl)-1H-imidazol-2-yl]-phenoxy]-2-propanol in 30 ml absolutem Methanol wird in Gegenwart von 0,5 g Palladium-auf-Kohle-Katalysator (5 %) unter Normaldruck bei Raumtemperatur bis zur beendeten Wasserstoffaufnahme hydriert. Die erhaltene Suspension wird mit 30 ml Methanol verdünnt, unter Rückfluss erwärmt und heiss filtriert. Das Filtrat wird am Rotationsverdampfer vom Lösungsmittel befreit und der Rückstand aus Isopropanol umkristallisiert. Man erhält das 1-[2-(3-Carbamoyl-4-hydroxy-phenoxy)-äthylamino]-3-[4-[4-(trifluormethyl)-1H-imidazol-2-yl]-phenoxy]-2-propanol vom Smp. 155-158°.

Das als Ausgangsmaterial verwendete 1-[N-Benzyl-2-(3-carbamoyl-4-hydroxy-phenoxy)-äthylamino]-3-[4-[(4-trifluormethyl)-1H-imidazol-2-yl-phenoxy]-2-propanol kann wie folgt hergestellt werden :

Eine Lösung von 1,8 g 2-[4-(2,3-Epoxy-propoxy)-phenyl]-(4-trifluormethyl)-imidazol (J. Med. Chem. *20*, 1024 (1977)) und 1,8 g 4-[2-(Benzylamino)-äthoxy]-salicylamid in 100 ml Isopropanol wird während 15 Stunden unter Rückfluss erhitzt und das Lösungsmittel anschliessend am Rotationsverdampfer entfernt. Der ölige Rückstand wird in Essigsäureäthylester aufgenommen und über Kieselgel filtriert. Das nach Entfernung des Lösungsmittels resultierende rohe 1-[N-Benzyl-2-(3-carbamoyl-4-hydroxy-phenoxy)-äthylamino]-3-[4-(4-trifluormethyl)-1H-imidazol-2-yl-phenoxy]-2-propanol wird als solches weiterverarbeitet.

Beispiel 4

Eine Lösung von 13,2 g rohem 1-[N-Benzyl-[2-(3-carbamoyl-4-hydroxy-phenoxy)-äthylamino]]-3-[4-(4-acetyl-1,5-dimethyl-1H-imidazol-2-yl)-phenoxy]-2-propanol in 140 ml Methanol wird unter Zusatz von 1,3 g Palladium-auf-Kohle-Katalysator bis zur Aufnahme von 1 Mol-äquivalent Wasserstoff hydriert. Nach Filtration und Abdampfen des Lösungsmittels erhält man ein Oel, welches nach zweimaligem Umkristallisieren aus Aethanol das 1-[2-(3-Carbamoyl-4-hydroxy-phenoxy)-äthylamino]-3-[4-(4-acetyl-1,5-dimethyl-1H-imidazol-2-yl)-phenoxy]-2-propanol vom Smp. 128-130° ergibt.

Das Ausgangsmaterial wird auf folgende Weise hergestellt :

a) Analog dem von H. Lettau (Z. Chem. *10*, 338, 1970 und ibid. *11*, 10, 1971) beschriebenen Verfahren erhält man unter Verwendung von 3-Hydroximino-pentan-2,4-dion, 4-Hydroxybenzaldehyd und Methylamin in Essigsäure das 4-Acetyl-1,5-dimethyl-2-(4-hydroxyphenyl)-1H-imidazol vom Smp. 198-202° als Rohprodukt.

b) Ein Gemisch von 11,5 g 4-Acetyl-1,5-dimethyl-2(4-hydroxyphenyl)-1H-imidazol, 13,8 g Kaliumcarbonat, 7,0 g Epichlorhydrin und 120 ml Dimethylformamid wird während 20 Stunden in einem Bad von 60-70° gerührt. Das Unlösliche wird abfiltriert, das Filtrat eingedampft und der Rückstand zwischen Aethylacetat und Wasser verteilt. Aus der organischen Phase erhält man durch Eindampfen ein Oel, welches an Silicagel mit Aethylacetat chromatographiert wird. Aus den zuerst eluierten sechs Fraktionen wird das 4-Acetyl-1,5-dimethyl-2-[4-(2,3-epoxypropoxy)-phenyl]-1H-imidazol als gelbes Oel erhalten.

c) Eine Lösung von 8 g rohem 4-Acetyl-1,5-dimethyl-2-[4-(2,3-epoxypropoxy)-phenyl]-1H-imidazol in 80 ml Isopropanol wird mit 7,2 g 5-(2-Benzylamino-äthoxy)-salicylamid 18 Stunden unter Rückfluss gekocht. Durch Eindampfen erhält man das 1-[N-Benzyl-[2-(3-carbamoyl-4-hydroxy-phenoxy)-äthylamino]]-3-[4-(4-acetyl-1,5-dimethyl-1H-imidazol-2-yl)-phenoxy]-2-propanol als gelben Schaum, welcher als Rohprodukt weiterverarbeitet wird.

Beispiel 5

Eine Lösung von 13,1 g rohem 1-[N-Benzyl-2-(3-carbamoyl-4-hydroxy-phenoxy)-äthylamino]-3-[4-[4-methyl-6-(trifluormethyl)-pyrimidin-2-yl]-phenoxy]-2-propanol-hydrochlorid wird analog Beispiel 1 hydriert und aufgearbeitet. Aus der erhaltenen Lösung des Hydrochlorids wird die freie Base mittels Kaliumhydrogencarbonat in wässrigem Methanol ausgefällt. Nach dem Umkristallisieren aus Methylcellosolve erhält man das 1-[2-(3-Carbamoyl-4-hydroxy-phenoxy)-äthylamino]-3-[4-[4-methyl-6-(trifluormethyl)-pyrimidin-2-yl]-phenoxy]-2-propanol vom Smp. 227-229°.

Der Ausgangsstoff kann auf folgende Weise hergestellt werden :

a) In einem Druckrohr wird das Gemisch von 17,2 g 4-Hydroxybenzamidin-dihydrat, 60 ml Aethanol

und 14,6 ml Trifluoracetylaceton während 18 Stunden bei einer Badtemperatur von 110° erwärmt. Darauf wird das Reaktionsgemisch mit 200 ml Aether gerührt, eine geringe Menge Niederschlag abgesaugt und das Filtrat mit zwei Portionen 2-n. Natronlauge extrahiert. Die alkalische Lösung wird mit Kohle behandelt, filtriert und darauf mit 400 ml Essigsäureäthylester und 160 ml 2-n. Salzsäure bis pH 1 versetzt. Die organische Phase wird abgetrennt, mit Wasser gewaschen, über Natriumsulfat getrocknet und eingedampft. Der Rückstand wird aus Aether-Hexan umkristallisiert, wonach man das 2-(4-Hydro xyphenyl)-4-methyl-6-(trifluormethyl)-pyrimidin vom Smp. 131-132 °C erhält.

b) Das Gemisch von 17,0 g 2-(4-Hydroxyphenyl)-4-methyl-6-(trifluormethyl)-pyrimidin, 23,1 g Kaliumcarbonat und 135 ml Epichlorhydrin wird während 1,5 Stunden bei einer Temperatur von 130° gerührt, anschliessend heiss abgesaugt, der Filterrückstand mit Methylenchlorid nachgewaschen und das Filtrat unter vermindertem Druck zur Trockne verdampft. Der Rückstand wird aus Isopropanol umkristallisiert und ergibt das 2-[4-(2,3-Epoxypropoxy)-phenyl]-4-methyl-6-(trifluormethyl)-pyrimidin von Smp. 97-99°.

c) 9,3 g des erhaltenen 2-[4-(2,3-Epoxypropoxy)-phenyl]-4-methyl-6-(trifluormethyl)-pyrimidin und 8,15 g 5-[2-(Benzylamino)-äthoxy]-salicylamid werden in 130 ml Isopropanol bei einer Badtemperatur von 70° während 15 Stunden gerührt. Man versetzt mit 10 ml 3,5-n. ätherischer Salzsäure und 600 ml Aether, worauf das rohe 1-[N-Benzyl-2-(3-carbamoyl-4-hydroxy-phenoxy)-äthylamino]-3-[4-[4-methyl-6-(trifluormethyl)-pyrimidin-2-yl]-phenoxy]-2-propanol-hydrochlorid zunächst in schmieriger Form, dann als gelbes Pulver erhalten wird.

## Beispiel 6

16 g rohes 1-[N-Benzyl-2-(3-carbamoyl-4-hydroxy-phenoxy)-äthylamino]-3-[4-(4,6-dimethyl-pyrimidin-2-yl)-phenoxy]-2-propanol-hydrochlorid werden analog Beispiel 1 hydriert und gemäss Beispiel 5 die freie Base isoliert. Nach dem Umkristallisieren aus Methylcellosolve erhält man das 1-[2-(3-Carbamoyl-4-hydroxy-phenoxy)-äthylamino]-3-[4-(4,6-dimethyl-pyrimidin-2-yl)-phenoxy]-2-propanol vom Smp. 209-211°.

Der Ausgangsstoff kann auf folgende Weise erhalten werden :

a) 4,6-Dimethyl-2-(4-hydroxyphenyl)-pyrimidin werden mit Epichlorhydrin in Gegenwart von Kaliumcarbonat analog Beispiel 5b) umgesetzt. Nach dem Aufarbeiten erhält man das 4,6-Dimethyl-2-[4-(2,3-epoxy-propoxy)-phenyl]-pyrimidin, welches nach dem Umkristallisieren aus Isopropanol den Smp. 88-90° zeigt.

b) Das erhaltene 4,6-Dimethyl-2-[4-(2,3-epoxypropoxy)-phenyl]-pyrimidin wird analog Beispiel 5c) mit 5-[2-Benzylamino-äthoxy]-salicylamid in Isopropanol umgesetzt. Nach dem Aufarbeiten erhält man das rohe 1-[N-Benzyl-2-(3-carbamoyl-4-hydroxy-phenoxy)-äthylamino]-3-[4-(4,6-dimethyl-pyrimidin-2-yl)-phenoxy]-2-propanol-hydrochlorid als beigefarbenes Pulver, welches als solches weiterverarbeitet wird.

## Beispiel 7

11,6 g 1-[N-Benzyl-2-(3-carbamoyl-4-hydroxy-phenoxy)-äthylamino]-3-[4-(3,5-di-carbäthoxy-2,6-dimethyl-pyridin-4-yl)-phenoxy]-2-propanol werden analog Beispiel 1 hydriert und aufgearbeitet. Die erhaltene Base wird mit Fumarsäure in Isopropanol umgesetzt und das erhaltene Salz aus Aethanol umkristallisiert. Nach dem Trocknen bei 0,05 mm Hg und 80° während 15 Stunden erhält man das 1-[2-(3-Carbamoyl-4-hydroxy-phenoxy)-äthylamino]-3-[4-(3,5-dicarbäthoxy-2,6-dimethyl-pyridin-4-yl)-phenoxy]-2-propanol als neutrales Fumarat mit 1/4 Mol Kristall-Wasser vom Smp. 133-135°.

Der Ausgangsstoff kann auf folgende Weise hergestellt werden :

a) 19,8 g 2,6-Dimethyl-4-(4-hydroxyphenyl)-1,4-dihydropyridin-3,5-dicaräureäthylester werden mit 3,7 g Schwefel während 30 Minuten bei einer Badtemperatur von 220° geschmolzen. Die Reaktionsmasse wird in kochendem Acetonitril gelöst und die Lösung mit Kohle behandelt. Beim Erkalten kristallisiert der 2,6-Dimethyl-4-(4-hydroxyphenyl)-pyridin-3,5-dicarbonsäurediäthylester, der noch überschüssigen Schwefel enthält, aus. Durch Erhitzen bei 90° und 0,05 mm Hg in einer Sublimationsapparatur wird der grösste Teil des Schwefels entfernt, wonach das Produkt bei 172-174° schmilzt.

b) 9,0 g des erhaltenen 2,6-Dimethyl-4-(4-hydroxyphenyl)-pyridin-3,5-dicarbonsäurediäthylesters werden in einer Lösung, die aus 0,723 g Natriummetall und 80 ml Aethanol hergestellt ist, gelöst. Diese Lösung lässt man langsam zu einer siedenden Lösung von 11,9 ml Epichlorhydrin in 80 ml Aethanol zutropfen, saugt nach dem Erkalten vom abgeschiedenen Natriumchlorid ab und dampft das Filtrat zur Trockne ein. Der Rückstand wird in Aether gelöst und nacheinander mit wässriger 1-n. Natronlauge, 1-n. Salzsäure, gesättigter wässriger Kaliumbicarbonat-Lösung und schliesslich mit Wasser gewaschen. Nach Abdestillieren des Aethers erhält man den rohen 2,6-Dimethyl-4-[4-(2,3-epoxypropoxy)-phenyl]-pyridin-3,5-dicarbonsäurediäthylester als gelbes Oel.

c) Der erhaltene 2,6-Dimethyl-4-[4-(2,3-epoxypropoxy)-phenyl]-pyridin-3,5-dicarbonsäure-diäthylester wird mit 5-[2-Benzylamino-äthoxy]-salicylamid in Isopropanol analog Beispiel 5c) umgesetzt.

Das erhaltene Hydrochlorid wird mit wässriger gesättigter Kaliumbicarbonat-Lösung in die freie Base überführt, welche das 1-[N-benzyl-2-(3-carbamoyl-4-hydroxy-phenoxy)-äthylamino]-3-[4-(3,5-di-carbäthoxy-2,6-dimethyl-pyridin-4-yl)-phenoxy]-2-propanol darstellt, und als solches weiterverarbeitet wird.

Beispiel 8

Eine Lösung von 0,95 g rohem 1-[N-Benzyl-2-(3-carbamoyl-4-benzyloxy-phenoxy)-äthylamino]-3-[4-[1-methyl-4-(trifluormethyl)-1H-imidazol-2-yl]-phenoxy]-2-propanol in 10 ml Methanol wird nach Zusatz von 0,3 g Palladium-auf-Kohle-Katalysator (5 %) unter Normalbedingungen bis zur beendeten Wasserstoffaufnahme hydriert, wobei das Reaktionsprodukt teilweise aus der Lösung ausfällt. Die Suspension wird mit 20 ml Dioxan/Methanol 1 : 1 verdünnt, erwärmt, und durch ein Filterhilfsmittel filtriert. Das Filtrat wird eingedampft und der Rückstand 2 mal aus Methanol umkristallisiert, wobei das 1-[2-(3-Carbamoyl-4-hydroxy-phenoxy)-äthylamino]-3-[4-(1-methyl-4-(trifluormethyl)-1H-imidazol-2-yl]-phenoxy]-2-propanol vom Smp. 195-197° erhalten wird.

Das Ausgangsmaterial kann z. B. wie folgt hergestellt werden :

a) 57,3 g 5-[2-Benzylamino-äthoxy]-salicylamid werden in 280 ml Dimethylformamid gelöst und portionenweise mit 8,8 g Natriumhydrid versetzt. Nach 2 Stunden wird eine Lösung von 25,5 ml Benzylchlorid in 40 ml Dimethylformamid zugetropft und wiederum 2 Stunden nachgerührt. Das Reaktionsgemisch wird dann auf 2,8 Liter Eis/Wasser gegossen und nacheinander mit Essigsäureäthylester und mit Aether extrahiert. Die organische Phase wäscht man mit 2-n. Natronlauge, dann mit Wasser und behandelt sie mit Kohle. Das organische Filtrat versetzt man mit 200 ml 2-n. Salzsäure, rührt 15 Minuten und saugt das ausgefallene Hydrochlorid ab. Es wird mit Wasser, Essigsäureäthylester und dann mit Aether gewaschen und darauf in 600 ml heissem Methanol gelöst. Nach Zugabe von 650 ml Aether erhält man das 2-Benzyloxy-5-[2-benzylamino-äthoxy]-benzamid-hydrochlorid vom Smp. 200-203°.

b) 31,3 g 2-Benzyloxy-5-[2-(benzylamino)-äthoxy]-benzamid werden mit 17,7 g 4-(2,3-Epoxypropoxy)-benzaldehyd in 500 ml Isopropanol analog Beispiel 5c) zum 1-[N-Benzyl-2-(3-carbamoyl-4-benzyloxy-phenoxy)-äthylamino]-3-(4-formyl-phenoxy)-2-propanol-hydrochlorid umgesetzt.

c) Eine Lösung von 5,25 g Natriumacetat kristallisiert, in 20 ml Wasser wird mit 5,25 g 1,1,1-Trifluor-3,3-dibromaceton versetzt und das Gemisch während 1 Stunde bei 100° gerührt. Anschliessend wird das Reaktionsgemisch in einem Guss zu einer Lösung von 8,9 g 1-[N-Benzyl-2-(3-carbamoyl-4-benzyloxy-phenoxy)-äthylamino]-3-(4-formyl-phenoxy)-2-propanol in 180 ml Methanol bei Raumtemperatur zugegeben. Man tropft nun bei 15-20° (Eisbadkühlung) während 10 Minuten 24 ml Ammoniak konz. (ca. 25 %) zu. Nach 48 Stunden Rühren bei Raumtemperatur wird das Reaktionsgemisch eingedampft. Der Rückstand wird in Wasser gelöst und die Lösung mit Essigsäureäthylester extrahiert, die organische Phase mit gesättigter wässriger Natriumchloridlösung gewaschen und über Natriumsulfat getrocknet. Das nach Entfernung des Lösungsmittels zurückbleibende Oel wird mit Chloroform/Methanol 95 : 5 über Kieselgel filtriert. Man erhält so das 1-[N-Benzyl-2-(3-carbamoyl-4-benzyloxy-phenoxy)-äthylamino]-3-[4-[4-(trifluormethyl)-1H-imidazol-2-yl]-phenoxy]-2-propanol in Form eines Oels, welches als solches in der nächsten Stufe eingesetzt wird.

d) Eine Lösung von 1,0 g 1-[N-Benzyl-2-(3-carbamoyl-4-benzyloxy-phenoxy)-äthylamino]-3-[4-[4-(trifluormethyl)-1H-imidazol-2-yl]-phenoxy]-2-propanol in 5 ml Dimethylformamid wird bei 0° mit 0,18 g Kalium-tert. butylat versetzt und 20 Minuten im Eisbad gerührt. Man versetzt bei 0-5° mit einer Lösung von 0,12 ml Methyljodid in 3 ml Dimethylformamid und rührt anschliessend 4 Stunden bei Raumtemperatur. Die Reaktionslösung wird eingedampft, der Rückstand in Wasser gelöst und die Lösung mit Essigsäureäthylester extrahiert. Nach Trocknen der organischen Phase über Natriumsulfat wird diese zur Trockene eingedampft, wobei man das rohe 1-[N-Benzyl-2-(3-carbamoyl-4-benzyloxy-phenoxy)-äthylamino]-3-[4-[1-methyl-4-(trifluormethyl)-1H-imidazol-2-yl]-phenoxy]-2-propanol erhält, welches als solches weiterverarbeitet wird.

Beispiel 9

Eine Lösung von 10,2 g 1-[N-Benzyl-2-(3-carbamoyl-4-hydroxy-phenoxy)-äthylamino]-3-(4-biphenyloxy)-2-propanol in 300 ml Methanol wird nach Zusatz von 1 g Palladium-auf-Kohle-Katalysator (5 %) unter Normalbedingungen bis zur beendeten Wasserstoffaufnahme hydriert, wobei das Reaktionsprodukt teilweise aus der Lösung ausfällt. Die Suspension wird mit 1,4 Liter Dioxan/Methanol 1 : 1 verdünnt, erwärmt und durch ein Filterhilfsmittel filtriert. Das Filtrat wird eingedampft und der Rückstand 2 mal aus Methanol umkristallisiert, wobei man das 1-[2-(3-Carbamoyl-4-hydroxy-phenoxy)-äthylamino]-3-(4-biphenyloxy)-2-propanol vom Smp. 191-192° erhält.

Das auf übliche Weise erhaltene Monomethansulfonat zeigt nach dem Umkristallisieren aus Acetonitril den Smp. 169-170°.

Das als Ausgangsmaterial benötigte 1-[N-Benzyl-2-(3-carbamoyl-4-hydroxy-phenoxy)-äthylamino]-3-(4-biphenyloxy)-2-propanol kann wie folgt hergestellt werden :

a) Eine Suspension von 10 g 4-Hydroxy-biphenyl und 10 g pulverisiertem Kaliumcarbonat in 75 ml Epichlorhydrin wird 4 Stunden unter Rückfluss gerührt. Das Reaktionsgemisch wird eingedampft, der Rückstand mit Wasser versetzt und das Gemisch mit Essigsäureäthylester extrahiert. Die organische Phase wird über Natriumsulfat getrocknet und eingedampft. Der Rückstand wird aus Isopropanol umkristallisiert, wobei man das 4-(2,3-Epoxypropoxy)-biphenyl vom Smp. 86-88° erhält.

b) Eine Lösung von 5,97 g 4-(2,3-Epoxypropoxy)-biphenyl und 6,87 g 5-[2-(Benzylamino)-äthoxy]-salicylamid in 140 ml Isopropanol wird während 15 Stunden unter Rückfluss erhitzt und das Lösungsmittel anschliessend am Rotationsverdampfer entfernt. Der Rückstand wird aus Diäthyläther umkristallisiert, wobei man das 1-[N-Benzyl-2-(3-carbamoyl-4-hydroxy-phenoxy)-äthylamino]-3-(4-biphenyloxy)-2-propanol vom Smp. 135-138° erhält.

Beispiel 10

Eine Lösung von 0,85 g rohem 1-[N-Benzyl-2-(3-carbamoyl-4-benzyloxy-phenoxy)-äthylamino]-3-[4-[1-methyl-1H-imidazol-2-yl]-phenoxy]-2-propanol in 10 ml Methanol wird analog Beispiel 8 hydriert. Nach dem Aufarbeiten erhält man das 1-[2-(3-Carbamoyl-4-hydroxy-phenoxy)-äthylamino]-3-[4-(1-methyl-1H-imidazol-2-yl)-phenoxy]-2-propanol vom Smp. 108-112°.

Das als Ausgangsmaterial benötigte 1-[N-Benzyl-2-(3-carbamoyl-4-benzyloxy-phenoxy)-äthylamino]-3-[4-[1-methyl-1H-imidazol-2-yl]-phenoxy]-2-propanol kann z. B. wie folgt erhalten werden :

Analog der im Beispiel 8c) beschriebenen Arbeitsweise werden 8,9 g 1-[N-Benzyl-2-(3-carbamoyl-4-benzyloxy-phenoxy)-äthylamino]-3-(4-formyl-phenoxy)-2-propanol unter Verwendung von 4,2 g 3,3-Dibromaceton zum 1-[N-Benzyl-2-(3-carbamoyl-4-benzyloxy-phenoxy)-äthylamino]-3-[4-[1H-imidazol-2-yl]-phenoxy]-2-propanol umgesetzt, aus dem analog Beispiel 8d) das 1-[N-Benzyl-2-(3-carbamoyl-4-benzyloxy-phenoxy)-äthylamino]-3-[4-[1-methyl-1H-imidazol-2-yl]-phenoxy]-2-propanol als Rohprodukt erhalten wird, welches als solches weiterverarbeitet wird.

Beispiel 11

Analog der im Beispiel 1 beschriebenen Arbeitsweise werden 9,7 g rohes 1-[N-Benzyl-2-(4-Carbamoyl-3-hydroxy-phenoxy)-äthylamino]-3-[4-[1-methyl-4-(trifluormethyl)-1H-imidazol-2-yl]-phenoxy]-2-propanol mittels Hydrierung in das 1-[2-(4-Carbamoyl-3-hydroxy-phenoxy)-äthylamino]-3-[4-[1-methyl-4-(trifluormethyl)-1H-imidazol-2-yl]-phenoxy]-2-propanol umgewandelt, dessen auf übliche Weise erhaltenes Monomethansulfonat den Smp. 131-136° zeigt.

Das als Ausgangsmaterial benötigte 1-[N-Benzyl-2-(4-Carbamoyl-3-hydroxy-phenoxy)-äthylamino]-3-[4-[1-methyl-4-(trifluormethyl)-1H-imidazol-2-yl]-phenoxy]-2-propanol kann gemäss Beispiel 1b) aus 5 g 2-[4-(2,3-Epoxypropoxy)-phenyl]-1-methyl-4-(trifluormethyl)-imidazol und 4,7 g 4-[2-(Benzylamino)-äthoxy]-salicylamid als Rohprodukt erhalten werden, welches als solches weiterverarbeitet wird.

Beispiel 12

Eine Lösung von 11,0 g 5-(2-Aminoäthoxy)-salicylamid in 70 ml Dimethylsulfoxid wird auf 70° erwärmt, mit 19,5 g 5-[4-(2,3-Epoxy-propoxy)-phenyl]-1-methyl-3-methylthio-1H-1,2,4-triazol versetzt und 1 Stunde bei dieser Temperatur gerührt. Das Reaktionsgemisch wird auf 1 000 ml Eiswasser gegossen. Die sich nach einigen Minuten abscheidenden Kristalle werden abgesaugt und aus viel Methanol umkristallisiert. Man erhält so 1-[2-(3-Carbamoyl-4-hydroxyphenoxy)-äthylamino]-3-[4-(1-methyl-3-methylthio-1H-1,2,4-triazol-5-yl)-phenoxy]-2-propanol vom Smp. 180-181°. Aus der Mutterlauge erhält man durch Einengen eine weitere Menge dieser Verbindung.

Das als Ausgangsverbindung benötigte 5-[4-(2,3-Epoxy-propoxy)-phenyl]-1-methyl-3-methylthio-1H-1,2,4-triazol kann auf folgende Weise hergestellt werden :

12a) Ein Gemisch von 49,6 g 1-Methyl-3-methylthio-5-(4-methoxy-phenyl)-1H-1,2,4-triazol, 40 g L-Methionin und 380 ml Methansulfonsäure wird analog Beispiel 15b) 24 Stunden in einem Bad von 110-120° erhitzt und analog aufgearbeitet, wonach man 5-(4-Hydroxy-phenyl)-1-methyl-3-methylthio-1H-1,2,4-triazol vom Smp. 216-220° (aus Isopropanol) erhält.

12b) 27,2 g der erhaltenen Verbindung und 30,5 Kaliumcarbonat werden in 220 ml Epichlorhydrin 10 Minuten unter Rückfluss gerührt. Das nach dem Aufarbeiten analog Beispiel 16d) erhaltene Rohprodukt wird an 500 g Silicagel chromatographiert und mit Aether eluiert (200 ml — Fraktionen). Die vereinigten Fraktionen 18-34 werden eingedampft und aus Aethylacetat-Petroläther umkristallisiert, wonach man das 5-[4-(2,3-Epoxy-propoxy)-phenyl]-1-methyl-3-methylthio-1H-1,2,4-triazol vom Smp. 75° erhält.

Beispiel 13

Eine Lösung von 21 g rohem 1-[N-Benzyl-[2-(3-carbamoyl-4-hydroxy-phenoxy)-äthylamino]]-3-[2-(indol-2-yl)-phenoxy]-2-propanol in 210 ml Methanol wird unter Zusatz von 5 g Palladium-auf-Kohle-Katalysator (5 %) analog Beispiel 4 hydriert und aufgearbeitet.

Nach Umkristallisation aus Aethanol erhält man 1-[2-(3-Carbamoyl-4-hydroxy-phenoxy)-äthylamino]-3-[2-(indol-2-yl)-phenoxy]-2-propanol vom Smp. 93-95°, welches 1/2 Mol Aethanol enthält.
Die Ausgangsverbindung wird auf folgende Weise erhalten :

13a) Eine Lösung von 10,5 g 2-[2-(2,3-Epoxy-propoxy)-phenyl]-indol und 10,2 g 5-(2-Benzylamino-äthoxy)-salicylamid in 300 ml Isopropanol wird analog Beispiel 4c) umgesetzt und ergibt rohes 1-[N-Benzyl-[2-(3-carbamoyl-4-hydroxy-phenoxy)-äthylamino]]-3-[2-(indol-2-yl)-phenoxy]-2-propanol als oranges Harz, das als solches weiter verarbeitet wird.

## Beispiel 14

Eine Lösung von 25 g rohem 1-[N-Benzyl-[2-(3-carbamoyl-4-hydroxy-phenoxy)-äthylamino]]-3-[4-(1,4,5-trimethyl-1H-imidazol-2-yl)-phenoxy]-2-propanol in 250 ml Methanol wird unter Zusatz vom insgesamt 10 g Palladium-auf-Kohle-Katalysator (5 %) in 2 Portionen unter Normaldruck bis zum Stillstand der Wasserstoff-Aufnahme hydriert. Die filtrierte und eingedampfte Hydrierlösung wird an 500 g Silicagel chromatographiert und mit Chloroform-Methanol (9 : 1) eluiert (Fraktionen zu je 50 ml). Die Fraktionen 150-200 werden vereinigt und eingedampft und ergeben das 1-[2-(3-Carbamoyl-4-hydroxy-phenoxy)-äthylamino]-3-[4-(1,4,5-trimethyl-1H-imidazol-2-yl)-phenoxy]-2-propanol als beigefarbenen Schaum, dessen $^1H$-NMR-Spektrum (DMSO-d6) mit der angegebenen Struktur verträglich ist.
Die Ausgangsverbindung wird auf folgende Weise hergestellt :

14a) Analog Beispiel 4a) erhält man aus 3-Oximino-2-butanon, 4-Hydroxy-benzaldehyd und Methylamin in Eisessig und anschliessender Reduktion mit Zinkstaub das 2-(4-Hydroxyphenyl)-1,4,5-trimethyl-1H-imidazol vom Smp. 270-277°.

14b) Analog Beispiel 4b) erhält man aus der nach Beispiel 14a) erhaltenen Verbindung das 2-[4-(2,3-Epoxy-propoxy)-phenyl]-1,4,5-trimethyl-1H-imidazol als orangebraunes Oel, das teilweise kristallin erstarrt und ein $^1H$-NMR-Spektrum ($CDCl_3$) ergibt, das mit der angenommenen Struktur verträglich ist.

14c) Umsetzung von 1,2 Mol-Aequivalenten der erhaltenen rohen Verbindung mit 1,0 Mol-Aequivalent 5-(2-Benzylamino-äthoxy)-salicylamid analog Beispiel 4c) ergibt das 1-[N-Benzyl-[2-(3-carbamoyl-4-hydroxy-phenoxy)-äthylamino]]-3-[4-(1,4,5-trimethyl-1H-imidazol-2-yl)-phenoxy]-2-propanol als Oel, welches als solches weiterverarbeitet wird.

## Beispiel 15

Eine Lösung von 16 g rohem 1-[N-Benzyl-[2-(3-carbamoyl-4-hydroxy-phenoxy)-äthylamino]]-3-[4-(4-methyl-5-trifluormethyl-4H-1,2,4-triazol-3-yl)-phenoxy]-2-propanol in 160 ml Methanol wird unter Zusatz von 3,0 g Palladium-auf-Kohle-Katalysator (10 %) katalytisch unter Normalbedingungen hydriert, bis nach 35 Stunden die berechnete Menge Wasserstoff (eine zusätzliche Menge von 2 g des Katalysators ist hierfür erforderlich) aufgenommen ist. Filtration und Eindampfen des Filtrates ergeben eine halbfeste Masse, welche durch Umkristallisation aus Isopropanol unter Verwendung von Aktivkohle 1-[2-(3-Carbamoyl-4-hydroxy-phenoxy)-äthylamino]-3-[4-(4-methyl-5-trifluormethyl-4H-1,2,4-triazol-3-yl)-phenoxy]-2-propanol vom Smp. 151-153° ergibt.
Die Ausgangsverbindung wird auf folgende Weise erhalten :

15a) Eine Suspension von 12,8 g Trifluoracetylhydrazin in 100 ml Chloroform wird mit 20 ml Triäthylamin versetzt. Unter Rühren wird hierzu eine Lösung von 20 g rohem 4-Methoxy-N-methylbenzimid-chlorid in 50 ml Chloroform tropfenweise so zugegeben, dass die Innentemperatur 40-45° nicht übersteigt. Das Reaktionsgemisch wird über Nacht bei Zimmertemperatur gerührt und dann eingedampft. Der Eindampfrückstand wird zwischen Aethylacetat und 2-n. Sodalösung verteilt, die organische Phase abgetrennt, mit Wasser gewaschen, über Magnesiumsulfat getrocknet und eingedampft. Der teilweise kristalline Rückstand wird mit Aether verrührt, die Kristalle werden abfiltriert und aus Aethanol 2-mal umkristallisiert, wonach man 3-(4-Methoxyphenyl)-4-methyl-5-trifluormethyl-4H-1,2,4-triazol vom Smp. 155-160° erhält.

15b) 8,05 g der erhaltenen Verbindung und 5,1 g L-Methionin werden unter Stickstoff in 56 ml Methansulfonsäure gelöst und während 48 Stunden in einem Bad von 120° erhitzt. Nach dem Abkühlen wird das Reaktionsgemisch auf ca. 200 ml Eiswasser gegossen und mit konz. Ammoniaklösung auf pH 9-10 eingestellt, worauf das rohe 3-(4-Hydroxyphenyl)-4-methyl-5-trifluormethyl-4H-1,2,4-triazol auskristallisiert : Smp. 210-212° (sintert ab 204°).

15c) 6,9 g der erhaltenen Verbindung, 11,8 g Kaliumcarbonat und 4,5 ml Epichlorhydrin werden in 100 ml Aceton unter Rühren 20 Stunden unter Rückfluss gekocht. Nach Zusatz von weiteren 4,5 ml Epichlorhydrin und 11,8 g Kaliumcarbonat wird das Reaktionsgemisch weitere 24 Stunden gekocht. Durch Filtration und Eindampfen erhält man ein braunes, verunreinigtes Oel, welches durch Behandeln mit 100 ml 2-n. Natronlauge und 1,0 g Tetrabutylammoniumhydrogensulfat in 200 ml Dichlormethan (15 Stunden bei 20°) reines 3-[4-(2,3-Epoxy-propoxy)-phenyl]-4-methyl-5-trifluormethyl-4H-1,2,4-triazol ergibt, das nach Umkristallisation aus Isopropanol bei 104-106° schmilzt.

15d) 7,0 g 5-(2-Benzylamino-äthoxy)-salicylamid und 8,5 g der nach Beispiel 15c) erhaltenen Verbindung werden analog Beispiel 4c) umgesetzt und aufgearbeitet und ergeben rohes 1-[N-Benzyl-[2-(3-carbamoyl-4-hydroxy-phenoxy)-äthylamino]]-3-[4-(4-methyl-5-trifluormethyl-4H-1,2,4-triazol-3-yl)-phenoxy]-2-propanol als Schaum, der roh weiterverarbeitet wird.

Beispiel 16

Eine Lösung von 22 g rohem 1-[N-Benzyl-[2-(3-carbamoyl-4-hydroxy-phenoxy)-äthylamino]]-3-[4-(1-methyl-5-trifluormethyl-1H-1,2,4-triazol-3-yl)-phenoxy]-2-propanol in 320 ml Methanol, wird nach Zusatz von 2 g Palladium-auf-Kohle-Katalysator (5 %) unter Normalbedingungen hydriert, bis die berechnete Menge Wasserstoff aufgenommen ist (3 Stunden). Das teilweise auskristallisierte Produkt wird durch Zugabe von 1 000 ml Dioxan und Erwärmen in Lösung gebracht, der Katalysator abfiltriert und das Filtrat eingedampft. Der verbleibende kristalline Rückstand wird aus ca. 400 ml Dioxan umkristallisiert und ergibt 1-[2-(3-Carbamoyl-4-hydroxy-phenoxy)-äthylamino]-3-[4-(1-methyl-5-(trifluormethyl)-1H-1,2,4-triazol-3-yl)-phenoxy]-2-propanol vom Smp. 206-208°.

Die Ausgangsverbindung kann auf folgende Weise hergestellt werden :

16a) Eine Lösung von 80 g Aethyl-(4-methoxy-benzimidat) und 23 g Methylhydrazin in 1 000 ml Aethanol wird 16-18 Stunden unter Rückfluss gekocht und anschliessend eingedampft. Der kristalline Rückstand wird aus wenig Isopropanol umkristallisiert und ergibt 1-Methyl-4'-methoxy-benzamidrazon vom Smp. 111-113°.

16b) Zu der Lösung von 25,5 g der erhaltenen Verbindung in 250 ml wasserfreiem Dioxan werden unter Rühren 33,6 g Trifluoressigsäureanhydrid getropft. Nach Abklingen der exothermen Reaktion wird das Reaktionsgemisch 2 Stunden nachgerührt, eingedampft, der Eindampfrückstand in 500 ml Toluol gelöst und die Lösung über Nacht am Wasserabscheider unter Zusatz von 1 ml Methansulfonsäure erhitzt. Die Toluollösung wird hierauf mit 50 ml 2-n. Kaliumbicarbonat-Lösung gewaschen, über Magnesiumsulfat getrocknet und eingedampft. Der verbleibende kristalline Rückstand wird aus Aether-Petroläther umkristallisiert und ergibt 3-(4-Methoxyphenyl)-1-methyl-5-trifluormethyl-1H-1,2,4-triazol vom Smp. 82-83°.

16c) Aus der erhaltenen Verbindung erhält man analog der im Beispiel 15b) beschriebenen Arbeitsweise das 3-(4-Hydroxyphenyl)-1-methyl-5-trifluormethyl-1H-1,2,4-triazol vom Smp. 189-190° (aus Aether).

16d) 20,2 g der nach Beispiel 16c) erhaltenen Verbindung werden unter Rühren zu einer siedenden Suspension von 20,6 g Kaliumcarbonat in 150 ml Epichlorhydrin gegeben. Das Reaktionsgemisch wird 30-40 Minuten unter Rückfluss gekocht, noch warm filtriert und eingedampft. Das erhaltene Rohprodukt wird analog Beispiel 15c) in reines 3-[4-(2,3-Epoxy-propoxy)-phenyl]-1-methyl-5-trifluormethyl-1H-1,2,4-triazol überführt. Es schmilzt nach Umkristallisation aus Isopropanol bei 110-111°.

16e) Analog Beispiel 4c) erhält man aus 11,0 g der nach Beispiel 16d) erhaltenen Verbindung und 10,0 g 5-(2-Benzylamino-äthoxy)-salicylamid rohes 1-[N-Benzyl-[2-(3-carbamoyl-4-hydroxy-phenoxy)-äthylamino]]-3-[4-(1-methyl-5-trifluormethyl-1H-1,2,4-triazol-3-yl)-phenoxy]-2-propanol als zähflüssiges Oel, welches als solches weiterverwendet wird.

Beispiel 17

24,3 rohes 1-[N-Benzyl-[2-(3-carbamoyl-4-hydroxy-phenoxy)-äthylamino]]-3-[4-(1-methyl-3-methylsulfonyl-1H-1,2,4-triazol-5-yl)-phenoxy]-2-propanol werden analog Beispiel 4) katalytisch debenzyliert, wobei zur Aufarbeitung der kristalline Niederschlag in Dioxan durch Erwärmen gelöst wird. Durch Umkristallisieren aus Methanol-Dioxan erhält man 1-[2-(3-Carbamoyl-4-hydroxy-phenoxy)-äthylamino]-3-[4-(1-methyl-3-methylsulfonyl-1H-1,2,4-triazol-5-yl)-phenoxy]-2-propanol vom Smp. 190-191°.

Das Ausgangsmaterial kann auf folgende Weise erhalten werden :

17a) Eine Lösung von 11,5 g der nach Beispiel 12b) erhaltenen Verbindung in 200 ml Chloroform wird unter Eiskühlung und Rühren mit 15,1 g 3-Chlorperbenzoesäure portionenweise versetzt. Nach Abklingen der leicht exothermen Reaktion wird das Reaktionsgemisch 10-15 Stunden bei Raumtemperatur gerührt. Die auskristallisierte 3-Chlorbenzoesäure wird abfiltriert, das Filtrat mit je 50 ml einer wässrigen Natriumsulfitlösung (5 %), dann mit Kaliumbicarbonatlösung 2-mal gewaschen, über Magnesiumsulfat getrocknet und eingedampft. Man erhält rohes 5-[4-(2,3-Epoxy-propoxy)-phenyl]-1-methyl-3-methylsulfonyl-1H-1,2,4-triazol als gelbliches Oel, das welches als solches weiterverwendet wird.

17b) 13 g der erhaltenen rohen Verbindung und 10,6 g 5-(2-Benzylaminoäthoxy)-salicylamid in 150 ml Isopropanol werden analog der im Beispiel 4c) beschriebenen Arbeitsweise umgesetzt und ergeben rohes 1-[N-Benzyl-[2-(3-carbamoyl-4-hydroxy-phenoxy)-äthylamino]]-3-(4-(1-methyl-3-methylsulfonyl-1H-1,2,4-triazol-5-yl)-phenoxy]-2-propanol als orangefarbenes Oel, welches ohne weitere Reinigung katalytisch debenzyliert wird.

Beispiel 18

Analog der im Beispiel 12 beschriebenen Arbeitsweise erhält man durch Umsetzung von 2,3 g 5-[4-(2,3-Epoxy-propoxy)-phenyl]-2-methyl-2H-tetrazol und 1,5 g 5-(2-Aminoäthoxy)-salicylamid das 1-[2-(3-Carbamoyl-4-hydroxy-phenoxy)-äthylamino]-3-[4-(2-methyl-2H-tetrazol-5-yl)-phenoxy]-2-propanol, Smp. 174-175° (aus Dioxan).

18a) Das als Ausgangsmaterial benötigte 5-[4-(2,3-Epoxy-propoxy)-phenyl]-2-methyl-2H-tetrazol kann auf folgende Weise hergestellt werden :

Eine Lösung von 11 g 5-(4-Methoxy-phenyl)-tetrazol in 100 ml Dimethylformamid wird unter Rühren und Kühlen mit Eis portionenweise mit 2,86 g Natriumhydrid-Suspension (55 %) versetzt und hierauf 30 Minuten bei 40-50° Innentemperatur gerührt. Hierauf wird auf ca. 25° abgekühlt und 4,3 ml Methyljodid innerhalb von etwa 10 Minuten zugetropft. Nach Abklingen der exothermen Reaktion wird das Reaktionsgemisch 30 Minuten nachgerührt, filtriert und eingedampft. Der Eindampfrückstand wird mit 100 ml Wasser verrührt. Der kristalline Niederschlag wird abfiltriert und aus wenig Isopropanol umkristallisiert. Die so erhaltenen Kristalle bilden ein Gemisch, das vorwiegend aus 5-(4-Methoxy-phenyl)-2-methyl-2H-tetrazol besteht und bei 72-80° schmilzt.

18b) 63,6 g der erhaltenen Verbindung und 97,5 g DL-Methionin werden in 570 ml Methansulfonsäure gelöst und unter Stickstoff 18 Stunden in einem Bad von 110-120° gerührt. Das Reaktionsgemisch wird auf Eiswasser gegossen und mit konz. Ammoniak alkalisch gestellt. Die ausfallenden Kristalle werden abgesaugt und aus wenig Isopropanol umkristallisiert, wonach man reines 5-(4-Hydroxyphenyl)-2-methyl-2H-tetrazol erhält.

18c) Eine Suspension von 17,6 g der nach 18b) erhaltenen Verbindung und 30 g Kaliumcarbonat in 180 ml Epichlorhydrin wird während 30 Minuten unter Rückfluss erhitzt. Aufarbeitung analog Beispiel 15c) führt zu 23 g eines Oeles, das über 250 g Silicagel mittels Dichlormethan filtriert wird und das 5-[4-(2,3-Epoxy-propoxy)-phenyl]-2-methyl-2H-tetrazol vom Smp. 93-94° ergibt.

Beispiel 19

Eine Mischung von 1,5 g 1-Amino-3-[4-(1-methyl-4-(trifluormethyl)-1H-imidazol-2-yl)-phenoxy]-2-propanol und 0,82 g 5-(2-Bromäthoxy)-salicylamid wird während 1 Stunde in einem Oelbad bei einer Temperatur von 100° gerührt. Die Schmelze wird hierauf mit 30 ml Isopropanol ausgekocht. Die filtrierte Lösung wird auf 15 ml eingeengt, abgekühlt und die ausgefallenen Kristalle abfiltriert. Durch Umkristallisation aus Methanol erhält man 1-[2-(3-Carbamoyl-4-hydroxy-phenoxy)-äthylamino]-3-[4-(1-methyl-4-(trifluormethyl)-1H-imidazol-2-yl)-phenoxy]-2-propanol vom Smp. 195-197°.

Das als Ausgangsstoff verwendete 1-Amino-3-[4-(1-methyl-4-(trifluormethyl)-1H-imidazol-2-yl)-phenoxy]-2-propanol kann auf folgende Weise hergestellt werden :

19a) 3,0 g der gemäss Beispiel 1a) erhaltenen Verbindung und 2,4 g Dibenzylamin werden in 30 ml Isopropanol 10 Stunden unter Rückfluss erhitzt. Der Eindampfrückstand besteht aus öligem 1-(Dibenzylamino)-3-[4-(1-methyl-4-(trifluormethyl)-1H-imidazol-2-yl)-phenoxy]-2-propanol, das ohne weitere Reinigung weiterverarbeitet wird.

19b) Eine Lösung von 5,5 g der erhaltenen rohen Verbindung in 120 ml Methanol wird unter Zusatz von 1,0 g Palladium-auf-Kohle-Katalysator (5 %) bis zur Aufnahme von 1 Moläquivalent Wasserstoff hydriert. Durch Filtration und Eindampfen der Lösung erhält man rohes 1-Amino-3-[4-(1-methyl-4-(trifluormethyl)-1H-imidazol-2-yl)-phenoxy]-2-propanol als Oel, dessen Protonenresonanz-Spektrum mit der angenommenen Struktur verträglich ist.

Beispiel 20

Eine Lösung von 3,9 g 5-(2-Aminoäthoxy)-salicylamid in 30 ml Dimethylsulfoxid wird auf 70° erwärmt und mit 7,2 g 2-[4-(2,3-Epoxy-propoxy)-phenyl]-1-methyl-4-(trifluormethyl)-1H-imidazol versetzt. Das Reaktionsgemisch wird 1 Stunde bei 70° gerührt und analog Beispiel 12 aufgearbeitet. Man erhält 1-[2-(3-Carbamoyl-4-hydroxy-phenoxy)-äthylamino]-3-[4-(1-methyl-4-(trifluormethyl)-1H-imidazol-2-yl)-phenoxy]-2-propanol vom Smp. 195-197° (aus Methanol).

Das als Ausgangsstoff verwendete 2-[4-(2,3-Epoxy-propoxy)-phenyl]-1-methyl-4-(trifluormethyl)-1H-imidazol kann auf folgende Weise hergestellt werden :

20a) Zu einer Lösung von 81,6 g 4-Methoxybenzaldehyd in 1,5 l Methanol und 300 ml Wasser werden 48,1 g Ammoniumacetat und 300 ml konz. Ammoniak zugegeben. Bei Raumtemperatur werden anschliessend 81,0 g 3,3-Dibromtrifluoraceton zugetropft und das Reaktionsgemisch anschliessend während 18 Stunden gerührt. Das Methanol wird am Rotationsverdampfer entfernt, die zurückbleibende wässrige Emulsion mit Essigsäureäthylester mehrmals extrahiert, die organische Phase getrocknet und auf ein keines Volumen eingeengt. Durch Zugabe von Hexan und Kühlung im Eisbad wird das Produkt zur Kristallisation gebracht. Nach Abfiltrieren und Trocknen im Vakuum bei 60° erhält man das 2-(4-Methoxyphenyl)-4-(trifluormethyl)-imidazol vom Smp. 197-202°.

20b) Zu einer Lösung von 11,14 g 2-(4-Methoxyphenyl)-4-(trifluormethyl)-imidazol in 107 ml Dimethylformamid werden zuerst 5,62 g Kalium-tert.butylat bei einer Innentemperatur von 0-5° zugegeben. Nach 20 Minuten wird unter Rühren eine Lösung von 3,8 ml Methyljodid in 15 ml Dimethylformamid langsam zugetropft und anschliessend das Reaktionsgemisch weitere 4 Stunden bei Raumtemperatur gerührt. Nach Entfernung des Dimethylformamids am Rotationsverdampfer wird der Rückstand mit Wasser aufgeschlämmt und die Wasserphase mehrmals mit Essigsäureäthylester extrahiert. Die vereinigten organischen Phasen werden über Natriumsulfat getrocknet, auf ein kleines Volumen eingeengt und mit Hexan bis zur beginnenden Kristallisation versetzt. Nach dem Abfiltrieren und Trocknen im Vakuum erhält man das 2-(4-Methoxyphenyl)-1-methyl-4-(trifluormethyl)-imidazol vom Smp. 87-88°.

(In den Mutterlaugen wird ein Gemisch von 2-(4-Methoxyphenyl)-1-methyl-4-(trifluormethyl)-imidazol und 2-(4-Methoxyphenyl)-1-methyl-(5-trifluormethyl)-imidazol nachgewiesen. Dieses Gemisch wird in der nachfolgenden Stufe 20c) eingesetzt und danach die resultierenden isomeren Phenole getrennt.)

20c) In einem Gemisch von 46,1 ml Essigsäure und 46,5 ml 48 %iger Bromwasserstoffsäure werden 9,3 g 2-(4-Methoxyphenyl)-1-methyl-4-(trifluormethyl)-imidazol gelöst und die Lösung 48 Stunden unter Rückfluss erhitzt. Die Lösung wird am Rotationsverdampfer zu einem dicken Brei eingeengt, welcher mit Wasser verdünnt, mit konz. Ammoniak auf pH 11 eingestellt und schliesslich mehrmals mit Essigsäureäthylester extrahiert wird. Nach Trocknen der organischen Phase und Filtration wird am Rotationsverdampfer soweit eingeengt, dass eine dicke Suspension resultiert. Nach dem Abkühlen wird diese filtriert, und das Kristallisat im Vakuum getrocknet, wobei man das 2-(4-Hydroxyphenyl)-1-methyl-4-(trifluormethyl)-1H-imidazol vom Smp. 220-221° erhält.

Aus den am Schluss des Beispiels 20b) beschriebenen Mutterlaugen erhält man in analoger Weise das Gemisch bestehend aus 2-(4-Hydroxyphenyl)-1-methyl-4-(trifluormethyl)-1H-imidazol und 2-(4-Hydroxyphenyl)-1-methyl-5-(trifluormethyl)-1H-imidazol, welches durch mehrmaliges Umkristallisieren aus Essigsäureäthylester getrennt werden kann, wobei das erhaltene 2-(4-Hydroxyphenyl)-1-methyl-5-(trifluormethyl)-1H-imidazol den Smp. 173-174° zeigt.

20d) 40 g 2-(4-Hydroxyphenyl)-1-methyl-4-(trifluormethyl)-1H-imidazol und 40 g wasserfreies Kaliumcarbonat werden in 400 ml Epichlorhydrin suspendiert und die Suspension während 35 Minuten unter Rückfluss erhitzt. Nach dem Abkühlen des Reaktionsgemischs im Eisbad wird filtriert und das Filtrat am Rotationsverdampfer eingeengt. Das zurückbleibende Oel wird in Aether gelöst, die Lösung mit Aktivkohle behandelt, filtriert und im Eis/Kochsalzbad abgekühlt. Dabei kristallisiert das 2-[4-(2,3-Epoxypropoxy)-phenyl]-1-methyl-4-(trifluormethyl)-1H-imidazol aus, welches abfiltriert und getrocknet wird. Smp. 69-70°.

## Beispiel 21

Eine Lösung von 19,4 g rohem 1-[N-Benzyl-2-(3-carbamoyl-4-hydroxy-phenoxy)-äthylamino]-3-[4-[1-methyl-5-(trifluormethyl)-1H-imidazol-2-yl]-phenoxy]-2-propanol in 100 ml Methanol wird nach Zusatz von 1 g Palladium-auf-Kohle-Katalysator (5 %) unter Normalbedingungen bis zur beendeten Wasserstoffaufnahme hydriert. Der Katalysator wird abfiltriert, das Filtrat am Rotationsverdampfer eingeengt und das zurückbleibende Oel an einer Kieselgelsäule mit einem Gemisch von Chloroform/Methanol/Ammoniak im Volumenverhältnis 40 : 10 : 1 chromatographiert. Das aus den Hauptfraktionen isolierte Produkt wird einmal aus Isopropanol umkristallisiert, danach in Methanol gelöst, die Lösung mit einer äquivalenten Menge Methansulfonsäure versetzt und nach Behandlung mit Tierkohle und Filtration mit Essigsäureäthylester bis zur beginnenden Kristallisation versetzt. Nach Kühlung im Eisbad wird abfiltriert und dabei das 1-[2-(3-Carbamoyl-4-hydroxy-phenoxy)-äthylamino]-3-[4-[1-methyl-5-(trifluormethyl)-1H-imidazol-2-yl]-phenoxy]-2-propanol-methansulfonat vom Smp. 126-130° erhalten.

Das als Ausgangsmaterial benötigte 1-[N-Benzyl-2-(3-carbamoyl-4-hydroxy-phenoxy)-äthylamino]-3-[4-[1-methyl-5-(trifluormethyl)-imidazol-2-yl]-phenoxy]-2-propanol kann wie folgt hergestellt werden :

21a) 25 g des nach Beispiel 20c) erhaltenen 2-(4-Hydroxyphenyl)-1-methyl-5-(trifluormethyl)-1H-imidazol und 25 g wasserfreies Kaliumcarbonat werden in 250 ml Epichlorhydrin suspendiert und das Gemisch 10 Minuten unter Rückfluss erhitzt. Anschliessend wird filtriert und das überschüssige Epichlorhydrin am Rotationsverdampfer entfernt. Der Rückstand wird in Aether gelöst, die Lösung mit Kieselgel und Aktivkohle verrührt, die Lösung filtriert und mit Hexan bis zur beginnenden Kristallisation versetzt. Nach dem Abfiltrieren erhält man das 2-[4-(2,3-Epoxy-propoxy)-phenyl]-1-methyl-5-(trifluormethyl)-1H-imidazol als farbloses Kristallisat vom Smp. 99-101°.

21b) Eine Lösung von 5 g des erhaltenen 2-[4-(2,3-Epoxy-propoxy)-phenyl]-1-methyl-5-(trifluormethyl)-1H-imidazol und 4,7 g 5-[2-Benzylamino)-äthoxy]-salicylamid in 100 ml Isopropanol wird während 15 Stunden unter Rückfluss erhitzt und das Lösungsmittel anschliessend am Rotationsverdampfer entfernt. Das dabei als Oel anfallende rohe 1-[N-Benzyl-2-(3-carbamoyl-4-hydroxy-phenoxy)-äthylamino]-3-[4-[1-methyl-5-(trifluormethyl)-1H-imidazol-2-yl]-phenoxy]-2-propanol wird als solches weiterverarbeitet.

## Beispiel 22

Eine Lösung von 11 g rohem 1-[N-Benzyl-2-(3-carbamoyl-4-hydroxy-phenoxy)-äthylamino]-3-[4-[1-isopropyl-4-(trifluormethyl)-1H-imidazol-2-yl]-phenoxy]-2-propanol in 110 ml Methanol wird nach Zusatz von 1 g Palladium-auf-Kohle-Katalysator (5 %) unter Normalbedingungen bis zur beendeten Wasserstoffaufnahme hydriert. Der Katalysator wird abfiltriert, das Lösungsmittel am Rotationsverdampfer entfernt und der Rückstand 2-mal aus Isopropanol umkristallisiert. Nach dem Abfiltrieren und Trocknen der Kristalle erhält man das 1-[2-(3-Carbamoyl-4-hydroxy-phenoxy)-äthylamino]-3-[4-[1-isopropyl-4-(trifluormethyl)-1H-imidazol-2-yl]-phenoxy]-2-propanol vom Smp. 143-145°.

Das als Ausgangsmaterial benötigte 1-[N-Benzyl-2-(3-carbamoyl)-4-hydroxy-phenoxy)-äthylamino]-3-[4-[1-isopropyl-4-(trifluormethyl)-imidazol-2-yl]-phenoxy]-2-propanol kann wie folgt hergestellt werden :

22a) Zu einer Lösung von 20 g 2-(4-Methoxyphenyl)-4-(trifluormethyl)-1H-imidazol in 200 ml Dimethylformamid werden 10 g Kalium-tert.butylat bei einer Innentemperatur von 0-50° zugegeben. Nach 20 Minuten gibt man 10,7 ml Isopropyljodid zu und rührt das Reaktionsgemisch anschliessend bei Raumtemperatur. Nach jeweils 24 Stunden gibt man weitere 10 g Kalium-tert.butylat und anschliessend 10,7 ml Isopropyljodid zu. Diese Massnahmen werden insgesamt dreimal wiederholt. Die Gesamtreaktionszeit beträgt 120 Stunden. Schliesslich wird das Lösungsmittel am Rotationsverdampfer entfernt, der Rückstand in Wasser aufgenommen und mehrmals mit Essigsäureäthylester extrahiert. Nach dem Abdampfen der organischen Phase wird der Rückstand an Kieselgel mit Essigsäureäthylester/Hexan chromatographiert, wobei aus den Hauptfraktionen das 2-(4-Methoxyphenyl)-1-isopropyl-4-(trifluormethyl)-1H-imidazol als gelbliches Oel isoliert wird.

Dieses Produkt wird direkt in der nächsten Stufe eingesetzt.

22b) Eine Lösung von 15,5 g 2-(4-Methoxyphenyl)-1-(isopropyl)-4-(triflethyl)-1H-imidazol in einem Gemisch von 80 ml Eisessig und 80 ml Bromwasserstoffsäure (48 %ig) wird während 24 Stunden unter Rückfluss erhitzt. Die Lösung wird am Rotationsverdampfer zu einem dicken Brei eingeengt, welcher mit Wasser verdünnt, mit konz. Ammoniak auf pH 11 neutralisiert und schliesslich mehrmals mit Essigsäureäthylester extrahiert wird. Nach Trocknung der organischen Phase über Magnesiumsulfat und Filtration wird am Rotationsverdampfer auf ein kleines Volumen eingeengt und mit Hexan bis zur beginnenden Kristallisation versetzt. Man erhält so das 2-(4-Hydroxyphenyl)-1-isopropyl-5-(trifluormethyl)-1H-imidazol vom Smp. 249-250°.

22c) Eine Suspension von 10 g 2-(4-Hydroxyphenyl)-1-isopropyl-4-(trifluormethyl)-1H-imidazol und 10 g wasserfreies Kaliumcarbonat in 100 ml Epichlorhydrin wird während 35 Minuten unter Rückfluss erhitzt. Das Reaktionsgemisch wird im Eisbad abgekühlt, filtriert und das Filtrat am Rotationsverdampfer eingeengt. Das zurückbleibende Oel wird in Aether gelöst, die Lösung mit Aktivkohle behandelt, filtriert und im Eis/Kochsalzbad abgekühlt. Hierbei kristallisiert das 2-[4-(2,3-Epoxypropoxy)-phenyl]-1-isopropyl-4-(trifluormethyl)-1H-imidazol aus welches nach dem Abfiltrieren und Trocknen den Smp. 98-99° zeigt.

## Beispiel 23

Eine Lösung von 9,7 g rohem 1-[N-Benzyl-2-(3-carbamoyl-4-hydroxy-phenoxy)-äthylamino]-3-[4-[1-(2-hydroxyäthyl)-4-(trifluormethyl)-1H-imidazol-2-yl]-phenoxy]-2-propanol in 100 ml Methanol wird nach Zusatz von 1 g Palladium-auf-Kohle-Katalysator (5 %) unter Normalbedingungen bis zur beendeten Wasserstoffaufnahme hydriert, wobei das Reaktionsprodukt teilweise aus der Lösung ausfällt. Die Suspension wird mit 200 ml Dioxan/Methanol 1 : 1 verdünnt, erwärmt, und durch ein Filterhilfsmittel filtriert. Das Filtrat wird am Rotationsverdampfer vom Lösungsmittel befreit und der Rückstand 2 mal aus Methanol umkristallisiert, wobei man das 1-[2-(3-Carbamoyl-4-hydroxy-phenoxy)-äthylamino]-3-[4-[1-(2-hydroxyäthyl)-4-(trifluormethyl)-1H-imidazol-2-yl]-phenoxy]-2-propanol vom Smp. 151-153° erhält.

Das als Ausgangsmaterial benötigte 1-[N-Benzyl-2-(3-carbamoyl-4-hydroxy-phenoxy)-äthylamino]-3-[4-[1-(2-hydroxyäthyl)-4-(trifluormethyl)-1H-imidazol-2-yl]-phenoxy]-2-propanol kann wie folgt hergestellt werden :

23a) Zu einer Lösung von 35,0 g 2-(4-Methoxyphenyl)-4-(trifluormethyl)-imidazol in 200 ml Dimethylformamid gibt man 35,0 g Kalium-tert.butylat bei einer Innentemperatur von 0-50° zu. Nach 20 Minuten tropft man eine Lösung von 14,7 ml 2-Hydroxyäthyljodid in 100 ml Dimethylformamid innerhalb 5 Minuten zu, rührt anschliessend während 120 Stunden bei Raumtemperatur und entfernt dann das Lösungsmittel am Rotationsverdampfer, nimmt den Rückstand in Wasser auf und extrahiert mehrmals mit Essigsäureäthylester. Die organischen Phasen werden vereinigt, das Lösungsmittel entfernt und der Rückstand an Kieselgel mit dem Lösungsmittelgemisch Essigsäureäthylester/Hexan 1 : 1 chromatographiert, wobei man das 1-(2-Hydroxyäthyl)-2-(4-methoxyphenyl)-4-(trifluormethyl)-1H-imidazol als kristallisierendes Oel aus den Hauptfraktionen isoliert.

23b) Eine Lösung von 14,6 g 1-(2-Hydroxyäthyl)-2-(4-methoxyphenyl)-4-(trifluormethyl)-1H-imidazol und 8,7 g DL-Methionin in 90 ml Methansulfonsäure wird während 24 Stunden bei 110° gerührt, anschliessend abgekühlt, auf Eis gegossen und mit Ammoniak auf pH 10 eingestellt. Das Gemisch wird mehrmals mit Essigsäureäthylester extrahiert, die vereinigten organischen Phasen über Natriumsulfat getrocknet, filtriert und das Filtrat auf kleines Volumen eingeengt. Durch Zugabe von Hexan kann das 1-(2-Hydroxyäthyl)-2-(4-hydroxyphenyl)-4-(trifluormethyl)-1H-imidazol vom Smp. 186-188° kristallin erhalten werden.

23c) Eine Suspension von 3,26 g 1-(2-Hydroxyäthyl)-2-(4-hydroxyphenyl)-4-(trifluormethyl)-1H-imidazol und 9,26 g wasserfreies Kaliumcarbonat in 95 ml Epichlorhydrin wird unter Rühren während 1 Stunde am Rückfluss erhitzt. Anschliessend wird das Gemisch im Eisbad abgekühlt, filtriert und das Filtrat am Rotationsverdampfer vom Lösungsmittel befreit. Das zurückbleibende Oel wird mit Essigsäureäthylester an Kieselgel chromatographiert. Die Hauptfraktionen werden vereinigt, und durch Zugabe von Hexan zur Kristallisation gebracht, wobei man das 2-[4-(2,3-Epoxy-propoxy)-phenyl]-1-(2-hydroxyäthyl)-4-(trifluormethyl)-1H-imidazol vom Smp. 110-112° erhält.

## Beispiel 24

Eine Lösung von 1,0 g rohem 1-[N-Benzyl-2-(3-carbamoyl-4-benzyloxy-phenoxy)-äthylamino]-3-[4-[1,4-bzw. 1,5-dimethyl-1H-imidazol-2-yl]-phenoxy]-2-propanol in 10 ml Methanol wird nach Zusatz von 0,1 g Palladium-auf-Kohle-Katalysator (5 %) unter Normalbedingungen bis zur beendeten Wasserstoffaufnahme hydriert, der Katalysator anschliessend abfiltriert und das Lösungsmittel am Rotationsverdampfer entfernt. Der Rückstand wird aus einem Essigsäureäthylester-Aether-Gemisch umkristallisiert, wobei man das 1-[2-(3-Carbamoyl-4-hydroxy-phenoxy)-äthylamino]-3-[4-[1,4-bzw. 1,5-dimethyl-1H-imidazol-2-yl]-phenoxy]-2-propanol als Isomerengemisch im Verhältnis ca. 1 : 1 erhält.

Das als Ausgangsmaterial benötigte 1-[N-Benzyl-2-(3-carbamoyl-4-benzyloxy-phenoxy)-äthylamino]-3-[4-[1,4-bzw. 1,5-dimethyl-1H-imidazol-2-yl]-phenoxy]-2-propanol kann wie folgt hergestellt werden :

24a) Eine Lösung von 40 g 5-[2-(Benzylamino)-äthoxy]-[O-benzylsalicylamid in 800 ml Methanol wird während 16 Stunden unter Rückfluss gerührt. Die dabei erhaltene den 1-[N-Benzyl-2-(3-carbamoyl-4-benzyloxy-phenoxy)-äthylamino]-3-(4-benzaldehyd) enthaltende Lösung wird wie folgt weiterverarbeitet.

24b) Die Reaktionslösung wird mit weiteren 800 ml Methanol verdünnt. Anschliessend werden 69,1 g Natriumacetat und 177 ml Methylglyoxal (30 %ig in Wasser) zugegeben und schliesslich im Verlaufe von 15 Minuten 270 ml konz. Ammoniak zugetropft. Nach 5 Tagen Rühren bei Raumtemperatur wird das Lösungsmittel am Rotationsverdampfer weitgehend entfernt, der Rückstand mit Wasser verdünnt und mit viel Methylenchlorid extrahiert. Die organische Phase wird zur Trockne verdampft und der Rückstand an Kieselgel mit einem Gemisch von Chloroform/Methanol/Ammoniak (700 : 50 : 1) chromatographiert. Die Hauptfraktionen werden vereinigt, das Lösungsmittel am Rotationsverdampfer entfernt und das als gelblicher Schaum resultierende rohe 1-[N-Benzyl-2-(3-carbamoyl-4-benzyloxy-phenoxy)-äthylamino]-3-[4-[4-methyl-1H-imidazol-2-yl]-phenoxy]-2-propanol im Hochvakuum gründlich getrocknet und als solches in der nächsten Stufe eingesetzt.

24c) Eine Lösung von 1,6 g 1-[N-Benzyl-2-(3-carbamoyl-4-benzyloxyphenoxy)-äthylamino]-3-[4-[4-methyl-1H-imidazol-2-yl]-phenoxy]-2- propanol in 20 ml Dimethylformamid wird mit 0,311 g Kalium-tert.butylat und nach 15 Minuten tropfenweise mit 0,487 g Methyljodid versetzt. Nach 3 Stunden Rühren bei Raumtemperatur wird das Lösungsmittel am Rotationsverdampfer entfernt und der Rückstand an Kieselgel mit dem Lösungsmittelgemisch Chloroform/Methanol/Ammoniak (700 : 50 : 1) chromatographiert. Aus den Hauptfraktionen isoliert man das 1-[N-Benzyl-2-(3-carbamoyl-4-benzyloxy-phenoxy)-äthylamino]-3-[4-[1,4-bzw. 1,5-dimethyl-1H-imidazol-2-yl]-phenoxy]-2-propanol als Isomerengemisch ca. 1 : 1 in Form eines braunen Oels, welches als solches in der nächsten Stufe eingesetzt wird.

## Beispiel 25

Eine Lösung von 2,95 g rohem 1-[N-Benzyl-2-(3-carbamoyl-4-hydroxy-phenoxy)-äthylamino]-3-[4-[4-(trifluormethyl)-thiazol-2-yl]-phenoxy]-2-propanol in 80 ml Methanol wird nach Zusatz von insgesamt 5 g Palladium-auf-Kohle-Katalysator (5 %) bei 60° einem Druck von 4 bar bis zur beendeten Wasserstoffaufnahme hydriert, wobei das Reaktionsprodukt teilweise aus der Lösung ausfällt. Die Suspension wird mit 50 ml Dioxan/Methanol verdünnt, erwärmt und durch ein Filterhilfsmittel filtriert. Das Filtrat wird am Rotationsverdampfer vom Lösungsmittel befreit und der Rückstand 2-mal aus Methanol umkristallisiert, wobei man das 1-[2-(3-Carbamoyl-4-hydroxy-phenoxy)-äthylamino]-3-[4-[4-(trifluormethyl)-thiazol-2-yl]-phenoxy]-2-propanol vom Smp. 202-203° erhält.

Das als Ausgangsmaterial verwendete 1-[N-Benzyl-2-(3-carbamoyl-4-hydroxy-phenoxy)-äthylamino]-3-[4-[4-(trifluormethyl)-thiazol-2-yl]-phenoxy]-2-propanol kann wie folgt hergestellt werden :

25a) Eine Lösung von 9,18 g 4-Hydroxythiobenzamid in 90 ml Aethanol wird tropfenweise mit 12,6 g 1-Brom-3,3,3-trifluor-acetonversetzt, das Gemisch während 3 Stunden bei Raumtemperatur gerührt, dann mit 1 ml konz. Schwefelsäure versetzt und anschliessend 18 Stunden unter Rückfluss erhitzt. Anschliessend wird das Lösungsmittel am Rotationsverdampfer entfernt, der Rückstand mit Eiswasser verdünnt und das Gemisch mit Essigsäureäthylester extrahiert. Die vereinigten organischen Phasen werden getrocknet und auf ein kleines Volumen eingedampft. Durch Zugabe von Hexan bringt man das 2-(4-Hydroxyphenyl)-4-(trifluormethyl)-thiazol zur Kristallisation, Smp. 161-162°.

25b) Ein Gemisch von 5 ml Epichlorhydrin und 0,5 g Kaliumcarbonat wird unter Rückfluss erwärmt. In einer Portion werden 0,5 g 2-(4-Hydroxyphenyl)-4-(trifluormethyl)-thiazol zugegeben und das Reaktionsgemisch anschliessend weitere 15 Minuten unter Rückfluss erhitzt. Nach dem Abkühlen wird filtriert, das Filtrat am Rotationsverdampfer eingedampft und der Rückstand in 6 ml Dichlormethan aufgenommen, die Lösung mit 100 mg Tetrabutylammoniumhydrogensulfat und 6 ml 2-n. Natronlauge versetzt und 3 Stunden bei Raumtemperatur gerührt. Die organische Phase wird abgetrennt, gewaschen, getrocknet und schliesslich das Lösungsmittel am Rotationsverdampfer entfernt. Der Rückstand wird aus Aether/Hexan kristallisiert, wobei man das 2-[4-(2,3-Epoxy-propoxy)-phenyl]-4-(trifluormethyl)-thiazol vom Smp. 97-98° erhält.

25c) Eine Lösung von 7,0 g 2-[4-(2,3-Epoxy-propoxy)-phenyl]-4-(trifluormethyl)-thiazol und 6,01 g 5-[2-(Benzylamino)-äthoxy]-salicylamid in 150 ml Isopropanol wird während 18 Stunden unter Rückfluss erhitzt und das Lösungsmittel anschliessend am Rotationsverdampfer entfernt. Das dabei als Oel anfallende 1-[N-Benzyl-2-(3-carbamoyl-4-hydroxy-phenoxy)-äthylamino]-3-[4-[4-(trifluormethyl)-thiazol-2-yl]-phenoxy]-2-propanol wird als solches weiterverarbeitet.

## Beispiel 26

Eine Lösung von 3,0 g rohem 1-[N-Benzyl-2-(3-carbamoyl-4-hydroxy-phenoxy-äthylamino]-3-[4-(thiazol-2-yl)-phenoxy]-2-propanol in einem Gemisch von 30 ml Eisessig und 3 ml Trifluoressigsäure wird nach Zusatz von 0,69 Palladium-auf-Kohle-Katalysator (10 %) bei 45° und Normaldruck bis zur beendeten Wasserstoffaufnahme hydriert. Die Suspension wird durch ein Filterhilfsmittel filtriert, das Filtrat vom Lösungsmittel befreit, der Rückstand mit Eiswasser versetzt, mit Ammoniak auf ca. pH 9 eingestellt und mit Essigsäureäthylester mehrmals extrahiert. Die vereinigten organischen Phasen werden über Natriumsulfat getrocknet, eingedampft und der Rückstand aus Isopropanol umkristallisiert, wobei man das 1-[2-(3-Carbamoyl-4-hydroxy-phenoxy)-äthylamino]-3-[4-(thiazol-2-yl)-phenoxy]-2-propanol vom Smp. 136-138° erhält.

Das als Ausgangsmaterial verwendete 1-[N-Benzyl-2-(3-carbamoyl-4-hydroxy-phenoxy)-äthylamino]-3-[4-(thiazol-2-yl)-phenoxy]-2-propanol kann wie folgt hergestellt werden :

26a) Zu einem unter Rückfluss erwärmten Gemisch von 5 ml Epichlorhydrin und 0,79 g Kaliumcarbonat gibt man in einer Portion 0,5 g 2-(4-Hydroxyphenyl)-thiazol zu und erhitzt das Reaktionsgemisch anschliessend weitere 20 Minuten unter Rückfluss. Nach dem Abkühlen wird filtriert, das Filtrat am Rotationsverdampfer eingedampft und der Rückstand in 6 ml Dichlormethan gelöst, die Lösung mit 100 mg Tetrabutylammoniumhydrogensulfat und 6 ml 2-n. Natronlauge versetzt und 3 Stunden bei Raumtemperatur gerührt. Die organische Phase wird abgetrennt, mit Wasser gewaschen, über Natriumsulfat getrocknet und anschliessend das Lösungsmittel am Rotationsverdampfer entfernt. Der Rückstand wird aus einen Aether/Hexan-Gemisch umkristallisiert, wonach man 2-[4-(2,3-Epoxy-propoxy)-phenyl]-thiazol vom Smp. 82-83° erhält.

26b) Eine Lösung von 6,50 g 2-[4-(2,3-Epoxy-propoxy)-phenyl]-thiazol und 7,25 g 5-[2-(Benzylamino)-äthoxy]-salicylamid in 150 ml Isopropanol wird während 18 Stunden am Rückfluss erhitzt und das Lösungsmittel anschliessend am Rotationsverdampfer entfernt. Das dabei als Oel anfallende 1-[N-Benzyl-2-(3-carbamoyl-4-hydroxy-phenoxy)-äthylamino]-3-[4-(thiazol-2-yl)-phenoxy]-2-propanol wird als solches weiterverarbeitet.

## Beispiel 27

Eine Lösung von 8,39 1-Amino-3-[4-(1-methyl-4-(trifluormethyl)-1H-imidazol-2-yl)-phenoxy]-2-propanol in 250 ml Methanol wird mit 4,28 ml 5,9-n. methanolischer Chlorwasserstoffsäure versetzt. Durch Zusatz von festem Kaliumhydroxid wird auf pH 8-9 eingestellt ; anschliessend werden 25 g Molekularsiebe (Porengrösse 3 Å), 5,6 g 2,3-Dihydro-2,2-dimethyl-7-(2-oxo-äthoxy)-4H-1,3-benzoxazin-4-on und schliesslich 0,59 g Natriumcyanoborhydrid zugegeben. Das die entsprechende Schiff'sche Base enthaltende Gemisch wird 18 Stunden bei Raumtemperatur und nach Zugabe von 50 ml 5,9-n. methanolischer Salzsäure noch eine Stunde unter Rückfluss gerührt, filtriert und das Lösungsmittel am Rotationsverdampfer entfernt. Der Rückstand wird an Kieselgel mit einem Chloroform/Methanol/Ammoniak-Gemisch (40 : 50 : 5) chromatographiert, die Hauptfraktionen vom Lösungsmittel befreit und der Rückstand aus Methanol umkristallisiert, wobei man das 1-[2-(3-Carbamoyl-4-hydroxy-phenoxy)-äthylamino]-3-[4-[1-methyl-4-(trifluormethyl)-1H-imidazol-2-yl]-phenoxy]-2-propanol vom Smp. 195-197° erhält.

## Beispiel 28

Eine Lösung von 1 g des nach Beispiel 3 erhaltenen 1-[2-(3-Carbamoyl-4-hydroxy-phenoxy)-äthylamino]-3-[4-[4-(trifluormethyl)-1H-imidazol-2-yl]-phenoxy]-2-propanol in 20 ml Dimethylformamid wird unter Rühren im Eisbad mit 0,48 g Kalium-tert.butylat versetzt. Nach 10 Minuten wird eine Lösung von 0,3 g Methyljodid in 1 ml Dimethylformamid im Verlaufe von 20 Minuten zugetropft. Es wird 2 Stunden bei Raumtemperatur nachgerührt, das Lösungsmittel am Rotationsverdampfer entfernt und der Rückstand an Kieselgel mit einem Lösungsmittelgemisch Chloroform/Methanol/Ammoniak (700 : 50 : 1) chromatographiert, wobei man die beiden isomeren Verbindungen 1-[2-(3-Carbamoyl-4-hydroxyphenoxy)-äthyla-

mino]-3-[4-[1-methyl-4-(trifluormethyl)-1H-imidazol-2-yl]-phenoxy]-2-propanol vom Smp. 195-197° und das 1-[2-(3-Carbamoyl-4-hydroxy-phenoxy)-äthylamino]-3-[4-[1-methyl-5-(trifluormethyl)-1H-imidazol-2-yl]-phenoxy]-2-propanol vom Smp. 157-159° erhält.

Die beschriebene Methylierung kann auch mittels Diazomethan wie folgt durchgeführt werden :

Eine Lösung von 1 g 1-[2-(3-Carbamoyl-4-hydroxy-phenoxy)-äthylamino]-3-[4-[4-(trifluormethyl)-1H-imidazol-2-yl]-phenoxy]-2-propanol in 30 ml Methanol wird mit einem Ueberschuss einer ätherischen Diazomethan-Lösung versetzt und während einer Stunde bei Raumtemperatur gerührt.

Aufarbeitung der Reaktionslösung und Chromatographie erfolgen wie oben beschrieben, wobei die beiden identifizierten isomeren Endprodukte erhalten werden.

## Beispiel 29

Eine Lösung von 5,2 g 1-[N-Benzyl-2-(3-carbamoyl-4-hydroxyphenoxy)-äthylamino]-2-[4-(1-methyl-2-oxo-dihydropyridin-6-yl)-phenoxy]-2-propanol in 100 ml Methanol wird in Gegenwart von 1 g Palladium-auf-Kohle-Katalysator (5 %) unter Normalbedingungen hydriert. Das Hydriergemisch wird abgesaugt, das Filtrat zur Trockne eingedampft und der Rückstand mit Aethylacetat gewaschen. Nach dem Umkristallisieren aus Isopropanol erhält man reines 1-[4-(1,6-Dihydro-1-methyl-6-oxo-2-pyridinyl)-phenoxy]-3-[2-(3-carbamoyl-4-hydroxy-phenoxy)-äthylamino]-2-propanol vom Smp. 148-150°.

Die als Ausgangsmaterial benötigte N-Benzyl-Verbindung kann wie folgt hergestellt werden :

29a) Ein Gemisch von 5,54 g 6-(4-Benzyloxy-phenyl)-α-pyridon (J. Thesing und A. Müller, Chem. Ber. *1957*, 711 vom Smp. 210-212°), 4,2 g Kaliumcarbonat und 2 ml Methyljodid in 40 ml Dimethylformamid wird während 48 Stunden bei Raumtemperatur gerührt. Das Lösungsmittel wird unter Vakuum abdestilliert und der Rückstand in 190 ml Methylenchlorid verrührt. Die anorganischen Teile werden abgetrennt und das Filtrat zur Trockene eingedampft. Es verbleibt ein hellbraunes Pulver, das man mit 0,5 ml Methyljodid versetzt und im Druckrohr während 13 Stunden bei einer Badtemperatur von 150° erhitzt. Man löst die Reaktionsmasse in 100 ml Methylenchlorid, wäscht nacheinander mit Wasser und gesättigter wässriger Natriumchloridlösung, trocknet über Natriumsulfat und destilliert das Lösungsmittel ab. Der Rückstand wird aus 80 ml Aethylacetat umkristallisiert, wonach man 6-(4-Benzyloxy-phenyl)-1-methyl-α-pyridon vom Smp. 167-170° erhält.

29b) Eine Lösung von 18,4 g 6-(4-Benzyloxy-phenyl)-1-methyl-α-pyridon in 550 ml Methanol wird in Gegenwart von 10 g Palladium-auf-Kohle vom Katalysator (5 %) hydriert, das Hydriergemisch vom Katalysator abfiltriert und mit Methanol gewaschen. Aus der methanolischen Lösung kristallisiert nach Einengen das 6-(4-Hydroxy-phenyl)-1-methyl-α-pyridon vom Smp. 236-238° aus.

29c) Ein Gemisch von 6,35 g der nach Beispiel 29b) erhaltenen Substanz, 63 ml Epichlorhydrin und 8,7 g Kaliumcarbonat wird während 2 Stunden bei einer Badtemperatur von 125° erhitzt. Das Reaktionsgemisch wird darauf mit Methylenchlorid verdünnt, abgesaugt und das Filtrat zur Trockene eingedampft. Das verbleibende Oel-Kristallgemisch wird aus 10 ml Dimethoxyäthan umkristallisiert, wonach man das 3-[4-(1-Methyl-2-oxo-dihydropyridin-6-yl)-phenoxy]-1,2-epoxipropan erhält.

29d) Ein Gemisch aus 3,8 g des nach Beispiel 29c) erhaltenen Epoxids und 4,3 g 5-[2-(Benzylamino)-äthoxy]-salicylamid in 31 ml Isopropanol wird bei einer Badtemperatur von 90° erwärmt. Aus der anfänglichen Lösung entsteht eine Suspension, die nach 4 Stunden abgekühlt und abgesaugt wird. Die erhaltenen Kristalle werden aus 40 ml Methyl-cellosolve umkristallisiert, wonach man 1-[N-Benzyl-2-(3-carbamoyl-4-hydroxy-phenoxy)-äthylamino]-3-[4-(1-methyl-2-oxo-dihydropyridin-6-yl)-phenoxy]-2-propanol vom Smp. 177-179° erhält.

## Beispiel 30

Eine Lösung von 1,35 g 1-[4-(3,4-Dihydro-3,6-dimethyl-4-oxo-2-pyrimidinyl)-phenoxy]-3-[2-(3-carbamoyl-4-hydroxy-phenoxy)-N-benzylaminoäthyl]-2-propanol in 20 ml Methanol wird in Gegenwart von 0,3 g Palladium-auf-Kohle-Katalysator (5 %) unter Normalbedingungen hydriert. Zur Aufarbeitung wird das warme Reaktionsgemisch abgesaugt, mit warmen Methanol gewaschen und das Filtrat eingedampft. Den Rückstand versetzt man mit 45 ml Isopropanol, rührt 1 Stunde und filtriert einen amorphen Niederschlag ab. Das Filtrat wird mit 3-n. ätherischem Chlorwasserstoff auf pH ~ 1 gebracht, wobei 1-[4-(3,4-Dihydro-3,6-dimethyl-4-oxo-2-pyrimidinyl)-phenoxy]-3-[2-(3-carbamoyl-4-hydroxy-phenoxy)-äthylamino]-2-propanol als Hydrochlorid ausfällt. Nach dem Umkristallisieren aus Methanol wird das Produkt 18 Stunden bei 80° und 0,1 mm Hg getrocknet ; es zeigt den Smp. 174-176° (Gasentwicklung) ; die Verbindung enthält Kristallwasser.

Die als Ausgangsmaterial benötigte N-Benzyl-Verbindung kann wie folgt hergestellt werden :

30a) Zu einer Lösung von 3,5 g 4-Benzyloxy-N-methyl-benzamidin in 35 ml Acetessigester fügt man 20 mg Natriummethylat und rührt das Gemisch bei einer Badtemperatur von 100° während 17 Stunden. Die erhaltene braune Lösung wird unter Vakuum eingedampft und der Rückstand in Essigsäureäthylester gelöst. Man wäscht diese Lösung nacheinander mit 0,5 n. Natronlauge, Wasser, dann mit wässriger gesättigter Natriumchloridlösung, trocknet die organische Phase über Natriumsulfat und dampft ein. Der

Rückstand wird über Kieselgel chromatographiert (Methylenchlorid-Methanol 99 : 1), wonach man kristallines 2-(4-Benzyloxyphenyl)-1,4-dimethyl-1H-pyrimidin-6-on erhält.

30b) Eine Lösung von 1,8 g der nach Beispiel 30a) erhaltenen Verbindung in 20 ml Methanol wird in Gegenwart von 0,3 g Palladium-auf-Kohle-Katalysator (5 %) unter Normalbedingungen hydriert. Das nach dem Aufarbeiten erhaltene kristalline Produkt wird aus Methylcellosolve umkristallisiert, wonach man 2-(4-Hydroxyphenyl)-1,4-dimethyl-1H-pyrimidin-6-on vom Smp. ca. 260° erhält.

30c) Ein Gemisch von 0,6 g der nach Beispiel 30b) erhaltenen Verbindung mit 10 ml Epichlorhydrin und 0,58 g Kaliumcarbonat wird während 1 Stunde bei einer Badtemperatur von 130° gerührt. Nach dem Aufarbeiten erhält man 1-[4-(3,4-Dihydro-3,6-dimethyl-4-oxo-2-pyrimidinyl)-phenoxy]-2,3-epoxipropan als gelbes viskoses Oel.

30d) 0,9 g des erhaltenen rohen Produktes werden in 5 ml Isopropanol mit 0,87 g 5-[2-(Benzylamino)-äthoxy]-salicylamid während 7 Stunden bei einer Badtemperatur von 95° gerührt. Man verdünnt darauf mit 10 ml Isopropanol und fügt 1,5 ml 3-n. ätherischer Chlorwasserstofflösung zu. Man dekantiert vom gebildeten klebigen Niederschlag ab und behandelt diesen mit wässriger Kaliumbicarbonatlösung und Essigsäureäthylester. Aus der organischen Phase wird nach dem Aufarbeiten das 1-[4-(3,4-Dihydro-3,6-dimethyl-4-oxo-2-pyrimidinyl)-phenoxy]-3-[2-(3-carbamoyl-4-hydroxy-phenoxy)-N-benzylaminoäthyl]-2-propanol erhalten, welches als solches weiterverarbeitet wird.

## Beispiel 31

0,8 g l-Amino-3-[4-(1-methyl-4-(trifluormethyl)-1H-imidazol-2-yl)-phenoxy]-2-propanol und 0,6 g 5-(2-Oxopropoxy)-salicylamid werden in 20 ml Methanol gelöst. Die die gebildete entsprechende Schiff'sche Base enthaltende Lösung wird nach Zusatz vom 1 Tropfen konz. Schwefelsäure und 0,2 g Platin-auf-Kohle-Katalysator (10 %) bis zur Beendigung der Wasserstoff-Aufnahme (1-Mol-Aequivalent) hydriert. Der Katalysator wird abfiltriert, und das Filtrat eingedampft. Das verbleibende, bräunliche Oel stellt das 1-[2-(3-Carbamoyl-4-hydroxy-phenoxy)-1-methyl-äthylamino]-3-[4-(1-methyl-4-(trifluormethyl)-1H-imidazol-2-yl)-phenoxy]-2-propanol als Diastereoisomeren-Gemisch dar. Es zeigt ein mit der angenommenen Struktur vereinbares Protonen-NMR-Spektrum. (100 MHz, DMSO-$d_6$).

## Beispiel 32

Eine Lösung von 4,5 g des Natriumsalzes von 1-[2-(3-Carboxy-4-hydroxy-phenoxy)-äthylamino]-3-[4-[1-methyl-4-(trifluormethyl)-1H-imidazol-2-yl]-phenoxy]-2-propanol in 50 ml 1-n. methanolischer Salzsäure wird während 48 Stunden unter Rückfluss erwärmt. Das Reaktionsgemisch wird abgekühlt, vom Kochsalz abfiltriert und das Filtrat am Rotationsverdampfer vom Lösungsmittel befreit. Der den entsprechenden Methylester darstellende Rückstand wird noch 2-mal mit Methanol behandelt und dieses dann abgedampft, anschliessend in 20 ml Dioxan aufgenommen, die Lösung mit 50 ml konz. wässrigem Ammoniak versetzt und das Gemisch 40 Stunden bei Raumtemperatur gerührt. Nach dem Abdampfen des Lösungsmittels am Rotationsverdampfer wird der Rückstand mit Wasser verrührt, der Niederschlag abfiltriert und nach dem Trocknen aus Methanol umkristallisiert, wobei man das 1-[2-(3-Carbamoyl-4-hydroxy-phenoxy)-äthylamino]-3-[4-[1-methyl-4-(trifluormethyl)-1H-imidazol-2-yl]-phenoxy]-2-propanol vom Smp. 195-197° erhält.

Das als Ausgangsmaterial benötigte Natriumsalz des 1-[2-(3-Carboxy-4-hydroxy-phenoxy)-äthylamino]-3-[4-[1-methyl-4-(trifluormethyl)-1H-imidazol-2-yl]-phenoxy]-2-propanol kann wie folgt erhalten werden :

Eine Lösung von 5,0 g 1-[2-(3-Carbamoyl-4-hydroxy-phenoxy)-äthylamino]-3-[4-[1-methyl-4-(trifluormethyl)-1H-imidazol-2-yl]-phenoxy]-2-propanol in 75 ml 1-n. Natriumhydroxidlösung wird unter Stickstoff während 20 Stunden unter Rückfluss gerührt. Die Lösung wird abgekühlt, das ausgefallene Salz abfiltriert und aus Wasser umkristallisiert. Nach dem Trocknen bei 80° am Hochvakuum erhält man das Natriumsalz von 1-[2-(3-Carboxy-4-hydroxy-phenoxy)-äthylamino]-3-[4-[1-methyl-4-(trifluormethyl)-1H-imidazol-2-yl]-phenoxy]-2-propanol vom Smp, 236-238°.

## Beispiel 33

Eine Lösung von 1,35 g 1-[N-Benzyl-2-(3-carbamoyl-4-hydroxy-phenoxy)-äthylamino]-3-[4-(1,4-dimethyl-1H-pyrimidin-6-on-2-yl)-phenoxy]-2-propanol in 20 ml Methanol wird in Gegenwart von 0,3 g Palladium-auf-Kohle-Katalysator (5 %) unter Normalbedingungen hydriert. Zur Aufarbeitung wird das warme Reaktionsgemisch vom Katalysator abgesaugt, der Filterrückstand mit warmem Methanol gewaschen und das Filtrat eingedampft. Den Rückstand versetzt man mit 45 ml Isopropanol, rührt 1 Stunde und filtriert einen amorphen Niederschlag ab. Das Filtrat wird mit 3-n. ätherischer Salzsäure auf pH ~ 1 eingestellt, wobei ein Niederschlag ausfällt, der aus Methanol umkristallisiert wird. Man erhält das 1-[2-(3-Carbamoyl-4-hydroxy-phenoxy)-äthylamino]-3-[4-(1,4-dimethyl-1H-pyrimidin-6-on-2-yl)-phenoxy]-2-propanol-Hydrochlorid als Halbhydrat.

Nach dem Trocknen bei 100° und 0,1 mm Hg während 18 Stunden zeigt die Verbindung den Smp.

160-162° (Gasentwicklung). Die Substanz ist hygroskopisch.

Beispiel 34

Analog Beispiel 12 setzt man 19,5 g 3-[4-(2,3-Epoxy-propoxy)-phenyl]-4-methyl-5-methylthio-4H-1,2,4-triazol mit 11,0 g 5-(2-Aminoäthoxy)-salicylamid in 70 ml Dimethylsulfoxid um. Das beim Versetzen mit Wasser erhaltene harzartige Rohprodukt wird mit Dioxan ausgekocht, die Dioxanlösung eingedampft und der Rückstand aus Methanol umkristallisiert. Man erhält das 1-[2-(3-Carbamoyl-4-hydroxy-phenoxy)-äthylamino]-3-[4-(4-methyl-5-methylthio-4H-1,2,4-triazol-3-yl)-phenoxy]-2-propanol vom Smp. 123-125°. Das Ausgangsmaterial kann auf folgende Weise hergestellt werden :

34a) 30,4 g 3-(4-Methoxyphenyl)-4-methyl-5-methylthio-4H-1,2,4-triazol und 38,5 g D,L-Methionin werden in 230 ml Methansulfonsäure 20 Stunden auf 110-120° erhitzt. Nach dem Aufarbeiten analog Beispiel 15b) erhält man 3-(4-Hydroxyphenyl)-4-methyl-5-methylthio-4H-1,2,4-triazol vom Smp. 207-210° (aus Isopropanol).

34b) Ein Gemisch von 18,5 g der nach 34a) erhaltenen Verbindung, 90 g Kaliumcarbonat und 21 ml Epichlorhydrin in 1 Liter Aceton wird 24-30 Stunden unter Rückfluss gerührt. Nach dem Abfiltrieren und Eindampfen des Filtrats erhält man rohes 3-[4-(2,3-Epoxy-propoxy)-phenyl]-4-methyl-5-methylthio-4H-1,2,4-triazol als Oel, welches als solches weiterverwendet wird.

Durch Chromatographie einer Probe an Silicagel und Elution mit Chloroform, dem 1 % Methanol zugesetzt wird, erhält man die Verbindung in reiner Form als Kristalle vom Smp. 129-131°.

Beispiel 35

Die Lösungen von 5,0 g rohem 1,5-Dimethyl-2-[3-(2,3-epoxy-propoxy)-phenyl]-pyrrol in 15 ml Isopropanol und von 3,0 g 5-(2-Aminoäthoxy)-salicylamid in 30 ml Isopropanol werden vereinigt und während 2,5 Stunden unter Rückfluss gekocht. Durch Zugabe von 50 ml Methanol wird ein Teil des ausgefallenen Reaktionsproduktes in Lösung gebracht. Die filtrierte Lösung wird zur Trockne verdampft und der Rückstand 2-mal aus Aethylacetat umkristallisiert, wonach man 1-[2-(3-Carbamoyl-4-hydroxy-phenoxy)-äthylamino]-3-[3-(1,5-dimethylpyrrol-2-yl)-phenoxy]-2-propanol vom Smp. 108-112° erhält.

35a) Zu einer Lösung von 2,5 g Kaliumcyanid in 250 ml Dimethylformamid wird unter Rühren bei ca. 40° zuerst eine Lösung von 106 g 3-Benzyloxybenzaldehyd in 250 ml Dimethylformamid und hierauf eine Lösung von 26,5 g 1-Buten-3-on in 500 ml Dimethylformamid zugetropft. Nach beendeter Zugabe wird noch 2-3 Stunden bei 40° nachgerührt, das Reaktionsgemisch auf 3 Liter Wasser gegossen und mit je 500 ml Chloroform 3-mal extrahiert. Die vereinigten Chloroform-Extrakte werden nacheinander mit je 250 ml Wasser, 0,5-n. Salzsäure und gesättigter Natriumbicarbonat-Lösung gewaschen, über Magnesiumsulfat getrocknet und eingedampft, wonach man rohes 1-(3-Benzyloxy)-1,4-pentandion als dunkles Oel erhält, welches ohne weitere Reinigung weiterverarbeitet wird.

35b) Eine Lösung von 56 g des erhaltenen rohen 1-(3-Benzyloxy)-1,4-pentandion in 200 ml Eisessig wird mit 25 g Methylaminacetat versetzt und das Gemisch 24 Stunden unter Rückfluss gekocht. Nach dem Eindampfen verbleibt ein dunkelrotes Oel, das in Aether gelöst wird und nacheinander mit je 50 ml Wasser, 2-n. Natronlauge, Wasser und gesättigter wässriger Natriumchloridlösung gewaschen wird. Nach dem Trocknen und Eindampfen erhält man 2-(3-Benzyloxyphenyl)-1,5-di-methyl-pyrrol als braunes Oel, welches roh weiterverwendet wird.

35c) 35,7 g der nach 35b) erhaltenen rohen Verbindung werden in einem Gemisch von je 250 ml Methanol und Tetrahydrofuran in Gegenwart von 8 g Palladium-auf-Kohle-Katalysator (5 %) hydriert. Nach Aufnahme von 1 Mol-Aequivalent Wasserstoff kommt die Hydrierung zum Stillstand. Nach dem Abfiltrieren des Katalysators und Abdampfen des Lösungsmittels erhält man rohes 1,5-Dimethyl-2-(3-hydroxyphenyl)-pyrrol als braunes Oel.

35d) Ein Gemisch von 10 g des erhaltenen rohen 1,5-Dimethyl-2-(3-hydroxyphenyl)-pyrrol, 50 ml Epichlorhydrin und 15,2 g Kaliumcarbonat wird unter Rühren 3 Stunden zum Sieden erhitzt. Das Reaktionsgemisch wird abgekühlt, filtriert und eingedampft. Der Eindampfrückstand wird zwischen 200 ml Aether und 20 ml 2-n. Natronlauge verteilt, die Aetherphase abgetrennt, über Magnesiumsulfat getrocknet und eingedampft. Das als Oel erhaltene, rohe 1,5-Dimethyl-2-[3-(2,3-epoxy-propoxy)-phenyl]-pyrrol wird als solches weiterverwendet.

Beispiel 36

Analog den in den vorhergehenden Beispielen beschriebenen Arbeitsweisen können folgende Verbindungen oder deren N-Oxide oder Salze, insbesondere pharmazeutisch verwendbare, nicht-toxische Säure-additionssalze hergestellt werden :

1-[2-(3-Carbamoyl-4-hydroxy-phenoxy)-äthylamino]-3-[4-(1-methyl-3-trifluormethyl-1H-1,2,4-triazol-5-yl)-phenoxy]-2-propanol ;

1-[2-(3-Carbamoyl-4-hydroxy-phenoxy)-äthylamino]-3-[4-(1-methyl-1H-1,2,4-triazol-3-yl)-phenoxy]-2-propanol ;

1-[2-(3-Carbamoyl-4-hydroxy-phenoxy)-äthylamino]-3-[4-(1-methyl-5-methylsulfonyl-1H-1,2,4-triazol-3-yl)-phenoxy]-2-propanol ;

1-[2-(3-Carbamoyl-4-hydroxy-phenoxy)-äthylamino]-3-[4-(3-n-butyl-1-methyl-1H-1,2,4-triazol-5-yl)-phenoxy]-2-propanol ;

1-[2-(3-Carbamoyl-4-hydroxy-phenoxy)-äthylamino]-3-[4-(5-carbamoyl-1-methyl-1H-1,2,4-triazol-3-yl)-phenoxy]-2-propanol ;

1-[2-(3-Carbamoyl-4-hydroxy-phenoxy)-äthylamino]-3-[4-(5-trifluormethyl-1,3,4-oxadiazol-2-yl)-phenoxy]-2-propanol ;

1-[2-(3-Carbamoyl-4-hydroxy-phenoxy)-äthylamino]-3-[4-(4-methyl-5-methylsulfonyl-4H-1,2,4-triazol-3-yl)-phenoxy]-2-propanol ;

1-[2-(3-Carbamoyl-4-hydroxy-phenoxy)-äthylamino]-3-[4-(3-cyanpyridin-6-yl)-phenoxy]-2-propanol.

Beispiel 37

Tabletten enthaltend 20 mg an aktiver Substanz werden in folgender Zusammensetzung in üblicher Weise hergestellt :

Zusammensetzung :

| | |
|---|---|
| 1-[2-(3-Carbamoyl-4-hydroxy-phenoxy)-äthylamino]-3-[4-1-methyl-4-(trifluormethyl)-1H-imidazol-2-yl)-phenoxy]-2-propanol-monomethansulfonat | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung :

1-[2-(3-Carbamoyl-4-hydroxy-phenoxy)-äthylamino]-3-[4-[1-methyl-4-(trifluormethyl)-1H-imidazol-2-yl]-phenoxy]-2-propanol-monomethansulfonat wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restlichen Weizenstärke, Talk und Magnesiumstearat zugemischt und die Mischung zu Tabletten von 145 mg Gewicht mit Bruchkerbe verpresst.

Beispiel 38

Tabletten enthaltend 1 mg an aktiver Substanz werden in folgender Zusammensetzung in üblicher Weise hergestellt :

Zusammensetzung :

| | |
|---|---|
| 1-[2-(3-Carbamoyl-4-hydroxy-phenoxy)-äthylamino]-3-[4-[1-methyl-4-(trifluormethyl)-1H-imidazol-2-yl]-phenoxy]-2-propanol-monomethansulfonat | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung :

1-[2-(3-Carbamoyl-4-hydroxy-phenoxy)-äthylamino]-3-[4-[1-methyl-4-(trifluormethyl)-1H-imidazol-2-yl]-phenoxy]-2-propanol-monomethansulfonat wird mit einem Teil der Weizenstärke, mit Milchzucker und

kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restlichen Weizenstärke, Talk und Magnesiumstearat zugemischt und die Mischung zu Tabletten von 145 mg Gewicht mit Bruchkerbe verpresst.

Beispiel 39

Kapseln enthaltend 10 mg an aktiver Substanz werden wie folgt auf übliche Weise hergestellt :

Zusammensetzung :

| | |
|---|---|
| 1-[2-(3-Carbamoyl-4-hydroxy-phenoxy)-äthylamino]-3-[4-[1-methyl-4-(trifluormethyl)-1H-imidazol-2-yl]-phenoxy]-2-propanol-monomethansulfonat | 2 500 mg |
| Talkum | 200 mg |
| Kolloidale Kieselsäure | 50 mg |

Herstellung :

Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Beispiel 40

Eine sterile Lösung von 5,0 g 1-[2-(3-Carbamoyl-4-hydroxyphenoxy)-äthylamino]-3-[4-[1-methyl-4-(trifluormethyl)-1H-imidazol-2-yl]-phenoxy]-2-propanol-monomethansulfonat in 5 000 ml destilliertem Wasser wird in Ampullen zu 5 ml abgefüllt, die in 5 ml Lösung 5 mg Wirkstoff enthalten.

Beispiel 41

3,62 g 1-[2-(3-Carbamoyl-4-hydroxy-phenoxy)-äthylamino]-3-[4-[1-methyl-(4-trifluormethyl)-1H-imidazol-2-yl]-phenoxy]-2-propanol werden unter Zusatz von 100,0 ml 0,10-n. Salzsäure mit 18 000 ml destilliertem Wasser auf ein Volumen von 18 100 ml gelöst. Die sterilisierte Lösung wird in Ampullen à 5,0 ml abgefüllt, in denen 1 mg Wirkstoff enthalten sind.

Beispiel 42

Anstelle der in den Beispielen 37 bis 41 als aktive Substanz verwendeten Verbindung können auch folgende Verbindungen der Formel I, oder deren pharmazeutisch annehmbare nicht-toxischen Säureadditionssalze als Wirkstoffe in Tabletten, Dragées, Kapseln, Ampullenlösungen etc. verwendet werden : 1-[2-(3-Carbamoyl-4-hydroxy-phenoxy)-äthylamino]-3-[4-[5-[4-(trifluormethyl)-1H-imidazol-2-yl]-2-furyl]-phenoxy]-2-propanol, 1-[2-(3-Carbamoyl-4-hydroxy-phenoxy)-äthylamino]-3-[4-[4-(trifluormethyl)-1H-imidazol-2-yl]-phenoxy]-2-propanol, 1-[2-(3-Carbamoyl-4-hydroxy-phenoxy)-äthylamino]-3-[4-(4-methyl-6-(trifluormethyl)-pyrimidin-2-yl]-phenoxy]-2-propanol, 1-[2-(3-Carbamoyl-4-hydroxy-phenoxy)-äthylamino]-3-[4-(4,6-dimethyl-pyrimidin-2-yl)-phenoxy]-2-propanol, 1-[2-(3-Carbamoyl-4-hydroxy-phenoxy)-äthylamino]-3-[4-(3,5-dicarbäthoxy-2,6-dimethyl-pyridin-4-yl)-phenoxy]-2-propanol, 1-[2-(3-Carbamoyl-4-hydroxy-phenoxy)-äthylamino]-3-(4-biphenyloxy)-2-propanol, 1-[2-(3-Carbamoyl-4-hydroxy-phenoxy)-äthylamino]-3-[4-(1-methyl-1H-imidazol-2-yl]-phenoxy-2-propanol, 1-[2-[4-Carbamoyl-3-hydroxy-phenoxy)-äthylamino]-3-[4-[1-methyl-4-(trifluormethyl)-1H-imidazol-2-yl]-phenoxy]-2-propanol, 1-[2-(3-Carbamoyl-4-hydroxy-phenoxy)-äthylamino]-3-[4-(4-acetyl-1,5-dimethyl-1H-imidazol-2-yl)-phenoxy]-2-propanol, 1-[2-(3-Carbamoyl-4-hydroxy-phenoxy)-äthylamino]-3-[4-(1-methyl-3-methylthio-1H-1,2,4-triazol-5-yl)-phenoxy]-2-propanol, 1-[2-(3-Carbamoyl-4-hydroxy-phenoxy)-äthylamino]-3-[2-(indol-2-yl)-phenoxy]-2-propanol, 1-[2-(3-Carbamoyl-4-hydroxy-phenoxy)-äthylamino]-3-[4-(1,4,5-trimethyl-1H-imidazol-2-yl)-phenoxy]-2-propanol, 1-[2-(3-Carbamoyl-4-hydroxy-phenoxy)-äthylamino]-3-[4-(4-methyl-5-trifluormethyl-4H-1,2,4-triazol-3-yl)-phenoxy]-2-propanol, 1-[2-(3-Carbamoyl-4-hydroxy-phenoxy)-äthylamino]-3-[4-(1-methyl-5-(trifluormethyl)-1H-1,2,4-triazol-3-yl)-phenoxy]-2-propanol, 1-[2-(3-Carbamoyl-4-hydroxy-phenoxy)-äthylamino]-3-[4-(1-methyl-3-methylsulfonyl-1H-1,2,4-triazol-5-yl)-phenoxy]-2-propanol, 1-[2-(3-Carbamoyl-4-hydroxyphenoxy)-äthylamino]-3-[4-(2-methyl-2H-tetrazol-5-yl)-phenoxy]-2-propanol, 1-[2-(3-Carbamoyl-4-hydroxy-phenoxy]-äthylamino]-3-[4-[1-methyl-5-(trifluormethyl)-1H-imidazol-2-yl]-phenoxy]-2-propanol, 1-[2-(3-Carbamoyl-4-hydroxy-phenoxy)-äthylamino]-3-[4-[1-isopropyl-4-(trifluormethyl)-1H-imidazol-2-yl]-phenoxy]-2-propanol, 1-[2-(3-Carbamoyl-4-hydroxy-phenoxy)-äthylamino]-3-[4-[1-(2-hydroxyäthyl)-4-(trifluormethyl)-1H-imidazol-2-yl]-phenoxy]-2-propanol, 1-[2-(3-Carbamoyl-4-hydroxy-phenoxy)-äthylamino]-3-[4-[1,4- bzw. 1,5-dimethyl-1H-imidazol-2-yl]-phenoxy]-2-propanol als Isomerengemisch

ca. 1 : 1, 1-[2-(3-Carbamoyl-4-hydroxyphenoxy)-äthylamino]-3-[4-[4-(trifluormethyl)-thiazol-2-yl]-phenoxy]-2-propanol, 1-[2-(3-Carbamoyl-4-hydroxy-phenoxy)-äthylamino]-3-[4-(thiazol-2-yl)-phenoxy]-2-propanol, 1-[2-(3-Carbamoyl-4-hydroxy-phenoxy)-1-methyl-äthylamino]-3-[4-(1-methyl-4-(trifluormethyl)-1H-imidazol-2-yl)-phenoxy]-2-propanol, 1-[4-(1,6-Dihydro-1-methyl-6-oxo-2-pyridinyloxy)-phenoxy]-3-[2-(3-carbamoyl-4-hydroxyphenoxy)-äthylamino]-2-propanol, 1-[4-(3,4-Dihydro-3,6-dimethyl-4-oxo-2-pyrimidinyloxy))-phenoxy]-3-[2-(3-carbamoyl-4-hydroxy-phenoxy)-äthylamino]-2-propanol, 1-[2-(3-Carbamoyl-4-hydroxy-phenoxy)-äthylamino]-3-[4-(1,4-dimethyl-1H-pyrimidin-6-on-2-yl)-phenoxy]-2-propanol, 1-[2-(3-Carbamoyl-4-hydroxy-phenoxy)-äthylamino]-3-[4-(4-methyl-5-methyl-thio-4H-1,2,4-triazol-3-yl)-phenoxy]-2-propanol, oder das 1-[2-(3-Carbamoyl-4-hydroxy-phenoxy)-äthylamino]-3-[3-(1,5-dimethylpyrrol-2-yl)-phenoxy]-2-propanol.

**Patentansprüche** (für die Vertragsstaaten : BE, CH, DE, FR, GB, IT, LI, LU, NL, SE)

1. Substituierte Phenyläther der Formel

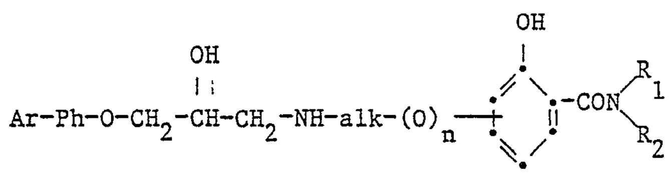

(I)

worin Ar einen gegebenenfalls substituierten Phenylrest, oder einen über ein Ringkohlenstoffatom an den Phenylenrest Ph gebundenen, ein Sauerstoff-, Schwefel- oder Stickstoffatom und gegebenenfalls 1 bis 3 zusätzliche Stickstoffatome als Ringglieder enthaltenden monocyclischen Heteroarylrest mit 5 bis 6 Ringgliedern bedeutet, der mindestens partiell hydriert und in diesem Fall durch Oxo substituiert sein kann, wobei Substituenten gegebenenfalls substituiertes Niederalkyl, oder Niederalkenyl, gegebenenfalls veräthertes oder verestertes Hydroxy, Niederalkylthio, Niederalkylsulfonyl, oder Halogen, Acyl, gegebenenfalls verestertes Carboxy, Carbamoyl, Cyan, Nitro, gegebenenfalls substituiertes, wie acyliertes Amino, gegebenenfalls durch Niederalkyl, Niederalkoxy, Halogen, Halogenniederalkyl, Nitro oder Cyan substituiertes Phenyl oder Benzoyl, und/oder gegebenenfalls, wie angegeben, substituiertes monocyclisches, ein Sauerstoff- oder Stickstoffatom und gegebenenfalls 1 bis 3 weitere Stickstoffatome enthaltendes Heteroaryl bedeuten, Ph gegebenenfalls durch Niederalkyl, Niederalkoxy, Halogen oder Cyan substituiertes 1,4-Phenylen darstellt, n die Zahl 0 oder 1, und alk einen Alkylenrest mit 2 bis 4 Kohlenstoffatomen bedeutet, wobei das Stickstoffatom und das Sauerstoffatom, oder falls n für 0 steht, der aromatische Rest durch 2 bis 3 Kohlenstoffatome in der unverzweigten Kette voneinander getrennt sind, $R_1$ und $R_2$ für Wasserstoff oder Niederalkyl stehen oder zusammen mit dem Stickstoff der Amidgruppe Pyrrolidino oder Morpholino bilden.

2. Verbindungen der Formel I, worin Ar einen gegebenenfalls substituierten Phenylrest, oder einen über ein Ringkohlenstoffatom an den Phenylenrest Ph gebundenen, ein Sauerstoff-, Schwefel- oder Stickstoffatom und gegebenenfalls 1 bis 2 zusätzliche Stickstoffatome als Ringglieder enthaltenden monocyclischen Heteroarylrest mit 5 bis 6 Ringgliedern bedeutet, oder einen mindestens partiell hydrierten Oxo-Heteroarylrest mit 1 bis 2 Stickstoffatomen als Ringglieder darstellt, wobei Substituenten gegebenenfalls substituiertes Niederalkyl, gegebenenfalls veräthertes oder verestertes Hydroxy, Niederalkylthio oder Niederalkylsulfonyl, oder Halogen, Acyl, gegebenenfalls verestertes Carboxy, Carbamoyl, Cyan, gegebenenfalls substituiertes wie acyliertes Amino, gegebenenfalls durch Niederalkyl, Niederalkoxy, Halogen, Halogenniederalkyl oder Cyan substituiertes Phenyl oder Benzoyl und/oder gegebenenfalls, wie angegeben, substituiertes monocyclisches, ein Sauerstoff- oder Stickstoffatom und gegebenenfalls 1 bis 2 weitere Stickstoffatome enthaltendes Heteroaryl bedeuten, Ph gegebenenfalls durch Niederalkyl, Niederalkoxy, Halogen oder Cyan substituiertes 1,4-Phenylen darstellt, n die Zahl 0 oder 1, und alk einen Alkylenrest mit 2 bis 3 Kohlenstoffatomen bedeutet, wobei das Stickstoffatom und das Sauerstoffatom, oder, falls n für 0 steht, der aromatische Rest durch 2 bis 3 Kohlenstoffatome in der unverzweigten Kette voneinander getrennt sind, $R_1$ und $R_2$ für Wasserstoff oder Niederalkyl stehen oder zusammen mit dem Stickstoffatom der Amidgruppe Morpholino bilden.

3. Verbindungen der Formel I, worin Ar einen gegebenenfalls substituierten Phenylrest, oder einen über ein Ringkohlenstoffatom an den Phenylenrest Ph gebundenen ein Sauerstoff-, Schwefel- oder ein Stickstoffatom und gegebenenfalls 1 bis 2 zusätzliche Stickstoffatome als Ringglieder enthaltenden Heteroarylrest mit 5 bis 6 Ringgliedern, z. B. Furyl, Oxazolyl, Imidazolyl, Triazolyl, Thiazolyl, Indolyl oder Pyridyl, bedeutet, oder einen Dihydro-oxo-Heteroarylrest mit 1-2 Stickstoffatomen als Ringglieder, z. B. Dihydro-oxo-2-pyridinyl oder Dihydro-4-oxo-2-pyrimidinyl darstellt, wobei Substituenten Niederalkyl, z. B. Methyl, Halogenniederalkyl, z. B. Trifluormethyl, Hydroxy, Niederalkoxy, z. B. Methoxy, Halogen, Niederalkanoyl, z. B. Acetyl, Carboxy, Niederalkoxycarbonyl, z. B. Methoxycarbonyl, Cyan, Carbamoyl, Amino, Niederalkanoylamino, z. B. Acetylamino, Niederalkoxycarbonylamino, z. B. Methoxycarbonylamino, und/oder gegebenenfalls, wie angegeben, substituiertes z. B. Imidazolyl, oder monocyclisches, ein Sauerstoff-

oder Stickstoffatom und gegebenenfalls ein weiteres Stickstoffatom enthaltendes Heteroaryl, z. B. Furyl oder Oxazolyl bedeuten, Ph gegebenenfalls durch Niederalkyl, wie Methyl, oder Halogen, wie Chlor, substituiertes 1,4-Phenylen darstellt, n die Zahl 0 oder 1, alk einen Alkylenrest mit 2 bis 3 Kohlenstoffatomen bedeutet, wobei das Stickstoffatom und Sauerstoffatom, oder, falls n für 0 steht, der aromatische Rest, durch 2 bis 3 Kohlenstoffatome in der unverzweigten Kette voneinander getrennt sind, $R_1$ und $R_2$ jeweils für Wasserstoff stehen.

4. 1-[2-(3-Carbamoyl-4-hydroxy-phenoxy)-äthylamino]-3-[4-[1-methyl-4-(trifluormethyl)-1H-imidazol-2-yl]-phenoxy]-2-propanol.

5. 1-[2-(3-Carbamoyl-4-hydroxy-phenoxy)-äthylamino]-3-[4-[1-methyl-1H-imidazol-2-yl]-phenoxy]-2-propanol.

6. 1-[4-(1,6-Dihydro-1-methyl-6-oxo-2-pyridinyloxy)-phenoxy]-3-[2-(3-carbamoyl-4-hydroxy-phenoxy)-äthylamino]-2-propanol.

7. 1-[2-(3-Carbamoyl-4-hydroxy-phenoxy)-äthylamino]-3-[4-(1-methyl-3-methylthio-1H-1,2,4-triazol-5-yl)-phenoxy]-2-propanol.

8. 1-[2-(3-Carbamoyl-4-hydroxy-phenoxy)-äthylamino]-3-[4-(1-methyl-3-methylsulfonyl-1H-1,2,4-triazol-5-yl)-phenoxy]-2-propanol.

9. 1-[2-(3-Carbamoyl-4-hydroxy-phenoxy)-äthylamino]-3-[4-(1,4,5-trimethyl-1H-imidazol-2-yl)-phenoxy]-2-propanol.

10. N-Oxide von Verbindungen der Ansprüche 1 bis 9.

11. Salze von Verbindungen der Ansprüche 1 bis 10.

12. Pharmazeutisch verwendbare, nicht-toxische Säureadditionssalze von Verbindungen der Ansprüche 1 bis 10.

13. Pharmazeutische Präparate enthaltend eine Verbindung der Ansprüche 1 bis 10 und 12.

14. Verbindungen der Formel I oder deren Salze zur Anwendung in einem Verfahren zur therapeutischen Behandlung des menschlichen oder tierischen Körpers.

15. Beta-Rezeptoren-blockierende Verbindungen der Formel I oder deren Salze als Mittel zur Bekämpfung von Angina pectoris, hypertrophen Kardiomyopathien und Herzrhythmusstörungen, des Bluthochdrucks, sowie des erhöhten intraokularen Drucks und der Thyreotoxikose.

16. Beta-Rezeptoren-stimulierende Verbindungen der Formel I oder deren Salze als Mittel zur Bekämpfung der Herzmuskelinsuffizienz.

17. Verwendung von Verbindungen der Formel I zur Herstellung von pharmazeutischen Präparaten.

18. Verfahren zur Herstellung von substituierten Phenyläthern der Formel

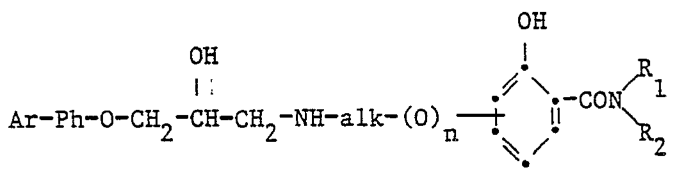

(I)

worin Ar einen gegebenenfalls substituierten Phenylrest, oder einen über ein Ringkohlenstoffatom an den Phenylenrest Ph gebundenen, ein Sauerstoff-, Schwefel- oder Stickstoffatom und gegebenenfalls 1 bis 3 zusätzliche Stickstoffatome als Ringglieder enthaltenden monocyclischen Heteroarylrest mit 5 bis 6 Ringgliedern bedeutet, der mindestens partiell hydriert und in diesem Fall durch Oxo substituiert sein kann, wobei Substituenten gegebenenfalls substituiertes Niederalkyl, oder Niederalkenyl, gegebenenfalls veräthertes oder verestertes Hydroxy, Niederalkylthio, Niederalkylsulfonyl, oder Halogen, Acyl, gegebenenfalls verestertes Carboxy, Carbamoyl, Cyan, Nitro, gegebenenfalls substituiertes, wie acyliertes Amino, gegebenenfalls durch Niederalkyl, Niederalkoxy, Halogen, Halogen-niederalkyl, Nitro oder Cyan substituiertes Phenyl oder Benzoyl, und/oder gegebenenfalls, wie angegeben, substituiertes monocyclisches, ein Sauerstoff- oder Stickstoffatom und gegebenenfalls 1 bis 3 weitere Stickstoffatome enthaltendes Heteroaryl bedeuten, Ph gegebenenfalls durch Niederalkyl, Niederalkoxy, Halogen oder Cyan substituiertes 1,4-Phenylen darstellt, n die Zahl 0 oder 1, und alk einen Alkylenrest mit 2 bis 4 Kohlenstoffatomen bedeutet, wobei das Stickstoffatom und das Sauerstoffatom, oder falls n für 0 steht, der aromatische Rest durch 2 bis 3 Kohlenstoffatome in der unverzweigten Kette voneinander getrennt sind, $R_1$ und $R_2$ für Wasserstoff oder Niederalkyl stehen oder zusammen mit dem Stickstoff der Amidgruppe Pyrrolidino oder Morpholino bilden, oder deren Salze, dadurch gekennzeichnet, dass man

a) in einer Verbindung der Formel

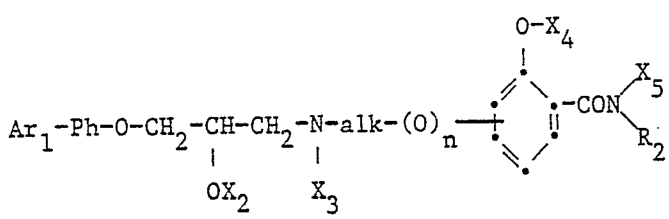

(II)

worin $Ar_1$ den Rest Ar oder einen diesem entsprechenden, mindestens eine funktionelle Gruppierung in geschützter Form enthaltenden Rest darstellt, $X_2$, $X_3$ und $X_4$ jeweils Wasserstoff oder einen durch Wasserstoff ersetzbaren Substituenten bedeuten, $X_5$ für $R_1$ oder einen durch Wasserstoff ersetzbaren Substituenten steht, oder $X_2$ und $X_3$ und/oder $X_4$ und $X_5$ zusammen einen zweiwertigen, durch zwei Wasserstoffatome ersetzbaren Rest bedeuten, und/oder im Rest $Ar_1$ gegebenenfalls vorhandene funktionelle Gruppen gegebenenfalls in geschützter Form vorliegen mit der Massgabe, dass mindestens einer der Reste $X_2$, $X_3$, $X_4$ oder $X_5$ von Wasserstoff verschieden ist, oder mindestens $Ar_1$ einen Rest Ar bedeutet, der mindestens eine funktionelle Gruppierung in geschützter Form enthält, oder mindestens $X_2$ und $X_3$ zusammen oder $X_4$ und $X_5$ zusammen einen zweiwertigen durch zwei Wasserstoffatome ersetzbaren Rest darstellen, oder in einem Salz davon, die von Wasserstoff verschiedenen Gruppen $X_2$, $X_3$, $X_4$ oder $X_5$ bzw. $X_2$ und $X_3$ zusammen oder $X_4$ und $X_5$ zusammen durch Wasserstoffatome ersetzt, und/oder in einem Rest $Ar_1$ die an eine oder mehrere funktionelle Gruppen gebundenen Schutzgruppen abspaltet und durch Wasserstoff ersetzt, oder

b) eine Verbindung der Formel

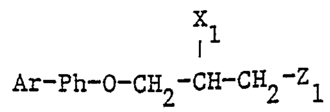

(III)

mit einer Verbindung der Formel

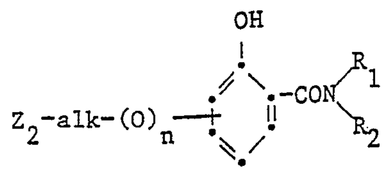

(IV)

oder einem Salz davon, worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, und Ar, Ph, n, alk, $R_1$ und $R_2$ obige Bedeutung haben, umsetzt oder

c) in einer Verbindung der Formel

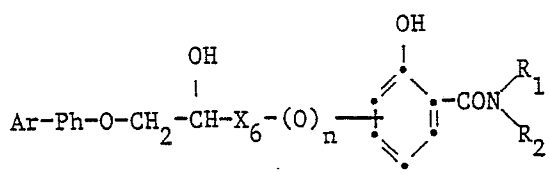

(V)

worin $X_6$ eine reduzierbare Gruppe der Formel

—CH=N—alk— (Va) oder —$CH_2$—N=$alk_1$— (Vb)

darstellt, wobei $alk_1$ für einen dem Rest alk entsprechenden Alkylylidenrest steht, und n für die Zahl 0 oder 1 steht, $X_6$ zur Gruppe der Formel

—$CH_2$—NH—alk— (Vc)

reduziert, gegebenenfalls vorhandene an der Umsetzung nicht teilnehmende, an funktionellen Gruppen stehende Schutzgruppen gleichzeitig oder nachfolgend abspaltet und durch Wasserstoff ersetzt, oder

d) eine Verbindung der Formel

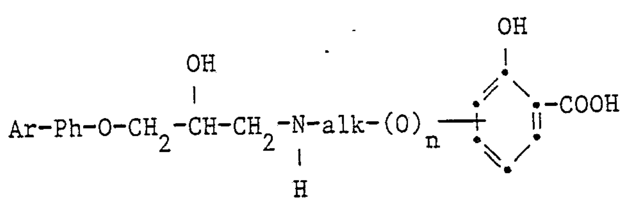

(VI)

worin an der Umsetzung nicht teilnehmende funktionelle Gruppen gegebenenfalls durch, mittels

Aminolyse oder Ammonolyse abspaltbare und durch Wasserstoff ersetzbare Schutzgruppen geschützt sind, mit einer Verbindung der Formel

$$HNR_1R_2 \qquad (VIa)$$

umsetzt, und gleichzeitig oder nachfolgend gegebenenfalls vorhandene Schutzgruppen abspaltet und durch Wasserstoff ersetzt, oder,

e) in einer Verbindung der Formel

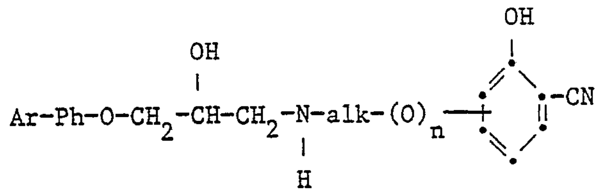

(VII)

worin an der Umsetzung nicht teilnehmende funktionelle Gruppen gegebenenfalls durch mittels Hydrolyse abspaltbare und durch Wasserstoff ersetzbare Schutzgruppen geschützt sind, die unter den Bedingungen des Verfahrens abgespalten und durch Wasserstoff ersetzt werden, die Gruppe —CN mittels Hydrolyse in die Gruppe $—CONH_2$ umwandelt, und gleichzeitig gegebenenfalls vorhandene Schutzgruppen abspaltet und durch Wasserstoff ersetzt, oder

f) zur Herstellung von Verbindungen der Formel I, worin Ar einen mono- oder bicyclischen Heteroarylrest darstellt, in einer Verbindung der Formel

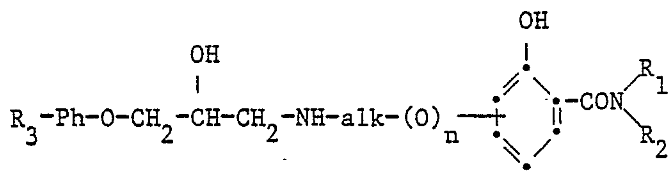

(VIII)

worin Hydroxygruppen und/oder die Aminogruppe durch Schutzgruppen geschützt sind, und $R_3$ einen zur Bildung eines mono- oder bicyclischen Heteroarylrestes Ar befähigten Substituenten darstellt, die mono- oder bicyclische Heteroarylgruppe Ar bildet, gegebenenfalls vorhandene Schutzgruppen gleichzeitig oder nachfolgend abspaltet und durch Wasserstoff ersetzt, und, wenn erwünscht, eine so erhältliche Verbindung in eine andere Verbindung der Formel I umwandelt und/oder, wenn erwünscht, eine erhaltene freie Verbindung in ein Salz oder ein erhaltenes Salz in eine freie Verbindung überführt, und/oder, wenn erwünscht, ein erhaltenes Isomerengemisch in die Isomeren, oder ein erhaltenes Racemat in die Antipoden auftrennt.

19. Die nach dem Verfahren gemäss Anspruch 18 erhältlichen Verbindungen.

**Patentansprüche** (für den Vertragsstaat AT)

1. Verfahren zur Herstellung von substituierten Phenyläthern der Formel

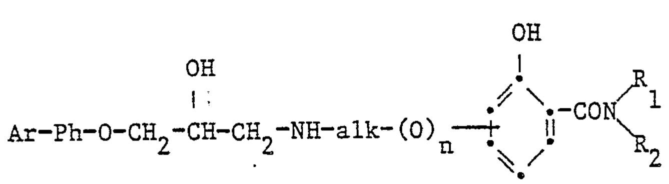

(I)

worin Ar einen gegebenenfalls substituierten Phenylrest, oder einen über ein Ringkohlenstoffatom an den Phenylenrest Ph gebundenen, ein Sauerstoff-, Schwefel- oder Stickstoffatom und gegebenenfalls 1 bis 3 zusätzliche Stickstoffatome als Ringglieder enthaltenden monocyclischen Heteroarylrest mit 5 bis 6 Ringgliedern bedeutet, der mindestens partiell hydriert und in diesem Fall durch Oxo substituiert sein kann, wobei Substituenten gegebenenfalls substituiertes Niederalkyl, oder Niederalkenyl, gegebenenfalls veräthertes oder verestertes Hydroxy, Niederalkylthio, Niederalkylsulfonyl, oder Halogen, Acyl, gegebenenfalls verestertes Carboxy, Carbamoyl, Cyan, Nitro, gegebenenfalls substituiertes, wie acyliertes Amino, gegebenenfalls durch Niederalkyl, Niederalkoxy, Halogen, Halogenniederalkyl, Nitro oder Cyan substituiertes Phenyl oder Benzoyl, und/oder gegebenenfalls, wie angegeben, substituiertes monocyclisches, ein Sauerstoff- oder Stickstoffatom und gegebenenfalls 1 bis 3 weitere Stickstoffatome enthaltendes Heteroaryl bedeuten, Ph gegebenenfalls durch Niederalkyl, Niederalkoxy, Halogen oder Cyan

substituiertes 1,4-Phenylen darstellt, n die Zahl 0 oder 1, und alk einen Alkylenrest mit 2 bis 4 Kohlenstoffatomen bedeutet, wobei das Stickstoffatom und das Sauerstoffatom, oder falls n für 0 steht, der aromatische Rest durch 2 bis 3 Kohlenstoffatome in der unverzweigten Kette voneinander getrennt sind, $R_1$ und $R_2$ für Wasserstoff oder Niederalkyl stehen oder zusammen mit dem Stickstoff der Amidgruppe Pyrrolidino oder Morpholino bilden, oder deren, Salze, dadurch gekennzeichnet, dass man

a) in einer Verbindung der Formel

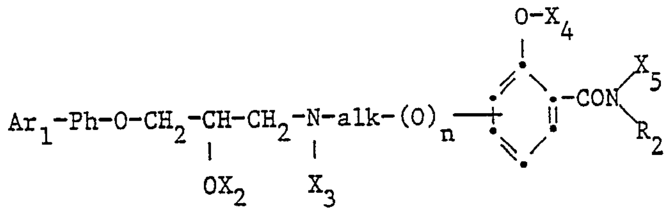

(II)

worin Ar den Rest Ar oder einen diesem entsprechenden, mindestens eine funktionelle Gruppierung in geschützter Form enthaltenden Rest darstellt, $X_2$, $X_3$ und $X_4$ jeweils Wasserstoff oder einen durch Wasserstoff ersetzbaren Substituenten bedeuten, $X_5$ für $R_1$ oder einen durch Wasserstoff ersetzbaren Substituenten steht, oder $X_2$ und $X_3$ und/oder $X_4$ und $X_5$ zusammen einen zweiwertigen, durch zwei Wasserstoffatome ersetzbaren Rest bedeuten, und/oder im Rest $Ar_1$ gegebenenfalls vorhandene funktionelle Gruppen gegebenenfalls in geschützter Form vorliegen mit der Massgabe, dass mindestens einer der Reste $X_2$, $X_3$, $X_4$ oder $X_5$ von Wasserstoff verschieden ist, oder mindestens $Ar_1$ einen Rest Ar bedeutet, der mindestens eine funktionelle Gruppierung in geschützter Form enthält, oder mindestens $X_2$ und $X_3$ zusammen oder $X_4$ und $X_5$ zusammen einen zweiwertigen durch zwei Wasserstoffatome ersetzbaren Rest darstellen, oder in einem Salz davon, die von Wasserstoff verschiedenen Gruppen $X_2$, $X_3$, $X_4$ oder $X_5$ bzw. $X_2$ und $X_3$ zusammen oder $X_4$ und $X_5$ zusammen durch Wasserstoffatome ersetzt, und/oder in einem Rest $Ar_1$ die an eine oder mehrere funktionelle Gruppen gebundenen Schutzgruppen abspaltet und durch Wasserstoff ersetzt, oder

b) eine Verbindung der Formel

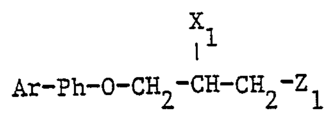

(III)

mit einer Verbindung der Formel

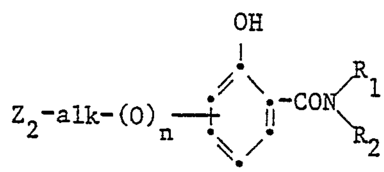

(IV)

oder einem Salz davon, worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, und Ar, Ph, n, alk, $R_1$ und $R_2$ obige Bedeutung haben, umsetzt oder

c) in einer Verbindung der Formel

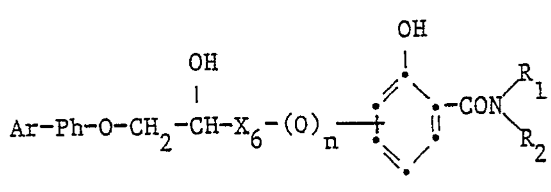

(V)

worin $X_6$ eine reduzierbare Gruppe der Formel

$$—CH{=}N—alk— \quad (Va) \quad \text{oder} \quad —CH_2—N{=}alk_1— \quad (Vb)$$

darstellt, wobei $alk_1$ für einen dem Rest alk entsprechenden Alkylylidenrest steht, und n für die Zahl 0 oder 1 steht, $X_6$ zur Gruppe der Formel

$$—CH_2—NH—alk— \quad (Vc)$$

reduziert, gegebenenfalls vorhandene an der Umsetzung nicht teilnehmende, an funktionellen Gruppen stehende Schutzgruppen gleichzeitig oder nachfolgend abspaltet und durch Wasserstoff ersetzt, oder

d) eine Verbindung der Formel

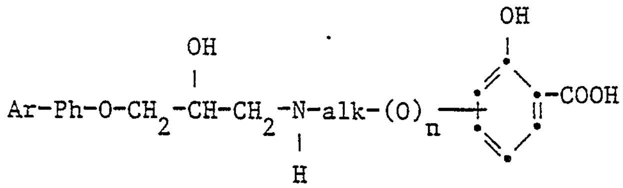

(VI)

worin an der Umsetzung nicht teilnehmende funktionelle Gruppen gegebenenfalls durch, mittels Aminolyse oder Ammonolyse abspaltbare und durch Wasserstoff ersetzbare Schutzgruppen geschützt sind, mit einer Verbindung der Formel

$$HNR_1R_2 \qquad \text{(VIa)}$$

umsetzt, und gleichzeitig oder nachfolgend gegebenenfalls vorhandene Schutzgruppen abspaltet und durch Wasserstoff ersetzt, oder,

e) in einer Verbindung der Formel

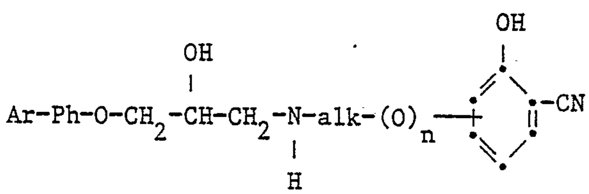

(VII)

worin an der Umsetzung nicht teilnehmende funktionelle Gruppen gegebenenfalls durch mittels Hydrolyse abspaltbare und durch Wasserstoff ersetzbare Schutzgruppen geschützt sind, die unter den Bedingungen des Verfahrens abgespalten und durch Wasserstoff ersetzt werden, die Gruppe —CN mittels Hydrolyse in die Gruppe $—CONH_2$ umwandelt, und gleichzeitig gegebenenfalls vorhandene Schutzgruppen abspaltet und durch Wasserstoff ersetzt, oder

f) zur Herstellung von Verbindungen der Formel I, worin Ar einen mono- oder bicyclischen Heteroarylrest darstellt, in einer Verbindung der Formel

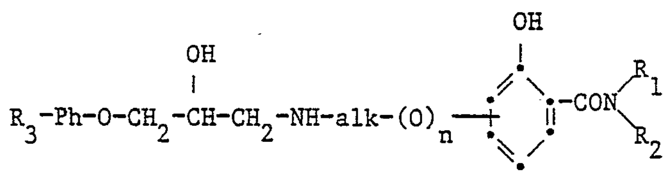

(VIII)

worin Hydroxygruppen und/oder die Aminogruppe durch Schutzgruppen geschützt sind, und $R_3$ einen zur Bildung eines mono- oder bicyclischen Heteroarylrestes Ar befähigten Substituenten darstellt, die mono- oder bicyclische Heteroarylgruppe Ar bildet, gegebenenfalls vorhandene Schutzgruppen gleichzeitig oder nachfolgend abspaltet und durch Wasserstoff ersetzt, und, wenn erwünscht, eine so erhältliche Verbindung in eine andere Verbindung der Formel I umwandelt und/oder, wenn erwünscht, eine erhaltene freie Verbindung in ein Salz oder ein erhaltenes Salz in eine freie Verbindung überführt, und/oder, wenn erwünscht, ein erhaltenes Isomerengemisch in die Isomeren, oder ein erhaltenes Racemat in die Antipoden auftrennt.

2. Verfahren nach Anspruch 1, worin Ar einen gegebenenfalls substituierten Phenylrest, oder einen über ein Ringkohlenstoffatom an den Phenylenrest Ph gebundenen, ein Sauerstoff-, Schwefel- oder Stickstoffatom und gegebenenfalls 1 bis 2 zusätzliche Stickstoffatome als Ringglieder enthaltenden monocyclischen Heteroarylrest mit 5 bis 6 Ringgliedern bedeutet, oder einen mindestens partiell hydrierten Oxo-Heteroarylrest mit 1 bis 2 Stickstoffatomen als Ringglieder darstellt, wobei Substituenten gegebenenfalls substituiertes Niederalkyl, gegebenenfalls veräthertes oder verestertes Hydroxy, Niederalkylthio oder Niederalkylsulfonyl, oder Halogen, Acyl, gegebenenfalls verestertes Carboxy, Carbamoyl, Cyan, gegebenenfalls substituiertes wie acyliertes Amino, gegebenenfalls durch Niederalkyl, Niederalkoxy, Halogen, Halogenniederalkyl oder Cyan substituiertes Phenyl oder Benzoyl und/oder gegebenenfalls, wie angegeben, substituiertes monocyclisches, ein Sauerstoff- oder Stickstoffatom und gegebenenfalls 1 bis 2 weitere Stickstoffatome enthaltendes Heteroaryl bedeuten, Ph gegebenenfalls durch Niederalkyl,

Niederalkoxy, Halogen oder Cyan substituiertes 1,4-Phenylen darstellt, n die Zahl 0 oder 1, und alk einen Alkylenrest mit 2 bis 3 Kohlenstoffatomen bedeutet, wobei das Stickstoffatom und das Sauerstoffatom, oder, falls n für 0 steht, der aromatische Rest durch 2 bis 3 Kohlenstoffatome in der unverzweigten Kette voneinander getrennt sind, $R_1$ und $R_2$ für Wasserstoff oder Niederalkyl stehen oder zusammen mit dem Stickstoffatom der Amidgruppe Morpholino bilden, dadurch gekennzeichnet, dass man von entsprechenden Ausgangsstoffen ausgeht.

3. Verfahren nach Anspruch 1, worin Ar einen gegebenenfalls substituierten Phenylrest, oder einen über ein Ringkohlenstoffatom an den Phenylenrest Ph gebundenen ein Sauerstoff-, Schwefel- oder ein Stickstoffatom und gegebenenfalls 1 bis 2 zusätzliche Stickstoffatome als Ringglieder enthaltenden Heteroarylrest mit 5 bis 6 Ringgliedern, z. B. Furyl, Oxazolyl, Imidazolyl, Triazolyl, Thiazolyl, Indolyl oder Pyridyl, bedeutet, oder einen Dihydro-oxo-Heteroarylrest mit 1-2 Stickstoffatomen als Ringglieder, z. B. Dihydro-oxo-2-pyridinyl oder Dihydro-4-oxo-2-pyrimidinyl darstellt, wobei Substituenten Niederalkyl, z. B. Methyl, Halogenniederalkyl, z. B. Trifluormethyl, Hydroxy, Niederalkoxy, z. B. Methoxy, Halogen, Niederalkanoyl, z. B. Acetyl, Carboxy, Niederalkoxycarbonyl, z. B. Methoxycarbonyl, Cyan, Carbamoyl, Amino, Niederalkanoylamino, z. B. Acetylamino, Niederalkoxycarbonylamino, z. B. Methoxycarbonylamino, und/oder gegebenenfalls, wie angegeben, substituiertes z. B. Imidazolyl, oder monocyclisches, ein Sauerstoff- oder Stickstoffatom und gegebenenfalls ein weiteres Stickstoffatom enthaltendes Heteroaryl, z. B. Furyl oder Oxazolyl bedeuten, Ph gegebenenfalls durch Niederalkyl, wie Methyl, oder Halogen, wie Chlor, substituiertes 1,4-Phenylen darstellt, n die Zahl 0 oder 1, alk einen Alkylenrest mit 2 bis 3 Kohlenstoffatomen bedeutet, wobei das Stickstoffatom und Sauerstoffatom, oder, falls n für 0 steht, der aromatische Rest, durch 2 bis 3 Kohlenstoffatome in der unverzweigten Kette voneinander getrennt sind, $R_1$ und $R_2$ jeweils für Wasserstoff stehen, dadurch gekennzeichnet, dass man von entsprechenden Ausgangsstoffen ausgeht.

4. Verfahren zur Herstellung von 1-[2-(3-Carbamoyl-4-hydroxyphenoxy)-äthylamino]-3-[4-[1-methyl-4-(trifluormethyl)-1H-imidazol-2-yl]-phenoxy]-2-propanol nach Anspruch 1, dadurch gekennzeichnet, dass man 1-[N-Benzyl-2-(3-carbamoyl-4-hydroxy-phenoxy)-äthylamino]-3-[4-[1-methyl-4-(trifluormethyl)-1H-imidazol-2-yl]-phenoxy]-2-propanol als Ausgangsstoff verwendet.

5. Verfahren zur Herstellung von 1-[2-(3-Carbamoyl-4-hydroxy-phenoxy)-äthylamino]-3-[4-[1-methyl-4-(trifluormethyl)-1H-imidazol-2-yl]-phenoxy]-2-propanol nach Anspruch 1, dadurch gekennzeichnet, dass man 1-Amino-3-[4-(1-methyl-4-(trifluormethyl)-1H-imidazol-2-yl)-phenoxy]-2-propanol und 5-(2-Bromäthoxy)-salicylamid als Ausgangsstoffe verwendet.

6. Verfahren zur Herstellung von 1-[2-(3-Carbamoyl-4-hydroxy-phenoxy)-äthylamino]-3-[4-[1-methyl-4-(trifluormethyl)-1H-imidazol-2-yl]-phenoxy]-2-propanol nach Anspruch 1, dadurch gekennzeichnet, dass man die aus 1-Amino-3-[4-(1-methyl-4-(trifluormethyl)-1H-imidazol-2-yl)-phenoxy]-2-propanol und 2,3-Dihydro-2,2-dimethyl-7-(2-oxo-äthoxy)-4H-1,3-benzoxazin-4-on gebildete Schiff'sche Base als Ausgangsstoff verwendet.

7. Verfahren zur Herstellung von 1-[2-(3-Carbamoyl-4-hydroxy-phenoxy)-äthylamino]-3-[4-[1-methyl-4-(trifluormethyl)-1H-imidazol-2-yl]-phenoxy]-2-propanol nach Anspruch 1, dadurch gekennzeichnet, dass man 1-[2-(3-Carbomethoxy-4-hydroxy-phenoxy)-äthylamino]-3-[4-[1-methyl-4-(trifluormethyl)-1H-imidazol-2-yl]-phenoxy]-2-propanol als Ausgangsstoff verwendet.

8. Verfahren zur Herstellung von 1-[2-(3-Carbamoyl-4-hydroxy-phenoxy)-äthylamino]-3-[4-(1-methyl-1H-imidazol-2-yl)-phenoxy]-2-propanol nach Anspruch 1, dadurch gekennzeichnet, dass man 1-[N-Benzyl-2-(3-carbamoyl-4-benzyloxy-phenoxy)-äthylamino]-3-[4-[1-methyl-1H-imidazol-2-yl]-phenoxy]-2-propanol als Ausgangsstoff verwendet.

9. Verfahren zur Herstellung von 1-[4-(1,6-Dihydro-1-methyl-6-oxo-2-pyridinyloxy)-phenoxy]-3-[2-(3-carbamoyl-4-hydroxy-phenoxy)-äthylamino]-2-propanol nach Anspruch 1, dadurch gekennzeichnet, dass man 1-[4-(1,6-Dihydro-1-methyl-6-oxo-2-pyridinyloxy)-phenoxy]-3-[2-(3-carbamoyl-4-hydroxy-phenoxy)-N-benzyläthylamino]-2-propanol als Ausgangsstoff verwendet.

10. Verfahren zur Herstellung von 1-[2-(3-Carbamoyl-4-hydroxy-phenoxy)-äthylamino]-3-[4-(1-methyl-3-methylthio-1H-1,2,4-triazol-5-yl)-phenoxy]-2-propanol nach Anspruch 1, dadurch gekennzeichnet, dass man 5-[4-(2,3-Epoxy-propoxy)-phenl]-1-methyl-3-methylthio-1H-1,2,4-triazol und 5-(2-Aminoäthoxy)-salicylamid als Ausgangsstoffe verwendet.

11. Verfahren zur Herstellung von 1-[2-(3-Carbamoyl-4-hydroxy-phenoxy)-äthylamino]-3-[4-(1-methyl-3-methylsulfonyl-1H-1,2,4,-triazol-5-yl)-phenoxy]-2-propanol nach Anspruch 1, dadurch gekennzeichnet, dass man 1-[N-Benzyl-[2-(3-carbamoyl-4-hydroxy-phenoxy)-äthylamino]]-3-[4-(1-methyl-3-methylsulfonyl-1H-1,2,4-triazol-5-yl)-phenoxy]-2-propanol als Ausgangsstoff verwendet.

12. Verfahren zur Herstellung von 1-[2-(3-Carbamoyl-4-hydroxyphenoxy)-äthylamino]-3-[4-(1,4,5-trimethyl-1H-imidazol-2-yl)-phenoxy]-2-propanol nach Anspruch 1, dadurch gekennzeichnet, dass man 1-[N-Benzyl-[2-(3-carbamoyl-4-hydroxy-phenoxy)-äthylamino]]-3-[4-(1,4,5-trimethyl-1H-imidazol-2-yl)-phenoxy]-2-propanol als Ausgangsstoff verwendet.

13. Verfahren nach einem der Ansprüche 1-12, dadurch gekennzeichnet, dass man eine erhaltene Verbindung der Formel I in ein pharmazeutisch verwendbares, nicht-toxisches Säureadditionssalz überführt.

**Claims** (for the Contracting States : DE, GB, FR, CH, LI, IT, NL, BE, SE, LU)

1. A substituted phenyl ether of the formula

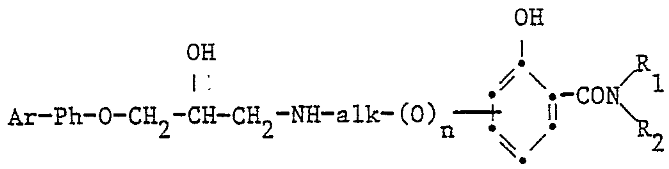

(I)

wherein Ar is an unsubstituted or substituted phenyl radical, or an unsubstituted or substituted monocyclic heteroaryl radical which has 5 or 6 ring members, is bonded to the phenylene radical Ph through a ring carbon atom, contains an oxygen sulfur or nitrogen atom and may also contain 1 to 3 additional nitrogen atoms as ring members which heteroaryl radical may be at least partially hydrogenated and, if so, may be substituted by oxo, the substituents being unsubstituted or substituted lower alkyl, or lower alkenyl, free, etherified or esterified hydroxy, lower alkylthio, lower alkylsulfonyl, or halogen, acyl, free or esterified carboxy, carbamoyl, cyano, nitro, unsubstituted or substituted, such as acylated, amino, or phenyl or benzoyl, each unsubstituted or substituted by lower alkyl, lower alkoxy, halogen, halo-lower alkyl, nitro or cyano, and/or monocyclic heteroaryl which contains an oxygen or nitrogen atom and may also contain 1 to 3 further nitrogen atoms and is unsubstituted or substituted as stated, Ph is 1,4-phenylene which is unsubstituted or substituted by lower alkyl, lower alkoxy, halogen or cyano, n is 0 or 1, and alk is an alkylene radical containing 2 to 4 carbon atoms, the nitrogen atom and the oxygen atom or, if n is 0, the aromatic radical being separated from each other by 2 or 3 carbon atoms in the unbranched chain, $R_1$ and $R_2$ are hydrogen or lower alkyl or, together with the nitrogen atom of the amide group, form pyrrolidino or morpholino.

2. A compound of formula I, wherein Ar is an unsubstituted or substituted phenyl radical, or an unsubstituted or substituted monocyclic heteroaryl radical which has 5 or 6 ring members, is bonded to the phenylene radical Ph through a ring carbon atom, contains an oxygen, sulfur or nitrogen atom and may also contain 1 or 2 additional nitrogen atoms as ring members, or is an at least partially hydrogenated oxo-heteroaryl radical containing 1 or 2 nitrogen atoms as ring members, the substituents being unsubstituted or substituted lower alkyl, free, etherified or esterified hydroxy, lower alkylthio or lower alkylsulfonyl, or halogen, acyl, free or esterified carboxy, carbamoyl, cyano, unsubstituted or substituted, such as acylated, amino, or phenyl or benzoyl, each unsubstituted or substituted by lower alkyl, lower alkoxy, halogen, halo-lower alkyl or cyano, and/or monocyclic heteroaryl which contains an oxygen or nitrogen atom and may also contain 1 or 2 further nitrogen atoms and is unsubstituted or substituted as stated, Ph is 1,4-phenylene which is unsubstituted or substituted by lower alkyl, lower alkoxy, halogen or cyano, n is 0 or 1, and alk is an alkylene radical containing 2 or 3 carbon atoms, the nitrogen atom and the oxygen atom or, if n is 0, the aromatic radical being separated from each other by 2 or 3 carbon atoms in the unbranched chain, $R_1$ and $R_2$ are hydrogen or lower alkyl or, together with the nitrogen atom of the amide group, form morpholino.

3. A compound of formula I, wherein Ar is an unsubstituted or substituted phenyl radical, or an unsubstituted or substituted heteroaryl radical which has 5 or 6 ring members, is bonded to the phenylene radical Ph through a ring carbon atom, contains an oxygen, sulfur or a nitrogen atom and may also contain 1 or 2 additional nitrogen atoms as ring members, e. g. furyl, oxazolyl, imidazolyl, triazolyl, thiazolyl, indolyl or pyridyl, or is a dihydro-oxo-heteroaryl radical containing 1 or 2 nitrogen atoms as ring members, e. g. dihydro-oxo-2-pyridinyl or dihydro-4-oxo-2-pyrimidinyl, the substituents being lower alkyl, e. g. methyl, halolower alkyl, e. g. trifluoromethyl, hydroxy, lower alkoxy, e. g. methoxy, halogen, lower alkanoyl, e. g. acetyl, carboxy, lower alkoxycarbonyl, e. g. methoxycarbonyl, cyano, carbamoyl, amino, lower alkanoylamino, e. g. acetylamino, lower alkoxycarbonylamino, e. g. methoxycarbonylamino, and/or unsubstituted or substituted, as stated, heteroaryl e. g. imidazolyl, or monocyclic heteroaryl which contains an oxygen or nitrogen atom and may also contain a further nitrogen atom, e. g. furyl or oxazolyl, Ph is 1,4-phenylene which is unsubstituted or substituted by lower alkyl such as methyl, or by halogen such as chlorine, n is 0 or 1, alk is an alkylene radical containing 2 or 3 carbon atoms, the nitrogen atom and the oxygen atom or, if n is 0, the aromatic radical being separated from each other by 2 or 3 carbon atoms in the unbranched chain, $R_1$ and $R_2$ are each hydrogen.

4. 1-[2-(3-Carbamoyl-4-hydroxyphenyl)ethylamino]-3-[4-[1-methyl-4-(trifluoromethyl)-1H-imidazol-2-yl]phenoxy]-2-propanol.

5. 1-[2-(3-Carbamoyl-4-hydroxyphenoxy)ethylamino]-3-[4-[1-methyl-1H-imidazol-2-yl]-phenoxy]-2-propanol.

6. 1-[4-(1,6-Dihydro-1-methyl-6-oxo-2-pyridinyloxy)phenoxy]-3-[2-(3-carbamoyl-4-hydroxyphenoxy) ethylamino]-2-propanol.

7. 1-[2-(3-Carbamoyl-4-hydroxyphenoxy)ethylamino]-3-[4-(1-methyl-3-methylthio-1H-1,2,4-triazol-5-yl)phenoxy]-2-propanol.

8. 1-[2-(3-Carbamoyl-4-hydroxyphenoxy)ethylamino]-3-[4-(1-methyl-3-methylsulfonyl-1H-1,2,4-

triazol-5-yl)phenoxy]-2-propanol.

9. 1-[2-(3-Carbamoyl-4-hydroxyphenoxy)ethylamino]-3-[4-(1,4,5-trimethyl-1H-imidazol-2-yl)phenoxy]-2-propanol.

10. An N-oxide of a compound according to any one of claims 1 to 9.

11. A salt of a compound according to any one of claims 1 to 10.

12. A pharmaceutically acceptable, non-toxic acid addition salt of a compound according to any one of claims 1 to 10.

13. A pharmaceutical composition containing a compound according to any one of claims 1 to 10 and 12.

14. A compound of formula I or a salt thereof for use in a therapeutic method of treating the human or animal body.

15. A beta-receptor-blocking compound of formula I or a salt thereof as agent for combating Angina pectoris, hypertrophic types of cardiomyopathy and heart rhythm disorders, high blood pressure, and also increased intraocular pressure and thyreotoxicosis.

16. A beta-receptor-stimulating compound of formula I or a salt thereof as agent for combating heart muscle insufficiency.

17. Use of a compound of formula I for the preparation of a pharmaceutical composition.

18. A process for the preparation of a substituted phenyl ether of the formula

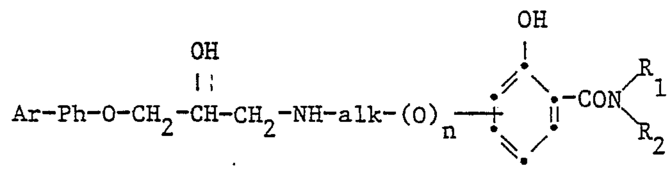

(I)

wherein Ar is an unsubstituted or substituted phenyl radical, or an unsubstituted or substituted monocyclic heteroaryl radical which has 5 or 6 ring members, is bonded to the phenylene radical Ph through a ring carbon atom, contains an oxygen, sulfur or nitrogen atom and may also contain 1 to 3 additional nitrogen atoms as ring members, which heteroaryl radical may be at least partially hydrogenated and, if so, may be substituted by oxo, the substituents being unsubstituted or substituted lower alkyl, or lower alkenyl, free, etherified or esterified hydroxy, lower alkylthio, lower alkylsulfonyl, or halogen, acyl, free or esterified carboxy, carbamoyl, cyano, nitro, unsubstituted or substituted, such as acylated, amino, or phenyl or benzoyl, each unsubstituted or substituted by lower alkyl, lower alkoxy, halogen, halo-lower alkyl, nitro or cyano, and/or monocyclic heteroaryl which contains an oxygen or nitrogen atom and may also contain 1 to 3 further nitrogen atoms and is unsubstituted or substituted as stated, Ph is 1,4-phenylene which is unsubstituted or substituted by lower alkyl, lower alkoxy, halogen or cyano, n is 0 or 1, and alk is an alkylene radical containing 2 to 4 carbon atoms, the nitrogen atom and the oxygen atom or, if n is 0, the aromatic radical being separated from each other by 2 or 3 carbon atoms in the unbranched chain, $R_1$ and $R_2$ are hydrogen or lower alkyl or, together with the nitrogen atom of the amide group, form pyrrolidino or morpholino, or of a salt thereof, characterised in that

a) in a compound of the formula

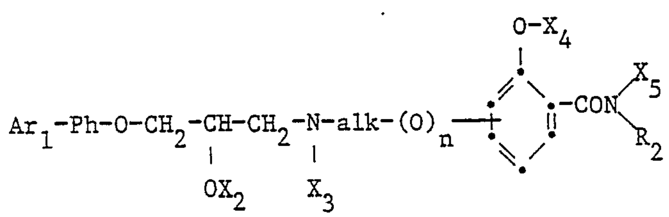

(II)

wherein $Ar_1$ is the radical Ar or a radical corresponding thereto having at least one functional grouping in protected form, $X_2$, $X_3$ and $X_4$ are each hydrogen or a substituent which can be replaced by hydrogen, $X_5$ is $R_1$ or a substituent which can be replaced by hydrogen, or $X_2$ and $X_3$ and/or $X_4$ and $X_5$ together are a bivalent radical which can be replaced by two hydrogen atoms, and/or any functional groups present in the radical $Ar_1$ are in unprotected or protected form, with the proviso that at least one of the radicals $X_2$, $X_3$, $X_4$ or $X_5$ is different from hydrogen, or at least $Ar_1$ is a radical Ar which contains at least one functional grouping in protected form, or at least $X_2$ and $X_3$ together or $X_4$ and $X_5$ together are a bivalent radical which can be replaced by two hydrogen atoms, or in a salt thereof, the groups $X_2$, $X_3$, $X_4$ or $X_5$, or $X_2$ and $X_3$ together, or $X_4$ and $X_5$ together which are different from hydrogen are replaced by hydrogen atoms, and/or the protecting groups bonded to one or more of the functional groups in a radical $Ar_1$ are split off and replaced by hydrogen, or

b) a compound of the formula

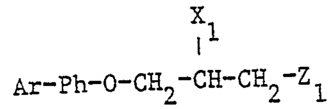

(III)

is reacted with a compound of the formula

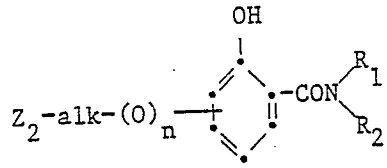

(IV)

or with a salt thereof, in which formulae one of the groups $Z_1$ and $Z_2$ is a reactive esterified hydroxy group and the other is a primary amino group and $X_1$ is hydroxy, or in which $X_1$ and $Z_1$ together are the epoxy group and $Z_2$ is a primary amino group and Ar, Ph, n, alk, $R_1$ and $R_2$ have the meanings given above, or

c) in a compound of the formula

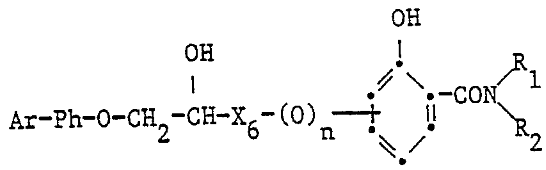

(V)

wherein $X_6$ is a reducible group of the formula

$$-CH=N-alk- \quad (Va) \quad \text{or} \quad -CH_2-N=alk_1- \quad (Vb)$$

wherein $alk_1$ is an alkylidene radical corresponding to the radical alk, and n is 0 or 1, $X_6$ is reduced to the group of the formula

$$-CH_2-NH-alk- \quad (Vc)$$

any protecting groups present which are located at functional groups and do not participate in the reaction are simultaneously or subsequently split off and replaced by hydrogen, or

d) a compound of the formula

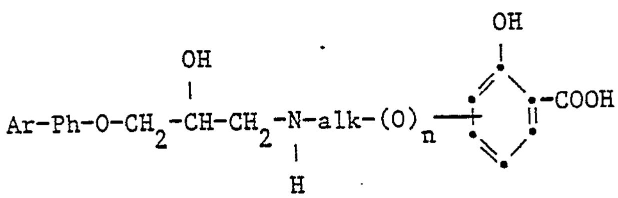

(VI)

in which functional groups not participating in the reaction are unprotected or protected by protecting groups which can be split off by aminolysis or ammonolysis and replaced by hydrogen, is reacted with a compound of the formula

$$HNR_1R_2 \quad (VIa)$$

and any protecting groups present are simultaneously or subsequently split off and replaced by hydrogen, or

e) in a compound of the formula

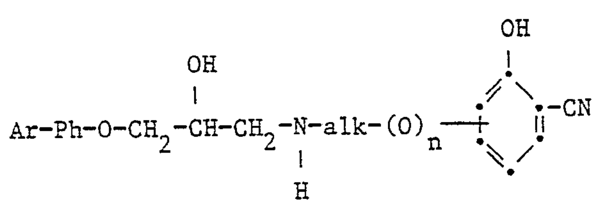

(VII)

in which functional groups not participating in the reaction are unprotected or protected by protecting

groups which can be split off by hydrolysis and replaced by hydrogen and which are split off and replaced by hydrogen under the conditions of the process, the group —CN is converted into the group —$CONH_2$ by means of hydrolysis and, at the same time, any protecting groups present are split off and replaced by hydrogen, or

f) to prepare a compound of the formula I wherein Ar is a mono- or bicyclic heteroaryl radical, in a compound of the formula

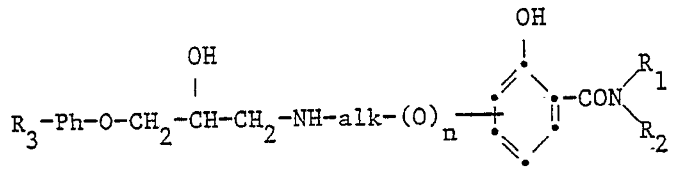
(VIII)

in which hydroxy groups and/or the amino group are protected by protecting groups and $R_3$ is a substituent capable of forming a mono- or bicyclic heteroaryl radical Ar, the mono- or bicyclic heteroaryl group Ar is formed, any protecting groups present are simultaneously or subsequently split off and replaced by hydrogen and, if desired, a compound obtainable in this manner is converted into a different compound of the formula I and/or, if desired, a resultant free compound is converted into a salt or a resultant salt is converted into a free compound, and/or, if desired, a resultant mixture of isomers is separated into the isomers, or a resultant racemate is resolved into the antipodes.

19. A compound obtainable by the process according to claim 18.

**Claims** (for the Contracting State AT)

1. A process for the preparation of a substituted phenyl ether of the formula

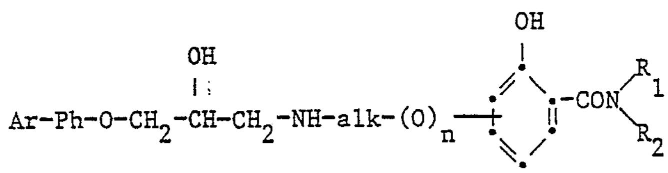
(I)

wherein Ar is an unsubstituted or substituted phenyl radical, or an unsubstituted or substituted monocyclic heteroaryl radical which has 5 or 6 ring members, is bonded to the phenylene radical Ph through a ring carbon atom, contains an oxygen, sulfur or nitrogen atom and may also contain 1 to 3 additional nitrogen atoms as ring members, which heteroaryl radical may be at least partially hydrogenated and, if so, may be substituted by oxo, the substituents being unsubstituted or substituted lower alkyl, or lower alkenyl, free, etherified or esterified hydroxy, lower alkylthio, lower alkylsulfonyl, or halogen, acyl, free or esterified carboxy, carbamoyl, cyano, nitro, unsubstituted or substituted, such as acylated, amino, or phenyl or benzoyl, each unsubstituted or substituted by lower alkyl, lower alkoxy, halogen, halo-lower alkyl, nitro or cyano, and/or monocyclic heteroaryl which contains an oxygen or nitrogen atom and may also contain 1 to 3 further nitrogen atoms and is unsubstituted or substituted as stated, Ph is 1,4-phenylene which is unsubstituted or substituted by lower alkyl, lower alkoxy, halogen or cyano, n is 0 or 1, and alk is an alkylene radical containing 2 to 4 carbon atoms, the nitrogen atom and the oxygen atom or, if n is 0, the aromatic radical being separated from each other by 2 or 3 carbon atoms in the unbranched chain, $R_1$ and $R_2$ are hydrogen or lower alkyl or, together with the nitrogen atom of the amide group, form pyrrolidino or morpholino, or of a salt thereof, characterised in that

a) in a compound of the formula

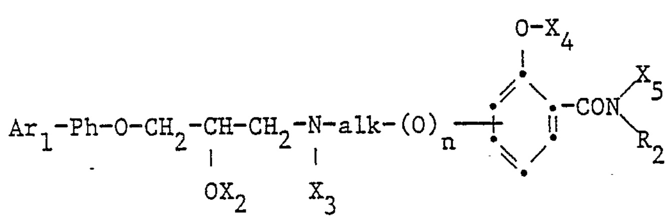
(II)

wherein $Ar_1$ is the radical Ar or a radical corresponding thereto having at least one functional grouping in protected from, $X_2$, $X_3$ and $X_4$ are each hydrogen or a substituent which can be replaced by hydrogen, $X_5$ is $R_1$ or a substituent which can be replaced by hydrogen, or $X_2$ and $X_3$ and/or $X_4$ and $X_5$ together are a bivalent radical which can be replaced by two hydrogen atoms, and/or any functional groups present in the radical $Ar_1$ are in unprotected or protected form, with the proviso that at least one of the radicals $X_2$,

$X_3$, $X_4$ or $X_5$ is different from hydrogen, or at least $Ar_1$ is a radical Ar which contains at least one functional grouping in protected form, or at least $X_2$ and $X_3$ together or $X_4$ and $X_5$ together are a bivalent radical which can be replaced by two hydrogen atoms, or in a salt thereof, the groups $X_2$, $X_3$, $X_4$ or $X_5$, or $X_2$ and $X_3$ together, or $X_4$ and $X_5$ together which are different from hydrogen are replaced by hydrogen atoms, and/or the protecting groups bonded to one or more of the functional groups in a radical $Ar_1$ are split off and replaced by hydrogen, or

b) a compound of the formula

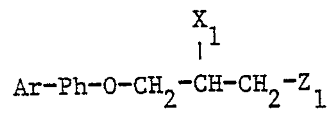

(III)

is reacted with a compound of thė formula

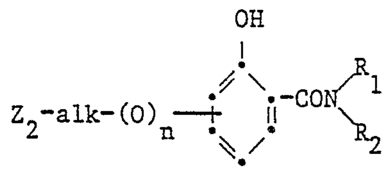

(IV)

or with a salt thereof, in which formulae one of the groups $Z_1$ and $Z_2$ is a reactive esterified hydroxy group and the other is a primary amino group and $X_1$ is hydroxy, or in which $X_1$ and $Z_1$ together are the epoxy group and $Z_2$ is a primary amino group and Ar, Ph, n, alk, $R_1$ and $R_2$ have the meanings given above, or

c) in a compound of the formula

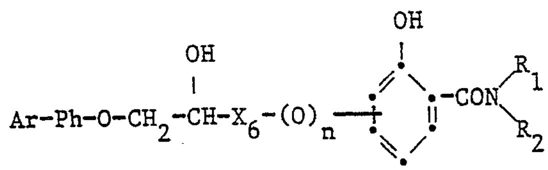

(V)

wherein $X_6$ is a reducible group of the formula

$$—CH{=}N—alk— \quad (Va) \quad \text{or} \quad —CH_2—N{=}alk_1— \quad (Vb)$$

wherein $alk_1$ is an alkylidene radical corresponding to the radical alk, and n is 0 or 1, $X_6$ is reduced to the group of the formula

$$—CH_2—NH—alk— \quad (Vc)$$

any protecting groups present which are located at functional groups and do not participate in the reaction are simultaneously or subsequently split off and replaced by hydrogen, or

d) a compound of the formula

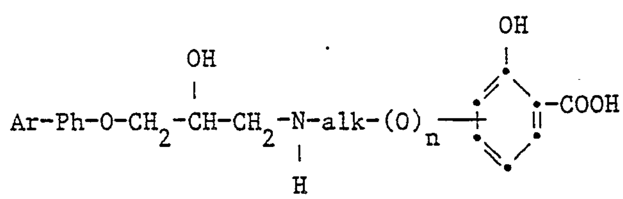

(VI)

in which functional groups not participating in the reaction are unprotected or protected by protecting groups which can be split off by aminolysis or ammonolysis and replaced by hydrogen, is reacted with a compound of the formula

$$HNR_1R_2 \quad (VIa)$$

and any protecting groups present are simultaneously or subsequently split off and replaced by hydrogen, or

e) in a compound of the formula

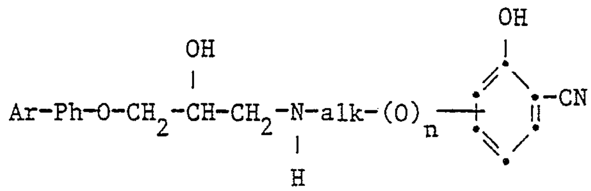

(VII)

in which functional groups not participating in the reaction are unprotected or protected by protecting groups which can be split off by hydrolysis and replaced by hydrogen and which are split off and replaced by hydrogen under the conditions of the process, the group —CN is converted into the group —$CONH_2$ by means of hydrolysis and, at the same time, any protecting groups present are split off and replaced by hydrogen, or

f) to prepare a compound of the formula I wherein Ar is a mono- or bicyclic heteroaryl radical, in a compound of the formula

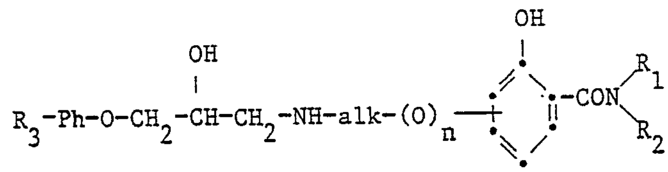

(VIII)

in which hydroxy groups and/or the amino group are protected by protecting groups and $R_3$ is a substituent capable of forming a mono- or bicyclic heteroaryl radical Ar, the mono- or bicyclic heteroaryl group Ar is formed, any protecting groups present are simultaneously or subsequently split off and replaced by hydrogen and, if desired, a compound obtainable in this manner is converted into a different compound of the formula I and/or if desired, a resultant free compound is converted into a salt or a resultant salt is converted into a free compound, and/or, if desired, a resultant mixture of isomers is separated into the isomers, or a resultant racemate is resolved into the antipodes.

2. A process according to claim 1, wherein Ar is an unsubstituted or substituted phenyl radical, or an unsubstituted or substituted monocyclic heteroaryl radical which has 5 or 6 ring members, is bonded to the phenylene radical Ph through a ring carbon atom, contains an oxygen, sulfur or nitrogen atom and may also contain 1 or 2 additional nitrogen atoms as ring members, or is an at least partially hydrogenated oxo-heteroaryl radical containing 1 or 2 nitrogen atoms as ring members, the substituents being unsubstituted or substituted lower alkyl, free, etherified or esterified hydroxy, lower alkylthio or lower alkylsulfonyl, or halogen, acyl, free or esterified carboxy, carbamoyl, cyano, unsubstituted or substituted, such as acylated, amino, or phenyl or benzoyl, each unsubstituted or substituted by lower alkyl, lower alkoxy, halogen, halo-lower alkyl or cyano, and/or monocyclic heteroaryl which contains an oxygen or nitrogen atom and may also contain 1 or 2 further nitrogen atoms and is unsubstituted or substituted as stated, Ph is 1,4-phenylene which is unsubstituted or substituted by lower alkyl, lower alkoxy, halogen or cyano, n is 0 or 1, and alk is an alkylene radical containing 2 or 3 carbon atoms, the nitrogen atom and the oxygen atom or, if n is 0, the aromatic radical being separated from each other by 2 or 3 carbon atoms in the unbranched chain, $R_1$ and $R_2$ are hydrogen or lower alkyl or, together with the nitrogen atom of the amide group, form morpholino, which process comprises starting from corresponding starting materials.

3. A process according to claim 1, wherein Ar is an unsubstituted or substituted phenyl radical, or an unsubstituted or substituted heteroaryl radical which has 5 or 6 ring members, is bonded to the phenylene radical Ph through a ring carbon atom, contains an oxygen, sulfur or a nitrogen atom and may also contain 1 or 2 additional nitrogen atoms as ring members, e. g. furyl, oxazolyl, imidazolyl, triazolyl, thiazolyl, indolyl or pyridyl, or is a dihydro-oxo-heteroaryl radical containing 1 or 2 nitrogen atoms as ring members, e. g. dihydro-oxo-2-pyridinyl or dihydro-4-oxo-2-pyrimidinyl, the substituents being lower alkyl, e. g. methyl, halolower alkyl, e. g. trifluoromethyl, hydroxy, lower alkoxy, e. g. methoxy, halogen, lower alkanoyl, e. g. acetyl, carboxy, lower alkoxycarbonyl, e. g. methoxycarbonyl, cyano, carbamoyl, amino, lower alkanoylamino, e. g. acetylamino, lower alkoxycarbonylamino, e. g. methoxycarbonylamino, and/or unsubstituted or substituted, as stated, heteroaryl e. g. imidazolyl, or monocyclic heteroaryl which contains an oxygen or nitrogen atom and may also contain a further nitrogen atom, e. g. furyl or oxazolyl, Ph is 1,4-phenylene which is unsubstituted or substituted by lower alkyl such as methyl, or by halogen such as chlorine, n is 0 or 1, alk is an alkylene radical containing 2 or 3 carbon atoms, the nitrogen atom and the oxygen atom or, if n is 0, the aromatic radical being separated from each other by 2 or 3 carbon atoms in the unbranched chain, $R_1$ and $R_2$ are each hydrogen, which process comprises starting from corresponding starting materials.

4. A process for the preparation of 1-[2-(3-carbamoyl-4-hydroxyphenoxy)ethylamino]-3-[4-[1-methyl-4-(trifluoromethyl)-1H-imidazol-2-yl]-phenoxy]-2-propanol according to claim 1, which process comprises using as starting material 1-[N-benzyl-2-(3-carbamoyl-4-hydroxyphenoxy)ethylamino]-3-[4-[1-methyl-4-(trifluoromethyl)-1H-imidazol-2-yl]phenoxy]-2-propanol.

5. A process for the preparation of 1-[2-(3-carbamoyl-4-hydroxyphenoxy)ethylamino]-3-[4-[1-methyl-4-(trifluoromethyl)-1H-imidazol-2-yl]phenoxy]-2-propanol according to claim 1, which process comprises using as starting materials 1-amino-3-[4-(1-methyl-4-(trifluoromethyl)-1H-imidazol-2-yl)phenoxy]-2-propanol and 5-(2-bromoethoxy)salicylamide.

6. A process for the preparation of 1-[2-(3-carbamoyl-4-hydroxyphenoxy)ethylamino]-3-[4-[1-methyl-4-(trifluoromethyl)-1H-imidazol-2-yl]phenoxy]-2-propanol according to claim 1, which process comprises using as starting material the Schiff's base formed from 1-amino-3-[4-(1-methyl-4-(trifluoromethyl)-1H-imidazol-2-yl)phenoxy]-2-propanol and 2,3-dihydro-2,2-dimethyl-7-(2-oxoethoxy)-4H-1,3-benzoxazin-4-one.

7. A process for the preparation of 1-[2-(3-carbamoyl-4-hydroxyphenoxy)ethylamino]-3-[4-[1-methyl-4-(trifluoromethyl)-1H-imidazol-2-yl]phenoxy]-2-propanol according to claim 1, which process comprises using as starting material 1-[2-(3-carbomethoxy-4-hydroxyphenoxy)ethylamino]-3-(4-[1-methyl-4-(trifluoromethyl)-1H-imidazol-2-yl]phenoxy]-2-propanol.

8. A process for the preparation of 1-[2-(3-carbamoyl-4-hydroxyphenoxy)ethylamino]-3-[4-(1-methyl-1H-imidazol-2-yl)phenoxy]-2-propanol according to claim 1, which process comprises using as starting material 1-[N-benzyl-2-(3-carbamoyl-4-benzyloxyphenoxy)ethylamino]-3-[4-[1-methyl-1H-imidazol-2-yl]phenoxy]-2-propanol.

9. A process for the preparation of 1-[4-(1,6-dihydro-1-methyl-6-oxo-2-pyridinyloxy)phenoxy]-3-[2-(3-carbamoyl-4-hydroxyphenoxy)ethylamino]-2-propanol according to claim 1, which process comprises using as starting material 1-[4-(1,6-dihydro-1-methyl-6-oxo-2-pyridinyloxy)phenoxy]-3-[2-(3-carbamoyl-4-hydroxyphenoxy)-N-benzylethylamino-2-propanol.

10. A process for the preparation of 1-[2-(3-carbamoyl-4-hydroxyphenoxy)ethylamino]-3-[4-(1-methyl-3-methylthio-1H-1,2,4-triazol-5-yl)phenoxy]-2-propanol according to claim 1, which process comprises using as starting materials 5-[4-(2,3-epoxypropoxy)phenyl]-1-methyl-3-methylthio-1H-1,2,4-triazole and 5-(2-aminoethoxy)salicylamide.

11. A process for the preparation of 1-[2-(3-carbamoyl-4-hydroxyphenoxy)ethylamino]-3-[4-(1-methyl-3-methylsulfonyl-1H-1,2,4-triazol-5-yl)phenoxy]-2-propanol according to claim 1, which process comprises using as starting material 1-[N-benzyl-[2-(3-carbamoyl-4-hydroxyphenoxy)ethylamino]]-3-[4-(1-methyl-3-methylsulfonyl-1H-1,2,4-triazol-5-yl)phenoxy]-2-propanol.

12. A process for the preparation of 1-[2-(3-carbamoyl-4-hydroxyphenoxy)ethylamino]-3-[4-(1,4,5-trimethyl-1H-imidazol-2-yl)phenoxy]-2-propanol according to claim 1, which process comprises using as starting material 1-[N-benzyl-[2-(3-carbamoyl-4-hydroxyphenoxy)ethylamino]]-3-[4-(1,4,5-trimethyl-1H-imidazol-2-yl)phenoxy]-2-propanol.

13. A process according to any one of claims 1 to 12, which comprises converting a resultant compound of formula I into a pharmaceutically acceptable, non-toxic acid addition salt.

**Revendications** (pour les Etats contractants : BE, CH, DE, FR, GB, IT, LI, LU, NL, SE)

1. Ethers phényliques substitués de formule

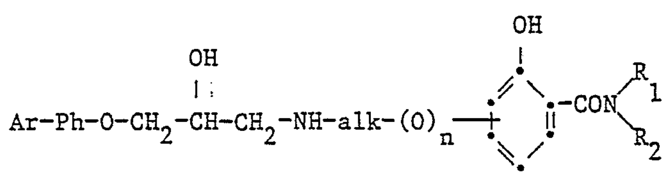

(I)

dans laquelle Ar représente un groupe phényle éventuellement substitué, ou bien un groupe hétéroaryle monocyclique à 5-6 chaînons cycliques reliés par l'intermédiaire d'un atome de carbone cyclique au groupe phénylène Ph, contenant en tant que chaînons cycliques un atome d'oxygène, de soufre ou d'azote et le cas échéant 1 à 3 autres atomes d'azote, qui est au moins partiellement hydrogéné et dans ce cas peut être substitué par un groupe oxo, les substituants étant des groupes alkyle inférieurs éventuellement substitués ou alcényle inférieurs, hydroxy éventuellement éthérifié ou estérifié, alkylthio inférieur, alkylsulfonyle inférieur ou des halogènes, des groupes acyle, carboxy éventuellement estérifié, carbamoyle, cyano, nitro, amino éventuellement substitué, par exemple acylé, phényle ou benzoyle éventuellement substitué par des groupes alkyle inférieurs, alcoxy inférieurs, des halogènes, des groupes halogénoalkyle inférieurs, nitro ou cyano et/ou un groupe hétéroaryle monocyclique éventuellement substitué comme indiqué ci-dessus, contenant un atome d'oxygène ou d'azote et le cas échéant de 1 à 3 atomes d'azote, Ph représente un groupe 1,4-phénylène éventuellement substitué par des groupes alkyle inférieurs, alcoxy inférieurs, des halogènes ou des groupes cyano, n est égal à 0 ou 1 et alk représente un groupe alkylène en C2-C4, le reste aromatique étant séparés l'un de l'autre par 2 à 3 atomes de carbone dans la chaîne non ramifiée, $R_1$ et $R_2$ représentent l'hydrogène ou des groupes alkyle inférieurs ou forment ensemble et avec l'atome d'azote du groupe amide un cycle pyrrolidino ou morpholino.

2. Composés de formule I dans laquelle Ar représente un groupe phényle éventuellement substitué

ou un groupe hétéroaryle monocyclique à 5-6 chaînons cycliques relié au groupe phénylène Ph par l'intermédiaire d'un atome de carbone cyclique, contenant en tant que chaînons cycliques un atome d'oxygène, de soufre ou d'azote et le cas échéant 1 à 2 autres atomes d'azote, ou un groupe oxo-hétéroaryle au moins partiellement hydrogéné, qui contient 1 à 2 atomes d'azote en tant que chaînons cycliques, les substituants étant des groupes alkyle inférieurs éventuellement substitués, hydroxy éventuellement éthérifié ou estérifié, alkylthio inférieur ou alkylsulfonyle inférieur, ou des halogènes, des groupes acyle, carboxy éventuellement estérifié, carbamoyle, cyano, amino éventuellement substitué, par exemple acylé, phényle ou benzoyle éventuellement substitué par des groupes alkyle inférieurs, alcoxy inférieur, des halogènes, des groupes halogénoalkyle inférieurs ou cyano et/ou un groupe hétéroaryle monocyclique éventuellement substitué comme indiqué ci-dessus, contenant un atome d'oxygène ou d'azote et le cas échéant 1 à 2 autres atomes d'azote, Ph représente un groupe 1,4-phénylène éventuellement substitué par des groupes alkyle inférieurs, alcoxy inférieurs, des halogènes ou des groupes cyano, n est égal à 0 ou 1 et alk représente un groupe alkylène en C2-C3, l'atome d'azote et l'atome d'oxygène ou bien, lorsque n est égal à 0, le groupe aromatique, étant séparés l'un de l'autre par 2 à 3 atomes de carbone dans la chaîne non ramifiée, $R_1$ et $R_2$ représentent l'hydrogène ou des groupes alkyle inférieurs ou forment ensemble et avec l'atome d'azote du groupe amide un cycle morpholino.

3. Composés de formule I dans laquelle Ar représente un groupe phényle éventuellement substitué ou un groupe hétéroaryle à 5-6 chaînons cycliques relié au groupe phénylène Ph par l'intermédiaire d'un atome de carbone cyclique, contenant en tant que chaînons cycliques un atome d'oxygène, de soufre ou un atome d'azote et le cas échéant 1 à 2 autres atomes d'azote, par exemple furyle, oxazolyle, imidazolyle, triazolyle, thiazolyle, indolyle ou pyridyle, ou un groupe dihydro-oxo-hétéroaryle contenant 1 à 2 atomes d'azote en tant que chaînons cycliques, par exemple dihydro-oxo-2-pyridinyle ou dihydro-4-oxo-2-pyrimidinyle, les substituants étant des groupes alkyle inférieurs, par exemple méthyle, halogénoalkyle inférieurs par exemple trifluorométhyle, hydroxy, alcoxy inférieur, par exemple méthoxy, des halogènes, des groupes alcanoyle inférieurs, par exemple acétyle, carboxy, (alcoxy inférieur)-carbonyle, par exemple méthoxycarbonyle, cyano, carbamoyle, amino, alcanoylamino inférieur, par exemple acétylamino, (alcoxy inférieur)-carbonylamino, par exemple méthoxycarbonylamino et/ou un groupe hétéroaryle éventuellement substitué comme indiqué ci-dessus, par exemple imidazolyle ou un groupe hétéroaryle monocyclique contenant un atome d'oxygène ou d'azote et le cas échéant un autre atome d'azote, par exemple un groupe furyle ou oxazolyle, Ph représente un groupe 1,4-phénylène éventuellement substitué par des groupes alkyle inférieurs tels que méthyle ou des halogènes tels que le chlore, n est égal à 0 ou 1, alk représente un groupe alkylène en C2-C3, l'atome d'azote et l'atome d'oxygène, ou bien, lorsque n est égal à 0, le reste aromatique, étant séparés l'un de l'autre par 2 à 3 atomes de carbone dans la chaîne non ramifiée, $R_1$ et $R_2$ représentent chacun l'hydrogène.

4. Le 1-[2-(3-carbamoyl-4-hydroxy-phénoxy)éthylamino]-3-[4-[1-méthyl-4-(trifluorométhyl)-1H-imidazole-2-yl]-phénoxy]-2-propanol.

5. Le 1-[2-(3-carbamoyl-4-hydroxy-phénoxy)éthylamino]-3-[4-[1-méthyl-1H-imidazole-2-yl]-phénoxy]-2-propanol.

6. Le 1-[4-(1,6-dihydro-1-méthyl-6-oxo-2-pyridinyloxy)-phénoxy]-3-[2-(3-carbamoyl-4-hydroxy-phénoxy)-éthyl-amino]-2-propanol.

7. Le 1-[2-(3-carbamoyl-4-hydroxy-phénoxy)éthylamino]-3-[4-(1-méthyl-3-méthylthio-1H-1,2,4-triazole-5-yl)-phénoxy]-2-propanol.

8. Le 1-[2-(3-carbamoyl-4-hydroxy-phénoxy)éthylamino]-3-[4-(1-méthyl-3-méthylsulfonyl-1H-1,2,4-triazole-5-yl)-phénoxy]-2-propanol.

9. Le 1-[2-(3-carbamoyl-4-hydroxy-phénoxy)éthylamino]-3-[4-(1,4,5-triméthyl-1H-imidazole-2-yl)-phénoxy]-2-propanol.

10. Les N-oxydes des composés des revendications 1 à 9.

11. Les sels des composés des revendications 1 à 10.

12. Les sels formés par addition avec des acides non toxiques, acceptables pour l'usage pharmaceutique, des composés des revendications 1 à 10.

13. Compositions pharmaceutiques contenant un composé des revendications 1 à 10 et 12.

14. Composés de formule I et leurs sels pour l'utilisation dans un procédé pour le traitement thérapeutique de l'organisme humain ou animal.

15. Composés de formule I bloquant les bêta-récepteurs ou leurs sels en tant que produits pour le traitement de l'angine de poitrine, des cardiomyopathies hypertrophiques et des troubles du rythme cardiaque, de l'hypertension sanguine ainsi que de l'hypertension intra-oculaire et des thyréotoxicoses.

16. Composés de formule I stimulant les bêta-récepteurs ou leurs sels en tant que produits pour le traitement de l'insuffisance musculaire cardiaque.

17. Utilisation des composés de formule I pour la préparation de compositions pharmaceutiques.

18. Procédé de préparation des éthers phényliques substitués de formule.

(Voir dessin page 54)

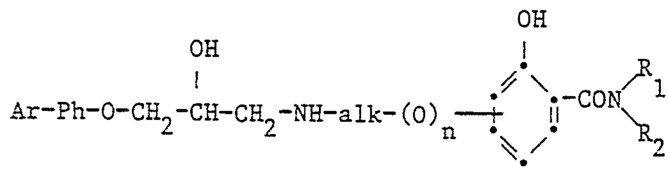

(I)

dans laquelle Ar représente un groupe phényle éventuellement substitué ou un groupe hétéroaryle monocyclique à 5-6 chaînons cycliques reliés au groupe phénylène Ph par l'intermédiaire d'un atome de carbone cyclique, contenant en tant que chaînons cycliques un atome d'oxygène, de soufre ou d'azote et le cas échéant 1 à 3 autres atomes d'azote, qui est au moins partiellement hydrogéné et dans ce cas peut être substitué par un groupe oxo, les substituants étant des groupes alkyle inférieurs éventuellement substitués ou alcényle inférieurs, hydroxy éventuellement éthérifié ou estérifié, alkylthio inférieur, alkylsulfonyle inférieur ou des halogènes, des groupes acyle, carboxy éventuellement estérifié, carbamoyle, cyano, nitro, amino éventuellement substitué, par exemple acylé, phényle ou benzoyle éventuellement substitué par des groupes alkyle inférieurs, alcoxy inférieur, des halogènes, des groupes halogénoalkyle inférieurs, nitro ou cyano ou un groupe hétéroaryle monocyclique éventuellement substitué comme indiqué ci-dessus, contenant un atome d'oxygène ou d'azote et le cas échéant de 1 à 3 autres atomes d'azote, Ph représente un groupe 1,4-phénylène éventuellement substitué par des groupes alkyle inférieurs, alcoxy inférieur, des halogènes ou des groupes cyano, n est égal à 0 ou 1 et alk représente un groupe alkylène en C2-C4, l'atome d'azote et l'atome d'oxygène ou bien, lorsque n est égal à 0, le groupe aromatique étant séparés l'un de l'autre par 2 à 3 atomes de carbone dans la chaîne non ramifiée, $R_1$ et $R_2$ représentent l'hydrogène ou des groupes alkyle inférieurs ou forment ensemble et avec l'atome d'azote du groupe amide un cycle pyrrolidino ou morpholino, ou de leurs sels, caractérisé en ce que :

a) dans un composé de formule :

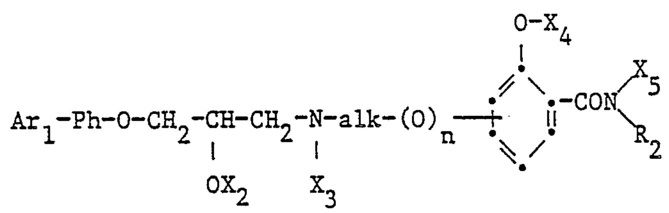

(II)

dans laquelle $Ar_1$ représente le groupe Ar ou un groupe correspondant à celui-ci, contenant au moins un groupement fonctionnel à l'état protégé, $X_2$, $X_3$ et $X_4$ représentent chacun l'hydrogène ou un substituant remplaçable par l'hydrogène, $X_5$ représente $R_1$ ou un substituant remplaçable par l'hydrogène, ou bien $X_2$ et $X_3$ et/ou $X_4$ et $X_5$ représentent ensemble un groupe divalent remplaçable par deux atomes d'hydrogène, et/ou les groupes fonctionnels éventuellement présents dans le groupe $Ar_1$ sont éventuellement à l'état protégé, étant spécifié que l'un au moins des substituants $X_2$, $X_3$, $X_4$ ou $X_5$ n'est pas l'hydrogène, ou qu'au moins $Ar_1$ représente un groupe Ar qui contient au moins un groupement fonctionnel à l'état protégé, ou qu'au moins $X_2$ et $X_3$ ensemble, ou $X_4$ et $X_5$ ensemble, représentent un reste divalent remplaçable par deux atomes d'hydrogène, ou dans un sel d'un tel composé, on remplace par des atomes d'hydrogène les groupes $X_2$, $X_3$, $X_4$ ou $X_5$ ou respectivement $X_2$ et $X_3$ ensemble ou $X_4$ ou $X_5$ ensemble, lorsqu'ils diffèrent de l'hydrogène, et/ou on élimine les groupes protecteurs fixés sur un ou plusieurs groupes fonctionnels dans un groupe $Ar_1$ et on les remplace par l'hydrogène, ou bien

b) on fait réagir un composé de formule

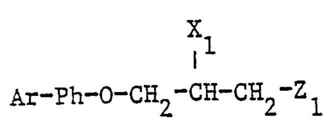

(III)

avec un composé de formule

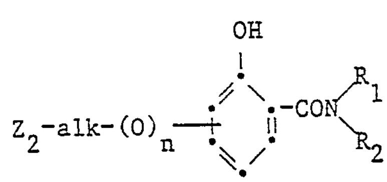

(IV)

ou avec un sel d'un tel composé, dans lesquels l'un des symboles $Z_1$ et $Z_2$ représente un groupe hydroxy

estérifié réactif et les autres le groupe amino primaire, et $X_1$ représente un groupe hydroxy, ou bien dans lesquels $X_1$ et $Z_1$ forment ensemble le groupe époxy et $Z_2$ représente le groupe amino primaire, et Ar, Ph, n, alk, $R_1$ et $R_2$ ont les significations indiquées ci-dessus, ou bien

c) dans un composé de formule

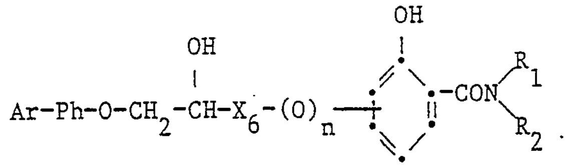 (V)

dans laquelle $X_6$ représente un groupe réductible de formule

$-CH{=}N-alk-$ (Va) ou $-CH_2-N = alk_1-$ (Vb)

$alk_1$ représentant un groupe alkylylidène correspondant au groupe alk, et n est égal à 0 ou 1, on réduit $X_6$ en le groupe de formule

$-CH_2-NH-alk-$ (Vc)

on élimine simultanément ou à la suite les groupes protecteurs éventuellement présents sur des groupes fonctionnels et qui ne participent pas à la réaction et on les remplace par l'hydrogène, ou bien

d) on fait réagir un composé de formule

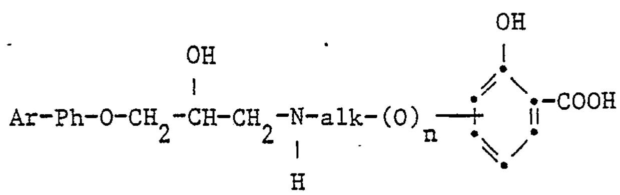 (VI)

dans laquelle les groupes fonctionnels qui ne participent pas à la réaction sont éventuellement protégés par des groupes protecteurs éliminables par aminolyse ou ammoniolyse et remplaçables par l'hydrogène, avec un composé de formule

$HNR_1R_2$ (VIa)

et simultanément ou à la suite, on élimine les groupes protecteurs éventuellement présents et on les remplace par l'hydrogène, ou bien

e) dans un composé de formule

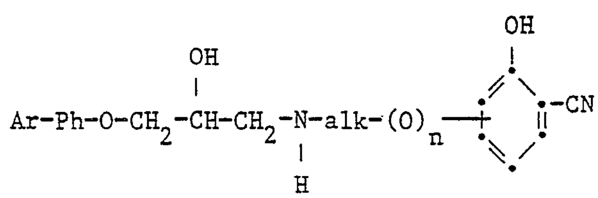 (VII)

dans lequel des groupes fonctionnels ne participant pas à la réaction sont éventuellement protégés par des groupes protecteurs éliminables par hydrolyse et remplaçables par l'hydrogène, ces groupes protecteurs étant éliminés dans les conditions opératoires et remplacés par l'hydrogène, on convertit le groupe $-CN$ par hydrolyse en le groupe $-CONH_2$ et on élimine simultanément les groupes protecteurs éventuellement présents et on les remplace par l'hydrogène, ou bien

f) pour préparer les composés de formule I dans laquelle Ar représente un groupe hétéroaryle mono- ou bi-cyclique, on part d'un composé de formule

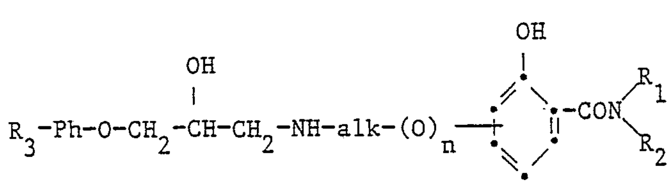 (VIII)

dans laquelle les groupes hydroxy et/ou le groupe amino sont protégés par des groupes protecteurs, et $R_3$ représente un substituant capable de former un groupe hétéroaryle mono- ou bi-cyclique Ar, on forme le groupe hétéroaryle mono- ou bi-cyclique Ar, on élimine simultanément ou à la suite les groupes protecteurs éventuellement présents et on les remplace par l'hydrogène, et, si on le désire, on convertit un composé ainsi obtenu en un autre composé de formule I et/ou, si on le désire, on convertit un composé libre obtenu en un sel ou un sel obtenu en un composé libre, et/ou, si on le désire, on sépare un mélange d'isomères obtenu en les isomères ou un racémate obtenu en les antipodes.

19. Les composés pouvant être obtenus par le procédé selon la revendication 18.

**Revendications** (pour l'Etat contractant AT)

1. Procédé de préparation d'éthers phényliques substitués de formule

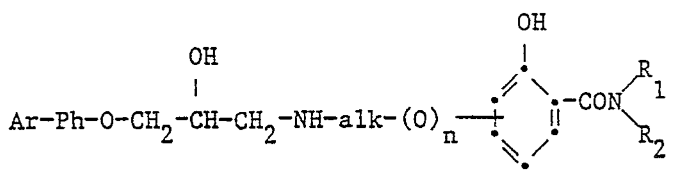

(I)

dans laquelle Ar représente un groupe phényle éventuellement substitué, ou bien un groupe hétéroaryle monocyclique à 5-6 chaînons cycliques reliés par l'intermédiaire d'un atome de carbone cyclique au groupe phénylène PH, contenant en tant que chaînons cycliques un atome d'oxygène, de soufre ou d'azote et le cas échéant 1 à 3 autres atomes d'azote, qui est au moins partiellement hydrogéné et dans ce cas peut être substitué par un groupe oxo, les substituants étant des groupes alkyle inférieurs éventuellement substitués ou alcényle inférieurs, hydroxy éventuellement éthérifié ou estérifié, alkylthio inférieur, alkylsulfonyle inférieur ou des halogènes, des groupes acyle, carboxy éventuellement estérifié, carbamoyle, cyano, nitro, amino éventuellement substitué, par exemple acylé, phényle ou benzoyle éventuellement substitué par des groupes alkyle inférieurs, alcoxy inférieurs, des halogènes, des groupes halogénoalkyle inférieurs, nitro ou cyano et/ou un groupe hétéroaryle monocyclique éventuellement substitué comme indiqué ci-dessus, contenant un atome d'oxygène ou d'azote et le cas échéant de 1 à 3 autres atomes d'azote, Ph représente un groupe 1,4-phénylène éventuellement substitué par des groupes alkyle inférieurs, alcoxy inférieurs, des halogènes ou des groupes cyano, n est égal à 0 ou 1 et alk représente un groupe alkylène en C2-C4, l'atome d'azote et l'atome d'oxygène ou bien, lorsque n est égal à 0, le groupe aromatique, étant séparés l'un de l'autre par 2 à 3 atomes de carbone dans la chaîne non ramifiée, $R_1$ et $R_2$ représentent l'hydrogène ou des groupes alkyle inférieurs ou forment ensemble et avec l'atome d'azote du groupe amide un cycle pyrrolidino ou morpholino, ou de leurs sels, caractérisé en ce que :

a) dans un composé de formule :

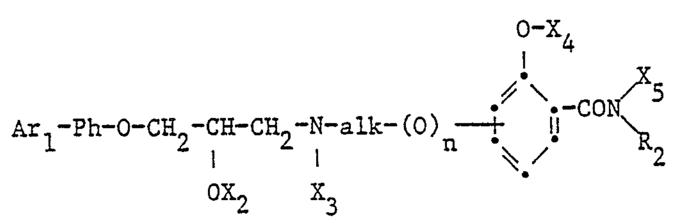

(II)

dans laquelle $Ar_1$ représente le groupe Ar ou un groupe correspondant à celui-ci, contenant au moins un groupement fonctionnel à l'état protégé, $X_2$, $X_3$ et $X_4$ représentent chacun l'hydrogène ou un substituant remplaçable par l'hydrogène, $X_5$ représente $R_1$ ou un substituant remplaçable par l'hydrogène, ou bien $X_2$ et $X_3$ et/ou $X_4$ et $X_5$ représentent ensemble un groupe divalent remplaçable par deux atomes d'hydrogène, et/ou les groupes fonctionnels éventuellement présents dans le groupe $Ar_1$ sont éventuellement à l'état protégé, étant spécifié que l'un au moins des substituants $X_2$, $X_3$, $X_4$ ou $X_5$ n'est pas l'hydrogène, ou qu'au moins $Ar_1$ représente un groupe Ar qui contient au moins un groupement fonctionnel à l'état protégé, ou qu'au moins $X_2$ et $X_3$ ensemble, ou $X_4$ et $X_5$ ensemble, représentent un reste divalent remplaçable par deux atomes d'hydrogène, ou dans un sel d'un tel composé, on remplace par des atomes d'hydrogène les groupes $X_2$, $X_3$, $X_4$ ou $X_5$ ou respectivement $X_2$ et $X_3$ ensemble ou $X_4$ ou $X_5$ ensemble, lorsqu'ils diffèrent de l'hydrogène, et/ou on élimine les groupes protecteurs fixés sur un ou plusieurs groupes fonctionnels dans un groupe $Ar_1$ et on les remplace par l'hydrogène, ou bien

b) on fait réagir un composé de formule

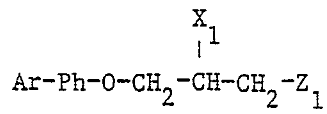

(III)

avec un composé de formule

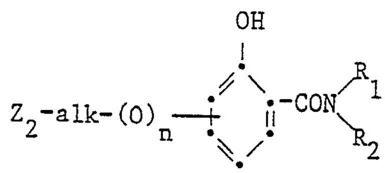

(IV)

ou avec un sel d'un tel composé, dans lesquels l'un des symboles $Z_1$ et $Z_2$ représente un groupe hydroxy estérifié réactif et les autres le groupe amino primaire, et $X_1$ représente un groupe hydroxy, ou bien dans lesquels $X_1$ et $Z_1$ forment ensemble le groupe époxy et $Z_2$ représente le groupe amino primaire, et Ar, Ph, n, alk, $R_1$ et $R_2$ ont les significations indiquées ci-dessus, ou bien

c) dans un composé de formule

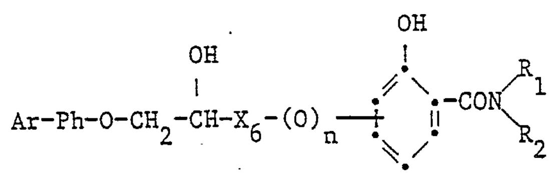

(V)

dans laquelle $X_6$ représente un groupe réductible de formule

—CH=N—alk— (Va) ou —$CH_2$—N=$alk_1$ (Vb)

$alk_1$ représentant un groupe alkylylidène correspondant au groupe alk, et n est égal à 0 ou 1, on réduit $X_6$ en le groupe de formule

—$CH_2$—NH—alk— (Vc)

on élimine simultanément ou à la suite les groupes protecteurs éventuellement présents sur des groupes fonctionnels et qui ne participent pas à la réaction et on les remplace par l'hydrogène, ou bien

d) on fait réagir un composé de formule

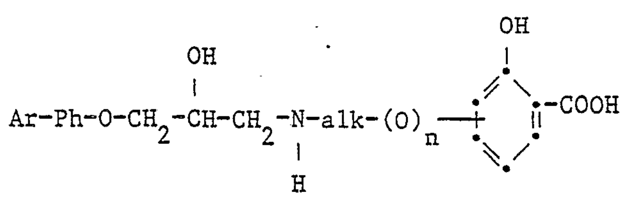

(VI)

dans laquelle les groupes fonctionnels qui ne participent pas à la réaction sont éventuellement protégés par des groupes protecteurs éliminables par aminolyse ou ammoniolyse et remplaçables par l'hydrogène, avec un composé de formule

$HNR_1R_2$ (VIa)

et simultanément ou à la suite, on élimine les groupes protecteurs éventuellement présents et on les remplace par l'hydrogène, ou bien

e) dans un composé de formule

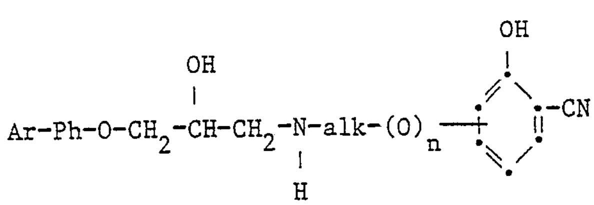

(VII)

dans lequel des groupes fonctionnels ne participant pas à la réaction sont éventuellement protégés par des groupes protecteurs éliminables par hydrolyse et remplaçables par l'hydrogène, ces groupes protecteurs étant éliminés dans les conditions opératoires et remplacés par l'hydrogène, on convertit le groupe —CN par hydrolyse en le groupe —$CONH_2$ et on élimine simultanément les groupes protecteurs éventuellement présents et on les remplace par l'hydrogène, ou bien

f) pour préparer les composés de formule I dans laquelle Ar représente un groupe hétéroaryle mono- ou bi-cyclique, on part d'un composé de formule

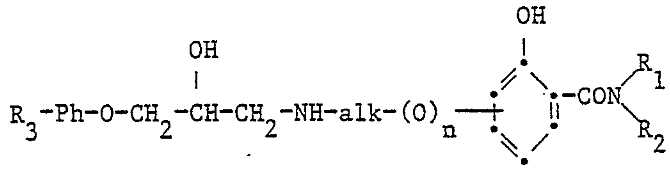

(VIII)

dans laquelle les groupes hydroxy et/ou le groupe amino sont protégés par des groupes protecteurs, et $R_3$ représente un substituant capable de former un groupe hétéroaryle mono- ou bi-cyclique Ar, on forme le groupe hétéroaryle mono- ou bi-cyclique Ar, on élimine simultanément ou à la suite les groupes protecteurs éventuellement présents et on les remplace par l'hydrogène, et, si on le désire, on convertit un composé ainsi obtenu en un autre composé de formule I et/ou, si on le désire, on convertit un composé libre obtenu en un sel ou un sel obtenu en un composé libre, et/ou, si on le désire, on sépare un mélange d'isomères obtenu en les isomères ou un racémate obtenu en les antipodes.

2. Procédé selon la revendication 1, dans lequel Ar représente un groupe phényle éventuellement substitué ou un groupe hétéroaryle monocyclique à 5-6 chaînons cycliques relié au groupe phénylène Ph par l'intermédiaire d'un atome de carbone cyclique, contenant en tant que chaînons cycliques un atome d'oxygène, de soufre ou d'azote et le cas échéant 1 à 2 autres atomes d'azote, ou un groupe oxo-hétéroaryle au moins partiellement hydrogéné, qui contient 1 à 2 atomes d'azote en tant que chaînons cycliques, les substituants étant des groupes alkyle inférieur éventuellement substitué, hydroxy éventuellement éthérifié ou estérifié, alkylthio inférieur ou alkylsulfonyle inférieur, ou des halogènes, des groupes acyle, carboxy éventuellement estérifié, carbamoyle, cyano, amino éventuellement substitué, par exemple acylé, phényle ou benzoyle éventuellement substitué par des groupes alkyle inférieur, alcoxy inférieur, des halogènes, des groupes halogénoalkyle inférieurs ou cyano et/ou un groupe hétéroaryle monocyclique éventuellement substitué comme indiqué ci-dessus, contenant un atome d'oxygène ou d'azote et le cas échéant 1 à 2 autres atomes d'azote, Ph représente un groupe 1,4-phénylène éventuellement substitué par des groupes alkyle inférieurs, alcoxy inférieurs, des halogènes ou des groupes cyano, n est égal à 0 ou 1 et alk représente un groupe alkylène en C2-C3, l'atome d'azote et l'atome d'oxygène ou bien, lorsque n est égal à 0, le groupe aromatique, étant séparés l'un de l'autre par 2 à 3 atomes de carbone dans la chaîne non ramifiée, $R_1$ et $R_2$ représentent l'hydrogène ou des groupes alkyle inférieurs ou forment ensemble et avec l'atome d'azote du groupe amide un cycle morpholino, caractérisé en ce que l'on part des produits de départ correspondants.

3. Procédé selon la revendication 1, dans lequel Ar représente un groupe phényle éventuellement substitué ou un groupe hétéroaryle à 5-6 chaînons cycliques relié au groupe phénylène Ph par l'intermédiaire d'un atome de carbone cyclique, contenant en tant que chaînons cycliques un atome d'oxygène, de soufre ou un atome d'azote et le cas échéant 1 à 2 autres atomes d'azote, par exemple furyle, oxazolyle, imidazolyle, triazolyle, thiazolyle, indolyle ou pyridyle, ou un groupe dihydro-oxo-hétéroaryle contenant 1 à 2 atomes d'azote en tant que chaînons cycliques, par exemple dihydro-oxo-2-pyridinyle ou dihydro-4-oxo-2-pyrimidinyle, les substituants étant des groupes alkyle inférieurs, par exemple méthyle, halogénoalkyle inférieurs par exemple trifluorométhyle, hydroxy, alcoxy inférieurs, par exemple méthoxy, des halogènes, des groupes alcanoyle inférieurs, par exemple acétyle, carboxy, (alcoxy inférieur)-carbonyle, par exemple méthoxycarbonyle, cyano, carbamoyle, amino, alcanoyl-amino inférieurs, par exemple acétylamino, (alcoxy inférieur)-carbonylamino, par exemple méthoxycarbonylamino et/ou un groupe hétéroaryle éventuellement substitué comme indiqué ci-dessus, par exemple imidazolyle ou un groupe hétéroaryle monocyclique contenant un atome d'oxygène ou d'azote et le cas échéant un autre atome d'azote, par exemple un groupe furyle ou oxazolyle, Ph représente un groupe 1,4-phénylène éventuellement substitué par des groupes alkyle inférieurs tels que méthyle ou des halogènes tels que le chlore, n est égal à 0 ou 1, alk représente un groupe alkylène en C2-C3, l'atome d'azote et l'atome d'oxygène, ou bien, lorsque n est égal à 0, le reste aromatique, étant séparés l'un de l'autre par 2 à 3 atomes de carbone dans la chaîne non ramifiée, $R_1$ et $R_2$ représentent chacun l'hydrogène, caractérisé en ce que l'on part des produits de départ correspondants.

4. Procédé de préparation du 1-[2-(3-carbamoyl-4-hydroxy-phénoxy)-éthylamino]-3-[4-[1-méthyl-4-(trifluorométhyl)-1H-imidazole-2-yl]-phénoxy]-2-propanol selon la revendication 1, caractérisé en ce que l'on utilise en tant que produit de départ le 1-[N-benzyl-2-(3-carbamoyl-4-hydroxy-phénoxy)-éthylamino]-3-[4-[1-méthyl-4-(trifluorométhyl)-1H-imidazole-2-yl]-phénoxy]-2-propanol.

5. Procédé de préparation du 1-[2-(3-carbamoyl-4-hydroxy-phénoxy)-éthylamino]-3-[4-[1-méthyl-4-(trifluorométhyl)-1H-imidazole-2-yl]-phénoxy]-2-propanol selon la revendication 1, caractérisé en ce que

l'on utilise en tant que produit de départ le 1-amino-3-[4-(1-méthyl-4-(trifluorométhyl)-1H-imidazole-2-yl)-phénoxy]-2-propanol et le 5-(2-brométhoxy)-salicylamide.

6. Procédé de préparation du 1-[2-(3-carbamoyl-4-hydroxy-phénoxy)-éthylamino]-3-[4-[1-méthyl-4-(trifluorométhyl)-1H-imidazole-2-yl]-phénoxy]-2-propanol selon la revendication 1, caractérisé en ce que l'on utilise en tant que produit de départ la base de Schiff formée à partir du 1-amino-3-[4-(1-méthyl-4-(trifluorométhyl)-1H-imidazole-2-yl)-phénoxy]-2-propanol et de la 2,3-dihydro-2,2-diméthyl-7-(2-oxo-éthoxy)-4H-1,3-benzoxazin-4-one.

7. Procédé de préparation du 1-[2-(3-carbamoyl-4-hydroxy-phénoxy)-éthylamino]-3-[4-[1-méthyl-4-(trifluorométhyl)-1H-imidazole-2-yl]-phénoxy]-2-propanol selon la revendication 1, caractérisé en ce que l'on utilise en tant que produit de départ le 1-[2-(3-carbométhoxy-4-hydroxy-phénoxy)-éthylamino]-3-[4-[1-méthyl-4-(trifluorométhyl)-1H-imidazole-2-yl]-phénoxy]-2-propanol.

8. Procédé de préparation du 1-[2-(3-carbamoyl-4-hydroxy-phénoxy)-éthylamino]-3-[4-(1-méthyl-1H-imidazole-2-yl)-phénoxy]-2-propanol selon la revendication 1, caractérisé en ce que l'on utilise en tant que produit de départ le 1-[N-benzyl-2-(3-carbamoyl-4-benzyloxy-phénoxy)-éthylamino]-3-[4-[1-méthyl-1H-imidazole-2-yl]-phénoxy]-2-propanol.

9. Procédé de préparation du 1-[4-(1,6-dihydro-1-méthyl-6-oxo-2-pyridinyloxy)-phénoxy]-3-[2-(3-carbamoyl-4-hydroxy-phénoxy)-éthylamino]-2-propanol selon la revendication 1, caractérisé en ce que l'on utilise en tant que produit de départ le 1-[4-(1,6-dihydro-1-méthyl-6-oxo-2-pyridinyloxy)-phénoxy]-3-[2-(3-carbamoyl-4-hydroxy-phénoxy)-N-benzyléthylamino]-2-propanol.

10. Procédé de préparation du 1-[2-(3-carbamoyl-4-hydroxy-phénoxy)-éthylamino]-3-[4-(1-méthyl-3-méthylthio-1H-1,2,4-triazole-5-yl)-phénoxy]-2-propanol selon la revendication 1, caractérisé en ce que l'on utilise en tant que produits de départ le 5-[4-2,3-époxy-propoxy)-phényl]-1-méthyl-3-méthylthio-1H-1,2,4-triazole et le 5-(2-aminoéthoxy)-salicylamide.

11. Procédé de préparation du 1-[2-(3-carbamoyl-4-hydroxy-phénoxy)-éthylamino]-3-[4-(1-méthyl-3-méthyl-sulfonyl-1H-1,2,4-triazole-5-yl)-phénoxy]-2-propanol selon la revendication 1, caractérisé en ce que l'on utilise en tant que produit de départ le 1-[N-benzyl-[2-(3-carbamoyl-4-hydroxy-phénoxy)-éthylamino]]-3-[4-(1-méthyl-3-méthylsulfonyl-1H-1,2,4-triazole-5-yl)-phénoxy]-2-propanol.

12. Procédé de préparation du 1-[2-(3-carbamoyl-4-hydroxy-phénoxy)-éthylamino]-3-[4-(1,4,5-triméthyl-1H-imidazole-2-yl)-phénoxy]-2-propanol selon la revendication 1, caractérisé en ce que l'on utilise en tant que produit de départ le 1-[N-benzyl-[2-(3-carbamoyl-4-hydroxy-phénoxy)-éthylamino]]-3-[4-(1,4,5-triméthyl-1H-imidazole-2-yl)-phénoxy]-2-propanol.

13. Procédé selon l'une des revendications 1 à 12, caractérisé en ce que l'on convertit un composé obtenu répondant à la formule I en un sel non toxique et acceptable pour l'usage pharmaceutique par addition avec un acide.